# EUROPEAN PATENT SPECIFICATION

(11) **EP 2 909 314 B1**
(45) Date of publication and mention of the grant of the patent: **02.12.2020**
(21) Application number: 13846458.1
(22) Date of filing: 18.10.2013
(51) Int. Cl.: A61K 35/50, A61K 35/51, A61K 35/28, A61P 29/00

(54) **TREATMENT OF PAIN USING AMNION DERIVED ADHERENT CELLS**
SCHMERZBEHANDLUNG MIT ADHÄRENTEN ZELLEN AUS AMNION
TRAITEMENT DE LA DOULEUR UTILISANT DES CELLULES ADHÉRENTES DÉRIVÉES DE LA MEMBRANE AMNIOTIQUE

(30) Priority: 19.10.2012 US 201261716091 P; 14.03.2013 US 201361783752 P
(43) Date of publication of application: 26.08.2015
(73) Proprietor: Celularity, Inc., Florham Park, NJ 07932 (US)
(72) Inventor: HERZBERG, Uri, Bridgewater, NJ 08807 (US); HARIRI, Robert, J., Bernardsville, NJ 07924 (US); GURNEY, Jodi, P., Chicago, IL 60631 (US)
(74) Representative: Mewburn Ellis LLP
(86) International application number: PCT/US2013/065653
(87) International publication number: WO 2014/063035

(56) References cited:
- WO-A1-2012/066583
- WO-A2-2011/064669
- US-A1- 2002 061 899
- US-A1- 2009 214 484
- US-A1- 2010 124 569
- US-A1- 2012 164 114
- US-A1- 2012 171 180
- T. MIZOKAMI ET AL: "Preferential expansion of human umbilical cord blood-derived CD34-positive cells on major histocompatibility complex-matched amnion-derived mesenchymal stem cells", HAEMATOLOGICA, vol. 94, no. 5, 31 March 2009 (2009-03-31) , pages 618-628, XP055078520, ISSN: 0390-6078, DOI: 10.3324/haematol.2008.004705

## Description

### 1. FIELD

Provided herein are OCT-4- tissue culture surface-adherent amnion-derived adherent cells (AMDACs) for use in methods of ameliorating pain, and treating individuals having pain, using isolated tissue culture surface-adherent cells from amnion, referred to herein as "amnion derived adherent cells" (AMDACs).

### 2. BACKGROUND

Because mammalian placentas are plentiful and are normally discarded as medical waste, they represent a unique source of medically-useful stem cells. There is a need in the medical field for improved compositions and methods of suppressing pain. As such, provided herein are AMDACs, and compositions comprising AMDACs, useful in the treatment of pain, and methods of using the same to treat pain.

US patent application US 2012/0171180 A1 relates to amnion derived adherent cells, and populations of said cells, as well as compositions comprising said cells, in the modulation of an immune response, or the treatment of an immune-related disease or disorder, including a graft-versus host disease, an allergy, or asthma.

### 3. SUMMARY

In one aspect, provided herein are OCT-4- tissue culture surface-adherent amnion-derived adherent cells (AMDACs) for use in method of treating pain, or abnormal sensory conditions such as dysaesthesia, allodynia and hyperalgesia, in an individual, wherein said pain is unresponsive to steroid therapy, or non-steroid anti-inflammatory therapy, comprising administering to the individual a therapeutically effective amount of AMDACs, wherein the therapeutically effective amount is an amount sufficient to cause a detectable improvement in said pain. In a specific embodiment, said method additionally comprises determining one or more first levels of pain in said individual prior to administration of said AMDACs, and determining one or more second levels of pain in said individual after administration of said AMDACs, wherein said therapeutically effective amount of AMDACs reduces said one or more second levels of said pain as compared to said one or more first level of pain. In a more specific embodiment, said therapeutically effective amount of AMDACs results in a detectable improvement in said pain that is greater than, or more long-lasting than, improvement due to administration of a placebo. In a more specific embodiment, said one or more first levels of pain and said one or more second levels of pain are determined by a pain assessment scale. In a more specific embodiment, said pain assessment scale is the Numeric Pain Intensity Scale; the Pain Quality Assessment Scale; the Simple Descriptive Pain Intensity Scale; the Visual Analog Scale; the Wong-Baker FACES Pain Rating Scale; the FLACC scale; the CRIES scale; or the COMFORT scale.

In another specific embodiment, said method additionally comprises determining a first level of one or more physiological indicia of pain in said individual prior to administration of said AMDACs, and determining a second level of one or more physiological indicia of pain in said individual after administration of said AMDACs, wherein said therapeutically effective amount of AMDACs reduces said second level as compared to said first level. In a more specific embodiment, said physiological indicium of pain is heart rate in the individual. In a more specific embodiment, said heart rate in said individual is lower after said administration compared to said heart rate in said individual before said administration. In another more specific embodiment, said physiological indicium of pain is the systolic of said individual. In a more specific embodiment, said systolic of said individual is lower after said administration compared to said systolic in said individual before said administration. In another more specific embodiment, said physiological indicium of pain is the diastolic of said individual. In a more specific embodiment, said diastolic of said individual is lower after said administration compared to said diastolic in said individual before said administration.

In another embodiment said pain is neuropathic pain. In a specific embodiment, said neuropathic pain is caused by diabetic neuropathy. In another specific embodiment, said neuropathic pain is caused by injury to a nerve in said individual. In another specific embodiment, said neuropathic pain is caused by a drug. In certain specific embodiments, said drug is or comprises a platinum-containing anticancer drug, *e.g.,* oxaliplatin, carboplatin or cisplatin, or another chemotherapeutic drug such as paclitaxel or vincristine. In another embodiment, the neuropathic pain is caused by a virus, *e.g.,* a viral disease such as varicella zoster, herpes (*e.g.,* herpes simplex) or human immunodeficiency virus (HIV). In a more specific embodiment, said neuropathic pain is postherpetic neuralgia. In yet another embodiment the pain is caused by radiation injury, *e.g.,* radiation injury that is part of cancer treatment. In yet another embodiment of the method of treating pain, said pain is pain from neuritis. In another embodiment of the method of treating pain, said pain is perineural pain. In yet another embodiment of the method of treating pain, said pain is sciatic nerve pain, *e.g.,* sciatica.

In another embodiment said pain is inflammatory pain. In another embodiment, said pain is bone pain. In a specific embodiment, said bone pain is associated with or caused by cancer. In another embodiment, said pain is caused by cancer. In another embodiment, said pain is caused by or associated with vulvodynia. In another embodiment, said pain is caused by or associated with interstitial cystitis. According to the invention said pain is unresponsive to steroid therapy. According to the invention said pain is unresponsive to nonsteroidal anti-inflammatory therapy. Said pain is may be unresponsive to opioid therapy. In another embodiment, said pain is unresponsive to opiate therapy.

In another aspect, provided herein is a therapeutically effective amount of AMDACs, for use in treating pain in an individual, wherein said pain is unresponsive to steroid therapy, or non-steroid anti-inflammatory therapy, wherein the therapeutically effective amount is an amount sufficient to cause a detectable improvement in said pain. In one embodiment, said level of pain in said individual before said use and said level of pain in the individual after said use are determined by a pain assessment scale, *e.g.,* the Numeric Pain Intensity Scale; the Pain Quality Assessment Scale; the Simple Descriptive Pain Intensity Scale; the Visual Analog Scale; the Wong-Baker FACES Pain Rating Scale; the FLACC scale; the CRIES scale; or the COMFORT scale. In another embodiment, said level of pain in said individual before said use and said level of pain in the individual after said use are determined by one or more physical indicia of pain. In a specific embodiment, said physiological indicium of pain is heart rate in the individual, *e.g.,* said heart rate in said individual is lower after said use than before said use. In another specific embodiment, said physiological indicium of pain is the systolic of said individual, *e.g.,* said systolic in said individual is lower after said use than before said use. In another specific embodiment, said physiological indicium of pain is diastolic of said individual, *e.g.,* said diastolic in said individual is lower after said use than before said use. In certain embodiments, said pain is neuropathic pain. In a more specific embodiment, said neuropathic pain is caused by diabetic neuropathy. In a more specific embodiment, said neuropathic pain is caused by injury to a nerve in said individual. In another more specific embodiment, said neuropathic pain is caused by a drug. In a more specific embodiment, said drug is or comprises a platinum-containing anticancer drug, *e.g.,* platinum-containing anticancer drug is or comprises oxaliplatin, carboplatin or cisplatin. In another specific embodiment, said drug is or comprises paclitaxel. In other specific embodiments, said pain is inflammatory pain, bone pain (*e.g.*, bone pain is associated with or caused by cancer), pain caused by cancer, pain caused by or associated with vulvodynia, pain caused by or associated with interstitial cystitis, or pain caused by degenerative joint disease such as osteoarthritis. According to the invention said pain is unresponsive to steroid therapy. According to the invention said pain is unresponsive to nonsteroidal anti-inflammatory therapy. Said pain may be unresponsive to opioid therapy or non-specific or mixed mu/delta opioids therapy.

The AMDACs are adherent to a tissue culture surface, *e.g.,* tissue culture plastic; and are OCT-4⁻. In certain embodiments, said OCT-4⁻ AMDACs are additionally HLA-G⁻, as determinable by RT-PCR. In certain other specific embodiments, said AMDACs are additionally CD49f⁺, as determinable by flow cytometry. In certain other specific embodiments, said AMDACs are OCT-4⁻, HLA-G⁻ and CD49f⁺. In another specific embodiment, said AMDACs are additionally CD90⁺, CD105⁺, or CD117⁻ as determinable by flow cytometry. In another specific embodiment, said AMDACs are additionally CD90⁺, CD105⁺, and CD117⁻ as determinable by flow cytometry. In another specific embodiment, said AMDACs are OCT-4⁻ and HLA-G⁻, as determinable by RT-PCR, and CD49f⁺, CD90⁺, CD105⁺, and CD117⁻ as determinable by flow cytometry. In another specific embodiment, said AMDACs are additionally VEGFR1/Flt-1⁺ (vascular endothelial growth factor receptor 1) and VEGFR2/KDR⁺ (vascular endothelial growth factor receptor 2), as determinable by immunolocalization. In another specific embodiment, said AMDACs are additionally one or more of CD9⁺, CD10⁺, CD44⁺, CD54⁺, CD98⁺, Tie-2⁺ (angiopoietin receptor), TEM-7⁺ (tumor endothelial marker 7), CDS1⁻, CD34⁻, CD45⁻, CD133⁻, CD143⁻ (angiotensin-I-converting enzyme, ACE), CD146⁻ (melanoma cell adhesion molecule), or CXCR4⁻ (chemokine (C-X-C motif) receptor 4) as determinable by immunolocalization. In a more specific embodiment, said AMDACs are additionally CD9⁺_{'} CD10⁺, CD44⁺, CD54⁺, CD98⁺, Tie-2⁺ (angiopoietin receptor), TEM-7⁺ (tumor endothelial marker 7), CD31⁻, CD34⁻, CD45⁻, CD133⁻, CD14T⁻, CD146⁻, and CXCR4⁻ as determinable by immunolocalization. In another specific embodiment, said AMDACs are additionally VE-cadherin⁻ as determinable by immunolocalization. In another specific embodiment, said AMDACs are additionally positive for CD105⁺ and CD200⁺ as determinable by immunolocalization. In another specific embodiment, said AMDACs additionally do not express CD34 as determinable by immunolocalization after exposure in culture to 50 ng/mL VEGF for 7 days.

In certain specific embodiments, the AMDACs are adherent to tissue culture plastic; wherein said cell is OCT-4⁻, as determinable by RT-PCR, and CD49f⁺, HLA-G⁻, CD90⁺, CD105⁺, and CD117⁻, as determinable by immunolocalization; and wherein said AMDACs: (a) express one or more of CD9, CD10, CD44, CD54, CD98, CD200, Tie-2, TEM-7, VEGFR1/Flt-1, or VEGFR2/KDR (CD309), as determinable by immunolocalization; (b) lack expression of CD31, CD34, CD38, CD45, CD133, CD143, CD144, CD146, CD271, CXCR4, HLA-G, or VE-cadherin, as determinable by immunolocalization, or lack expression of SOX2, as determinable by RT-PCR; (c) express mRNA for ACTA2, ADAMTS1, AMOT, ANG, ANGPT1, ANGPT2, ANGPTL1, ANGPTL2, ANGPTL4, BAI1, CD44, CD200, CEACAM1, CHGA, COL15A1, COL18A1, COL4A1, COL4A2, COL4A3, CSF3, CTGF, CXCL12, CXCL2, DNMT3B, ECGF1, EDG1, EDIL3, ENPP2, EPHB2, FBLN5, F2, FGF1, FGF2, FIGF, FLT4, FN1, FST, FOXC2, GRN, HGF, HEY1, HSPG2, IFNB1, IL8, IL12A, ITGA4, ITGAV, ITGB3, MDK, MMP2, MYOZ2, NRP1, NRP2, PDGFB, PDGFRA, PDGFRB, PECAM1, PF4, PGK1, PROX1, PTN, SEMA3F, SERPINB5, SERPINC1, SERPINF1, TIMP2, TIMP3, TGFA, TGFB1, THBS1, THBS2, TIE1, TIE2/TEK, TNF, TNNI1, TNFSF15, VASH1, VEGF, VEGFB, VEGFC, VEGFR1/FLT1, or VEGFR2/KDR; (d) express one or more of the proteins CD49d, Connexin-43, HLA-ABC, Beta 2-microglobulin, CD349, CD318, PDL1, CD106, Galectin-1, ADAM 17, angiotensinogen precursor, filamin A, alpha-actinin 1, megalin, macrophage acetylated LDL receptor I and II, activin receptor type IIB precursor, Wnt-9 protein, glial fibrillary acidic protein, astrocyte, myosin-binding protein C, or myosin heavy chain, nonmuscle type A; (e) secrete VEGF, HGF, IL-8, MCP-3, FGF2, Follistatin, G-CSF, EGF, ENA-78, GRO, IL-6, MCP-1, PDGF-BB, TIMP-2, uPAR, or galectin-1 into culture medium in which the AMDACs are cultured; (f) express micro RNAs miR-17-3p, miR-18a, miR-18b, miR-19b, miR-92, or miR-296 at a higher level than an equivalent number of bone marrow-derived mesenchymal stem cells; (g) express micro RNAs miR-20a, miR-20b, miR-221, miR-222, miR-15b, or miR-16 at a lower level than an equivalent number of bone marrow-derived mesenchymal stem cells; (h) express miRNAs miR-17-3p, miR-18a, miR-18b, miR-19b, miR-92, miR-20a, miR-20b, miR-296, miR-221, miR-222, miR-15b, or miR-16; and/or (i) express increased levels of CD202b, IL-8 or VEGF when cultured in less than about 5% O₂, compared to expression of CD202b, IL-8 or VEGF under 21% O₂.

According to the invention, treating an individual having pain may use a population of cells comprising any of the AMDACs described herein, wherein at least 50% of the cells in said population, at least 80% of the cells in said population, or at least 90% of the cells in said population are said AMDACs. In a specific embodiment, said population further comprises an isolated second type of cells, and wherein said population is not an amnion, portion of an amnion, or homogenate of an amnion. In a specific embodiment, said second type of cells are hematopoietic stem or progenitor cells, *e.g.,* CD34⁺ cells. In other more specific embodiments, said second type of cells are embryonic stem cells, blood cells, stem cells isolated from peripheral blood, stem cells isolated from placental blood, stem cells isolated from placental perfusate, stem cells isolated from placental tissue, stem cells isolated from umbilical cord blood, umbilical cord stem cells, bone marrow-derived mesenchymal stem cells, bone marrow-derived mesenchymal stromal cells, hematopoietic stem cells, somatic stem cells, chondrocytes, fibroblasts, muscle cells, endothelial cells, angioblasts, endothelial progenitor cells, pericytes, cardiomyocytes, myocytes, cardiomyoblasts, myoblasts, or cells manipulated to resemble embryonic stem cells. In certain more specific embodiments, said second type of cells comprises at least 10%, or at least 25% of cells in said population.

The isolated amnion derived adherent cells, and cell populations comprising AMDACs, useful in the methods provided herein, are not the isolated placental stem cells or cell populations described, *e.g.,* in U.S. Patent No. 7,468,276 or U.S. Patent Application Publication No. 2007/0275362. The isolated amnion derived adherent cells provided herein are also not endothelial progenitor cells, amniotic epithelial cells, trophoblasts, cytotrophoblasts, embryonic germ cells, embryonic stem cells, cells obtained from the inner cell mass of an embryo, or cells obtained from the gonadal ridge of an embryo.

As used herein, the term "about" means, *e.g.,* within 10% of a stated figure or value.

As used herein, the term "stem cell" defines the functional properties of any given cell population that can proliferate extensively, but not necessarily infinitely, and contribute to the formation of multiple tissues, either during embryological development or post-natal tissue replacement and repair.

As used herein, the term "progenitor cell" defines the functional properties of any given cell population that can proliferate extensively, but not necessarily infinitely, and contribute to the formation of a restricted set of multiple tissues in comparison to a stem cell, either during embryological development or post-natal tissue replacement and repair.

As used herein, the term "derived" means isolated from or otherwise purified. For example, amnion derived adherent cells are isolated from amnion. The term "derived" encompasses cells that are cultured from cells isolated directly from a tissue, *e.g.,* the amnion, and cells cultured or expanded from primary isolates.

As used herein, "immunolocalization" means the detection of a compound, *e.g.,* a cellular marker, using an immune protein, *e.g.,* an antibody or fragment thereof in, for example, flow cytometry, fluorescence-activated cell sorting, magnetic cell sorting, *in situ* hybridization, immunohistochemistry, or the like.

As used herein, the term "isolated cells" means cells that are substantially separated from other, *e.g.,* unlike, cells of the tissue, *e.g.,* amnion or placenta, from which the cells are derived. Cells are "isolated" if at least about 20%, 30%, 40%, 50%, 60%, 70%, 80%, 90%, 95%, or at least 99% of the cells with which the cells are naturally associated are removed from the cells, *e.g.,* during collection and/or culture of the cells. As used herein, the term "isolated population of cells" means a population of cells that is substantially separated from other cells of the tissue, *e.g.,* amnion or placenta, from which the population of cells is derived.

As used herein, cells are "positive" for a particular marker when that marker is detectable above background, *e.g.,* by immunolocalization, *e.g.,* by flow cytometry; or by RT-PCR. For example, cells are described as positive for, *e.g.,* CD105 if CD105 is detectable on the cells in an amount detectably greater than background (in comparison to, *e.g.,* an isotype control). In the context of, *e.g.,* antibody-mediated detection, "positive," as an indication a particular cell surface marker is present, means that the marker is detectable using an antibody, *e.g.,* a fluorescently-labeled antibody, specific for that marker; "positive" also means that the cells bear that marker in a amount that produces a signal, *e.g.,* in a flow cytometer, that is detectably above background, or above that of an isotype control. For example, cells are "CD105⁺" where the cell is detectably labeled with an antibody specific to CD105, and the signal from the antibody is detectably higher than a control (*e.g.*, background). Conversely, "negative" in the same context means that the cell surface marker is not detectable using an antibody specific for that marker compared to background. For example, cells are "CD34⁻" where the cells are not detectably labeled with an antibody specific to CD34. Unless otherwise noted herein, cluster of differentiation ("CD") markers are detected using antibodies. For example, OCT-4 can be determined to be present, and a cell is OCT-4⁺, if mRNA for OCT-4 is detectable using RT-PCR, *e.g.,* for 30 cycles.

In certain embodiments, said AMDACs are formulated to be administered locally. In certain other embodiments, said AMDACs are formulated to be administered systemically, *e.g.,* intravenously or intraarterially.

### 4. BRIEF DESCRIPTION OF THE FIGURES

FIG. 1 demonstrates the effect of IV administration of AMDACs on pain induced by sciatic nerve perineural inflammation (neuritis).
FIG 2 demonstrates the effect of intramuscular (IM) administration of AMDACs injected into the muscle on pain induced by sciatic nerve perineural inflammation.

### 5. DETAILED DESCRIPTION

### 5.1 METHODS OF TREATMENT OF PAIN

Pain is generally defined as an unpleasant sensory and emotional experience associated with actual or potential tissue damage or described in terms of such damage. Merskey H, Bogduk N, eds., Classification of Chronic Pain, International Association for the Study of Pain (IASP) Task Force on Taxonomy, IASP Press: Seattle, 209-214, 1994.

In one aspect, provided herein are AMDHCs for use in a method ot treating an individual having pain, wherein said pain is unresponsive to steroid therapy, or non-steroid anti-inflammatory therapy, comprising administering to the individual a therapeutically effective amount of AMDACs, wherein the therapeutically effective amount is an amount sufficient to cause a detectable improvement in said pain or a symptom associated with said pain. In one embodiment, said method additionally comprises determining a first level of pain in said individual prior to administration of said AMDACs, and determining a second level of pain in said individual after administration of said AMDACs, wherein said therapeutically effective amount of AMDACs reduces said second level of said pain as compared to said first level of pain.

In certain embodiments, the therapeutically effective amount of AMDACs, when administered, results in greater, or more long-lasting, improvement of pain in the individual as compared to administration of a placebo.

In certain embodiments, the pain is nociceptive pain. Nociceptive pain is typically elicited when noxious stimuli such as inflammatory chemical mediators are released following tissue injury, disease, or inflammation and are detected by normally functioning sensory receptors (nociceptors) at the site of injury. *See, e.g.,* Koltzenburg, M. Clin. J. of Pain 16:S131-S138 (2000). Examples of causes of nociceptive pain include, but are not limited to, chemical or thermal burns, cuts and contusions of the skin, osteoarthritis, rheumatoid arthritis, tendonitis, and myofascial pain. In certain embodiments, nociceptive pain is stimulated by inflammation.

In certain other embodiments, the pain is neuropathic pain. Neuropathic pain reflects injury or impairment of the nervous system, and has been defined as "pain initiated or caused by a primary lesion or dysfunction in the nervous system." Merskey H, Bogduk N, eds., Classification of Chronic Pain, International Association for the Study of Pain (IASP) Task Force on Taxonomy, IASP Press: Seattle, 209-214, 1994. In a specific embodiment, the neuropathic pain is characterized by altered excitability of peripheral neurons. In other specific embodiments, the neuropathic pain includes, but is not limited to, pain associated with diabetic neuropathy, postherpetic neuralgia, trigeminal neuralgia, and post-stroke pain. In certain embodiments, the neuropathic pain is continuous, episodic, and is described as, *e.g.,* burning, tingling, prickling, shooting, electric-shock-like, jabbing, squeezing, deep aching, or spasmodic. In certain other embodiments, the individual having neuropathic pain additionally experiences partial or complete sensory deficit, abnormal or unfamiliar unpleasant sensations (dysaesthesia), pain resulting from non-noxious stimuli, or disproportionate perception of pain in response to supra-threshold stimuli (hyperalgesia).

In another specific embodiment, the neuropathic pain is complex regional pain syndrome (CRPS). In a specific embodiment, CRPS affects the extremities in the absence of a nerve injury (CRPS type I). In a more specific embodiment, said CRPS type I includes reflex sympathetic dystrophy (RSD). In a more specific embodiment, said RSD is stage I RSD, or "early RSD". In early RSD, pain is more severe than would be expected from the injury, and it has a burning or aching quality. It may be increased by dependency of the limb, physical contact, or emotional upset. The affected area typically becomes edematous, may be hyperthermic or hypothermic, and may show increased nail and hair growth. Radiographs may show early bony changes. In another more specific embodiment, said RSD is stage II RSD, or "established RSD". In a more specific embodiment, said established RSD comprises, in addition to pain, induration of edematous tissue; hyperhidrosis of skin with livedo reticularis or cyanosis; hair loss; ridging, cracking or brittling of nails; development of dry hands; and/or noticeable atrophy of skin and subcutaneous tissues. Pain remains the dominant feature. In another more specific embodiment, said RSD is stage III RSD, or "late RSD". In a more specific embodiment, said late RSD comprises pain that spreads proximally; irreversible tissue damage; thin, shiny skin; and bone demineralization visible on radiographs.

In another specific embodiment, the neuropathic pain is pain caused by a drug, *e.g.,* a chemotherapeutic drug or anti-cancer drug. In specific embodiments, the drug is or comprises a platinum-containing drug, a taxane, an epothilone, a plant alkaloid, or a thalidomide. In more specific embodiments, the drug is or comprises bortezomib, carboplatin (*e.g*., PARAPLATIN®), cisplatinum (*e.g.,* PLATINOL®), cytarabine (*e.g.,* CYTOSAR®, Ara-C), docetaxel (*e.g.,* TAXOTERE®), etoposide/VP-16 (VEPESID®), gemcitabine (*e.g*., GEMZAR®), HALAVEN® (eribulin mesylate), hexamethylmelamine (*e.g.,* HEXALIN®), paclitaxel (*e.g.,* TAXOL®; ABRAXANE®), oxaliplatin (*e.g.,* ELOXATIN®), suramin, thalidomide (*e.g.,* THALOMID®), vinblastine (*e.g.,* VELBAN®; ALKABAN-AQ®), vincristine (*e.g.,* ONCOVIN®, VINCASAR PFS®, Vincrex), or vinorelbine (NAVELBINE®).

In certain other specific embodiments, the drug is an antibiotic. In certain other embodiments, the drug is a statin.

In certain other specific embodiments, the drug is or comprises amlodipine (*e.g.*, NORVASC®), Lotril or Lotrel), atorvastatin (*e.g.,* LIPITOR®), duloxetine (*e.g.,* CYMBALTA®), pregabalin (LYRICA®), allopurinol (*e.g*., LOPURIM®, ZYLOPRIM®), aminodipinberglate, amiodarone (*e.g*., CORDARONE®, PACERONE®), amiodipine, amitriptyline (*e.g.,* ELAVIL™, ENDEP™, VANATRIP™), metronidazole (*e.g.,* FLAGYL®, METROGEL™), nitrofurantoin (*e.g*., FURADANTIN®, MACROBID®, MACRODANTIN®, NITRO MACRO), perhexiline, VYTORIN®, ciprofloxacin (*e.g.*, CIPRO®, PROQUIN®), disulfiram (*e.g.,* ANTABUSE), zolpidem (*e.g.,* AMBIEN®), buspirone (*e.g.,* BUSPAR), clonazepam (*e.g.,* KLONOPIM, CEBERKLON, VALPAX), alprazolam (*e.g.,* XANAX®), phenytoin (DILANTIN®), citalopram (*e.g.,* CELEXA), duloxetine (*e.g.,* CYMBALTA®), venlafaxine (*e.g.,* EFFEXOR, EFFEXOR XR®), nortriptyline (*e.g.,* AVENTYL HCL, PAMELOR), sertraline (*e.g.,* ZOLOFT®), paroxetine (*e.g.,* PAXIL, PAXIL CR®), atenolol (*e.g.,* TENORMIN, SENORMIN), perindopril (*e.g.,* ACEON), altace (*e.g.,* RAMIPRIL®), losartan (*e.g.,* COZAAR®, HYZAAR®), hydralazine (*e.g.,* APRESOLINE®), hydrochlorothiazide (*e.g.,* HYDRODIURIL™, EZIDE™, HYDRO-PAR™, MICROZIDE™), lisinopril (*e.g.,* PRINIVIL®, ZESTRIL®), telmisartan (*e.g.,* MICARDIS™), perhexiline, prazosin (*e.g.,* MINIPRESS®), lisinopril (*e.g.,* PRINIVIL®, ZESTRIL®), lovastatin (*e.g.,* ALTOCOR®, MEVACOR®), CADUET®, rosuvastatin (*e.g.,* CRESTOR®), fluvastatin (*e.g.,* LESCOL®, LESCOL® XL), simvastatin (*e.g.,* ZOCOR®), cerivastatin (*e.g.,* LIPOBAY™), gemfibrozil (*e.g.,* LOPID®), pravastatin (*e.g.,* PRAVACHOL®, PRAVIGARD PAC™), d4T (stavudine, *e.g.,* ZERIT®), ddC (zalcitibine; *e.g.,* HIVID®), ddI (didanosine, *e.g.,* VIDEX® EC), isoniazid (*e.g*., TUBIZID®), diaminodiphenylsulfone (DDS, dapsone)

In another specific embodiment, said CRPS affects the extremities in the presence of a nerve injury (CRPS type II). In a more specific embodiment, said CRPS II includes causalgia. In another specific embodiment, said CRPS includes sympathetic maintained pain syndrome. In certain embodiments, symptoms of CRPS include but are not limited to pain, autonomic dysfunction, edema, movement disorder, dystrophy, atrophy, burning pain, allodynia (pain with light touch). In certain embodiments, CRPS-related pain is accompanied by swelling and joint tenderness, increased sweating, sensitivity to temperature, and/or color change of the skin.

In certain other specific embodiments, the neuropathic pain is neuropathic pain caused by or related to a dietary deficiency. In a more specific embodiment, the dietary deficiency is vitamin B12 (cobalamin, cyanocobalamin) deficiency. In another more specific embodiment, the dietary deficiency is vitamin B6 (pyridoxine, pyridoxal phosphate) deficiency. In another more specific embodiment, the dietary deficiency is vitamin B1 (thiamine) deficiency. In another specific embodiment, the individual having neuropathic pain, caused by nutritional deficiency, has had bariatric surgery. In another specific embodiment, the neuropathic pain is caused by or is related to alcoholism or consumption of alcohol by the individual having pain.

In certain embodiments, the pain is caused by or associated with vulvodynia. Vulvodynia is pain of the vulva, *e.g.,* pain unexplained by vulvar or vaginal infection or skin disease. In one embodiment, the pain of vulvodynia is localized to the vulvar region, *e.g.,* in the vestibular region such as vulvar vestibulitis or vestibulodynia. In another embodiment, the pain of vulvodynia may extend into the clitoris, *e.g.,* clitorodynia. Example of causes of vulvodynia include, but are not limited to, dyspareunia, injury to or irritation of the nerves that innervate the vulva, genetic predisposition to inflammation, allergy, autoimmune disorders (*e.g*., lupus erythematosus or Sjogren's Syndrome), infection (*e.*g., yeast infections, HPV or bacterial vaginosis), and neuropathy. Exemplary symptoms of vulvodynia include without limitation, diffuse pain or burning sensation on or around the vulva, the labia majora, labia minor, or the vestibule.

In certain embodiments, the pain is caused by or associated with interstitial cystitis. Interstitial cystitis, also known as bladder pain syndrome, is a chronic condition, often characterized by, *e.g.,* pain or pressure associated with the bladder, pain associated with urination, irritative voiding, urinary frequency, urgency, or pain or pressure in pelvis. The pathology and pathogenesis of interstitial cystitis is not clearly understood. However, several possible causes have been proposed, *e.g.,* vascular obstruction, autoimmunity, inflammation, leaky bladder lining, mast cells, stress, and genetic, neurogenic and endocrine causes. In one embodiment, diagnosis of interstitial cystitis can be done by, *e.g.,* the Pelvic Pain Urgency/Frequency (PUF) Patient Survey or the KCl test, also known as the potassium sensitivity test.

In certain other embodiments, the pain is visceral pain.

In certain other embodiments, the pain is post-operative pain, such as that resulting from trauma to tissue caused during surgery.

In certain other embodiments, the pain is mixed pain, *e.g.,* is chronic pain that has nociceptive and neuropathic components. In specific embodiments, said mixed pain is cancer pain or low back pain.

In certain other embodiments, the pain is migraine pain or pain from headache, *e.g.,* vascular headache, cluster headache or toxic headache.

In other embodiments, the pain is caused by a viral infection, *e.g.,* infection by a herpesvirus, varicella virus (*e.g.*, varicella zoster), or human immunodeficiency virus. In a specific embodiment, the pain is, or is related to, postherpetic neuralgia.

In specific embodiments, said symptoms associated with pain include, but are not limited to, one or more of autonomic dysfunction, inability to initiate movement, weakness, tremor, muscle spasm, dystonia, dystrophy, atrophy, edema, stiffness, joint tenderness, increased sweating, sensitivity to temperature, light touch (allodynia), color change to the skin, hyperthermic or hypothermic, increased nail and hair growth, early bony changes, hyperhidrotic with livedo reticularis or cyanosis, lost hair, ridged, cracked or brittle nails, dry hand, diffuse osteoporosis, irreversible tissue damage, thin and shiny skin, joint contractures, and marked bone demineralization.

### 5.1.1 Pain Assessment Scales

According to the invention, the therapeutically effective amount of AMDACs administered to the individual having pain is an amount that results in a detectable reduction in the pain in the individual. The reduction can be detectable to the individual, detectable to an observer, or both. In certain embodiments the level of pain in the individual is assessed by the individual, *e.g.,* as guided by a medical doctor, or as part of a pretreatment workup, according to one or more individual pain scales. In certain other embodiments, the level of pain in the individual is assessed by an observer using one or more observer pain scales. Where levels of pain are assessed according to the method before and after administration of AMDACs, the same scale is preferably used for each assessment. Pain in the individual can be assessed once or more than once, *e.g.,* 2, 3, 4, or 5 times, before administration of AMDACs, and once or more than once, *e.g.,* 2, 3, 4, or 5 times, after administration of AMDACs.

In one embodiment, pain in the individual is assessed by the 0-10 Numeric Pain Intensity Scale. In this scale, zero equals no pain, and 10 equals the worst pain. In certain embodiments, *e.g.,* the Pain Quality Assessment Scale, the pain is broken down into more than one numeric descriptor, *e.g.,* 0-10 for how "hot" the pain feels, 0-10 for how "intense" the pain feels, 0-10 for how "sharp" the pain feels, 0-10 for how "dull" the pain feels, 0-10 for how "cold" the pain feels, 0-10 for how "sensitive" the pain feels, 0-10 for how "tender" the pain feels, 0-10 for how "itchy" the pain feels, 0-10 for how "shooting" the pain feels, 0-10 for how "numb" the pain feels, 0-10 for how "tingling" the pain feels, 0-10 for how "electrical" the pain feels, 0-10 for how "cramping" the pain feels, 0-10 for how "throbbing" the pain feels, 0-10 for how "radiating" the pain feels, 0-10 for how "aching" the pain feels, 0-10 for how "heavy" the pain feels, and/or 0-10 for how "unpleasant" the pain feels.

In another embodiment, pain in the individual is assessed by the Simple Descriptive Pain Intensity Scale. In this scale, pain is described as, *e.g.,* "no pain", "mild pain", "moderate pain", "severe pain", "very severe pain" or "worst possible pain".

In another embodiment, pain in the individual is assessed by the Visual Analog Scale. In the Visual Analog Scale, the individual is presented with a graph consisting of a vertical line; one end of the line is labeled "no pain" and the other end is labeled "worst possible pain". The individual is asked to mark the line at a point between the two ends indicating the level of pain perceived by the individual.

In another embodiment, pain in the individual is assessed by the Wong-Baker FACES Pain Rating Scale. In the FACES Pain Rating Scale, the level of pain is indicated by a series of cartoon faces, typically six faces, appearing happy to progressively more unhappy. In a specific embodiment, the faces are subtexted with phrases such as "no hurt", "hurts little bit" "hurts little more", "hurts even more", "hurts whole lot" and "hurts worst". In another specific embodiment, the faces are subtexted with phrases such as "no pain", "mild, annoying pain", "nagging, uncomfortable, troublesome pain", "distressing, miserable pain", "intense, dreadful, horrible pain" and "worst possible, unbearable, excruciating pain", either alone or accompanied by a numeric 0 to 10 scale.

In certain embodiments, pain in the individual is assessed by the FLACC (Face, Legs, Activity, Cry and Consolability) scale. In specific embodiments, each of the five characteristics is rated from, *e.g.,* 0 to 2, with 2 indicating pain and 0 indicating no pain. The scores may be used separately or totaled.

In certain other embodiment, pain in the individual is assessed by the CRIES (Crying, Requires O₂ for SaO₂ (hemoglobin saturation), Increased vital signs (blood pressure and heart rate, Expression and Sleepless) scale. In specific embodiments, each of the five characteristics is rated from, *e.g.,* 0 to 2, with 2 indicating pain and 0 indicating no pain. The scores may be used separately or totaled.

In certain embodiment, pain in the individual is assessed by the COMFORT scale, which assesses nine different characteristics (alertness, calmness, respiratory distress, crying, physical movement, muscle tone, facial tension, blood pressure and heart rate), each rated on a scale of 1-5, with 1 indicating no or least pain, and 5 most pain. The scores may be used individually or totaled.

### 5.1.2 Physiological indicia of pain

As used herein, "treatment of pain" and the like can comprise completely eliminating pain; noticeable reduction of pain by the individual suffering the pain; detectable reduction of pain or indicia of pain by objective criteria (*e.g*., heart rate, blood pressure, muscle tone, or the like); or a combination of any two or all three. In certain other embodiments, pain in the individual can be assessed, either before or after administration of AMDACs, or both, by physiological criteria, *e.g.,* physiological criteria of stress. Such physiological criteria can include objectively measurable criteria such as heart rate or blood pressure, *e.g.,* elevated heart rate or blood pressure as compared to a non-pain state in the individual, or as compared to an expected norm (*e.g.,* 120 systolic and 80 diastolic; 60 beats per minute). Such physiological criteria can also, or instead, include subjectively measurable criteria such as facial expressions, muscle tensioning (muscle tone), sweating, trembling, and the like.

Thus, in certain embodiments, the therapeutically effective amount of AMDACs, administered to the individual having pain, results in a detectable reduction in heart rate in the individual, *e.g.,* a 5%, 10%, 15%, 20%, 25%, 30%, 35%, 40%, 45% or 50% reduction; a reduction of heart rate from 120 beats per minute (bpm) or above to below 110 bpm; a reduction from 110 bpm or above to below 100 bpm; a reduction from 100 bpm or above to below 90 bpm; a reduction from 90 bpm or above to below 80 bpm; a reduction from 120 bpm or above to below 100 bpm; a reduction from above to below 90 bpm; a reduction from 100 bpm above to below 80 bpm; a reduction from 130 bpm above to below 100 bpm; a reduction from 120 bpm above to below 90 bpm; a reduction from 110 bpm to below 80 bpm; or a reduction from 120 bpm or above to below 80 bpm.

In certain other embodiments, the therapeutically effective amount of AMDACs, when administered to the individual having pain, results in a detectable reduction in blood pressure in the individual, *e.g.,* a reduction of 5%, 10%, 15%, 20%, 25%, 30%, 35%, 40%, 45% or 50% reduction in the individual's systolic, diastolic, or both; a reduction in the individual's systolic from 200 or above to under 190; a reduction in the systolic from 190 or above to under 180; a reduction in the systolic from 180 or above to under 170; a reduction in the systolic from 170 or above to under 160; a reduction in the systolic from 160 or above to under 150; a reduction in the systolic from 150 or above to under 140; a reduction in the systolic from 140 or above to under 130; a reduction in the systolic from 200 or above to under 180; a reduction in the systolic from 190 or above to under 170; a reduction in the systolic from 180 or above to under 160; a reduction in the systolic from 170 or above to under 150; a reduction in the systolic from 160 or above to under 140; a reduction in the systolic from 150 or above to under 130; a reduction in the systolic from 200 or above to under 170; a reduction in the systolic from 190 or above to under 160; a reduction in the systolic from 180 or above to under 150; a reduction in the systolic from 170 or above to under 140; a reduction in the systolic from 160 or above to under 130; a reduction in the systolic from 200 or above to under 160; a reduction in the systolic from 190 or above to under 150; a reduction in the systolic from 180 or above to under 140; a reduction in the systolic from 200 or above to under 130; a reduction in the systolic from 200 or above to under 150; a reduction in the systolic from 190 or above to under 140; a reduction in the systolic from 180 or above to under 130; a reduction in the systolic from 200 or above to under 140; a reduction in the systolic from 190 or above to under 130; or a reduction in the systolic from 200 or above to under 130; a reduction in the individual's diastolic from 140 or above to under 130; a reduction in the diastolic from 130 or above to under 120; a reduction in the diastolic from 120 or above to under 110; a reduction in the diastolic from 110 or above to under 100; a reduction in the diastolic from 100 or above to under 90; a reduction in the diastolic from 140 or above to under 120; a reduction in the diastolic from 110 or above to below 90; a reduction in the diastolic from 140 or above to below 110; a reduction in the diastolic from 130 or above to under 100; a reduction in the diastolic from 120 or above to under 90; a reduction in the diastolic from 140 or above to below 100; a reduction in the diastolic from 130 or above to below 90; or a reduction in the diastolic from 140 or above to below 90.

### 5.2 SECOND THERAPEUTIC COMPOSITIONS AND SECOND THERAPIES

In any of the above AMDACs for use in methods of treatment of pain in an individual, the method can comprise the administration of a second therapeutic composition or second therapy, *e.g.,* an antipain medication or therapy. In a preferred embodiment, the second active agents are capable of relieving pain, inhibiting inflammatory reactions, providing a sedative effect or an antineuralgic effect, or ensuring patient comfort.

In certain embodiments, the second therapeutic compositions comprise, but are not limited to, opioid analgesics, non-narcotic analgesics, antiinflammatories, cox-2 inhibitors, alpha-adrenergic receptor agonists or antagonists, ketamine, anesthetic agents, NMDA antagonists, immunomodulatory agents, immunosuppressive agents, antidepressants, anticonvulsants, antihypertensives, anxiolytics, calcium channel blockers, muscle relaxants, corticosteroids, hyperbaric oxygen, JNK inhibitors, other therapeutics known to relieve pain, and pharmaceutically acceptable salts, solvates, hydrates, stereoisomers, clathrates, prodrugs and pharmacologically active metabolites thereof.

In certain embodiments, the second therapeutic composition is an opioid. Opioids can be used, *e.g.,* to treat severe pain. Examples of opioid analgesics include, but are not limited to, oxycodone (*e.g.,* OXYCONTIN®), morphine sulfate (*e.g.,* MS CONTIN®, DURAMORPH®, and/or ASTRAMORPH®), meperidine (*e.g.*, DEMEROL®), and fentanyl transdermal patch (*e.g.,* DURAGESIC®) and other known conventional medications. Oxycodone (*e.g.,* OXYCONTIN®) is a long-acting form of an opioid and may be used, *e.g.,* in initial and later stages of CRPS.

Non-narcotic analgesics and anti-inflammatories may be used, *e.g.,* for treatment of pain during pregnancy and breastfeeding. Non-steroidal anti-inflammatory drugs (NSAIDs) may be used, *e.g.,* in the early stage of pain syndrome. Examples of anti-inflammatories include, but are not limited to, salicylic acid acetate (*e.g.,* aspirin), ibuprofen (*e.g.,* MOTRIN®, ADVIL®, or the like), ketoprofen (*e.g.,* ORUVAIL®), rofecoxib (*e.g.,* VIOXX®), naproxen sodium (*e.g.,* ANAPROX®, NAPRELAN®, NAPROSYN®, or the like), ketorolac (*e.g.*, ACULAR®), or other known conventional medications. A specific cox-2 inhibitor is celecoxib (*e.g.*, CELEBREX).

Examples of second therapeutic compounds that are antidepressants include, but are not limited to, nortriptyline (PAMELOR®), amitriptyline (ELAVIL®), imipramine (TOFRANIL®), doxepin (SINEQUAN®), clomipramine (ANAFRANIL®), fluoxetine (PROZAC®), sertraline (ZOLOFT®), nefazodone (SERZONE®), venlafaxine (EFFEXOR®), trazodone (DESYREL®), bupropion (WELLBUTRIN®) and other known conventional medications. See, *e.g.,* Physicians' Desk Reference, 329, 1417, 1831 and 3270 (57th ed., 2003).

Examples of second therapeutic compounds that are anticonvulsant drugs include, but are not limited to, carbamazepine, oxcarbazepine, gabapentin (NEURONTIN®), phenyloin, sodium valproate, clonazepam, topiramate, lamotrigine, zonisamide, and tiagabine. See, *e.g.,* Physicians' Desk Reference, 2563 (57th ed., 2003).

Other second therapeutic compounds include, but are not limited to, corticosteroids (*e.g.*, prednisone, dexamethasone or hydrocortisone), orally active class Ib anti-arrhythmic agents (*e.g.,* mexiletine), calcium channel blockers (*e.g.,* nifedipine), beta-blockers (*e.g.,* propranolol), alpha-blocker (*e.g.,* phenoxybenzamine), and alpha2-adrenergic agonists (*e.g.,* clonidine) can also be used in combination with an immunomodulatory compound. See, *e.g.,* Physicians' Desk Reference, 1979, 2006 and 2190 (57th ed., 2003).

In another specific embodiment, said second therapy comprises an immunomodulatory compound, wherein the immunomodulatory compound is 3-(4-amino-1-oxo-1,3-dihydroisoindol-2-yl)-piperidine-2,6-dione; 3-(4'aminoisolindoline-1'-one)-1-piperidine-2,6-dione; 4-(Amino)-2-(2,6-dioxo(3-piperidyl))-isoindoline-1,3-dione; or α-(3-aminophthalimido) glutarimide. In a more specific embodiment, said immunomodulatory compound is a compound having the structure 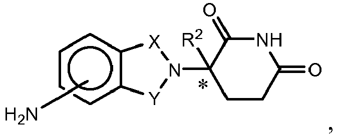 wherein one of X and Y is C=O, the other of X and Y is C=O or CH₂, and R² is hydrogen or lower alkyl, or a pharmaceutically acceptable salt, hydrate, solvate, clathrate, enantiomer, diastereomer, racemate, or mixture of stereoisomers thereof. In another more specific embodiment, said immunomodulatory compound is a compound having the structure 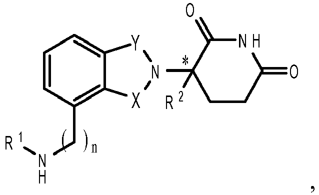 wherein one of X and Y is C=O and the other is CH₂ or C=O;
R¹ is H, (C₁-C₈)alkyl, (C₃-C₇)cycloalkyl, (C₂-C₈)alkenyl, (C₂-C₈)alkynyl, benzyl, aryl, (C₀-C₄)alkyl-(C₁-C₆)heterocycloalkyl, (C₀-C₄)alkyl-(C₂-C₅)heteroaryl, C(O)R³, C(S)R³, C(O)OR⁴, (C₁-C₈)alkyl-N(R⁶)₂, (C₁-C₈)alkyl-OR⁵, (C₁-C₈)alkyl-C(O)OR⁵, C(O)NHR³, C(S)NHR³, C(O)NR³R^{3'}, C(S)NR³R^{3'} or (C₁-C₈)alkyl-O(CO)R⁵;
R² is H, F, benzyl, (C₁-C₈)alkyl, (C₂-C₈)alkenyl, or (C₂-C₈)alkynyl;
R³ and R^{3'} are independently (C₁-C₈)alkyl, (C₃-C₇)cycloalkyl, (C₂-C₈)alkenyl, (C₂-C₈)alkynyl, benzyl, aryl, (C₀-C₄)alkyl-(C₁-C₆)heterocycloalkyl, (C₀-C₄)alkyl-(C₂-C₅)heteroaryl, (C₀-C₈)alkyl-N(R⁶)₂, (C₁-C₈)alkyl-OR⁵, (C₁-C₈)alkyl-C(O)OR⁵, (C₁-C₈)alkyl-O(CO)R⁵, or C(O)OR⁵;
R⁴ is (C₁-C₈)alkyl, (C₂-C₈)alkenyl, (C₂-C₈)alkynyl, (C₁-C₄)alkyl-OR⁵, benzyl, aryl, (C₀-C₄)alkyl-(C₁-C₆)heterocycloalkyl, or (C₀-C₄)alkyl-(C₂-C₈)heteroaryl;
R⁵ is (C₁-C₈)alkyl, (C₂-C₈)alkenyl, (C₂-C₈)alkynyl, benzyl, aryl, or (C₂-C₅)heteroaryl;
each occurrence of R⁶ is independently H, (C₁-C₈)alkyl, (C₂-C₈)alkenyl, (C₂-C₈)alkynyl, benzyl, aryl, (C₂-C₅)heteroaryl, or (C₀-C₈)alkyl-C(O)O-R⁵ or the R⁶ groups can join to form a heterocycloalkyl group;
n is 0 or 1; and
* represents a chiral-carbon center;
or a pharmaceutically acceptable salt, hydrate, solvate, clathrate, enantiomer, diastereomer, racemate, or mixture of stereoisomers thereof. In another more specific embodiment, said immunomodulatory compound is a compound having the structure 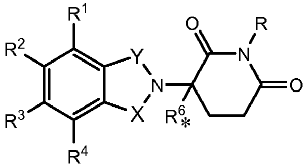 wherein:
one of X and Y is C=O and the other is CH₂ or C=O;
R is H or CH₂OCOR';
(i) each of R¹, R², R³, or R⁴, independently of the others, is halo, alkyl of 1 to 4 carbon atoms, or alkoxy of 1 to 4 carbon atoms or (ii) one of R¹, R², R³, or R⁴ is nitro or -NHR⁵ and the remaining of R¹, R², R³, or R⁴ are hydrogen;
R⁵ is hydrogen or alkyl of 1 to 8 carbons
R⁶ hydrogen, alkyl of 1 to 8 carbon atoms, benzo, chloro, or fluoro;
R' is R⁷-CHR¹⁰-N(R⁸R⁹);
R⁷ is m-phenylene or p-phenylene or -(CₙH₂ₙ)- in which n has a value of 0 to 4;
each of R⁸ and R⁹ taken independently of the other is hydrogen or alkyl of 1 to 8 carbon atoms, or R⁸ and R⁹ taken together are tetramethylene, pentamethylene, hexamethylene, or -CH₂CH₂X₁CH₂CH₂- in which X₁ is -O-, -S-, or -NH-;
R¹⁰ is hydrogen, alkyl of to 8 carbon atoms, or phenyl; and
* represents a chiral-carbon center;
or a pharmaceutically acceptable salt, hydrate, solvate, clathrate, enantiomer, diastereomer, racemate, or mixture of stereoisomers thereof. In specific embodiments, the second therapeutic compound is lenalidomide or pomalidomide.

Any combination of the above therapeutic agents can be administered. Such therapeutic agents can be administered in any combination with the AMDACs, at the same time or as a separate course of treatment.

It should be noted that some of the second therapeutic compounds listed above (*e.g*., fluoxetine), though having a beneficial effect, may themselves as a side effect cause neuropathic pain in a small number of recipients. Generally, such compounds are considered safe to administer; however, one of ordinary skill in the art (*e.g.,* a physician) will be able to determine the relative benefit of administering such a second therapeutic compound compared to the risk of further neuropathic pain.

AMDACs can be administered to the individual suffering from pain in the form of a pharmaceutical composition, *e.g.,* a pharmaceutical composition suitable for intravenous, intramuscular or intraperitoneal injection. AMDACs can be administered to the individual in a single dose, or in multiple doses. Where AMDACs are administered in multiple doses, the doses can be part of a therapeutic regimen designed to relieve the pain, or can be part of a long-term therapeutic regimen designed to treat the underlying cause of the pain. In embodiments in which AMDACs are administered with a second therapeutic agent, or with a second type of stem cell, the AMDACs and second therapeutic agent and/or second type of stem cell can be administered at the same time or different times, *e.g.,* the administrations can take place within 1, 2, 3, 4, 5, 6, 7, 8, 9 10, 20, 30, 40, or 50 minutes of each other, or 1, 2, 3, 4, 5, 6, 7, 8, 9, 10, 12, 14, 16, 18, 20, or 22 hours of each other, or within 1, 2, 3, 4, 5, 6, 7 8, 9 or 10 days or more of each other.

### 5.3 CHARACTERISTICS OF AMNION DERIVED ADHERENT CELLS

The AMDACs, useful in treating pain according to the invention are obtainable from the amniotic membrane by a two-step isolation procedure described below; adhere to a cell culture surface, *e.g.,* to tissue culture plastic; are OCT-4⁻, as determinable by RT-PCR; and display some or all of the characteristics listed below. AMDACs are described in U.S. Patent Application Publication No. 2010/0124569.

AMDACs display cellular markers that distinguish them from other amnion-derived, or placenta-derived, cells. For example, in one embodiment, the AMDACs are OCT-4⁻, as determinable by RT-PCR, and CD49f⁺, as determinable by immunolocalization. In another specific embodiment, said OCT-4⁻ AMDACs are HLA-G⁻, as determinable by RT-PCR. In another specific embodiment, the OCT-4⁻ AMDACs are VEGFR1/Flt-1⁺ (vascular endothelial growth factor receptor 1) and/or VEGFR2/KDR⁺ (vascular endothelial growth factor receptor 2), as determinable by immunolocalization. In a specific embodiment, the OCT-4⁻ AMDACs, or a population of OCT-4⁻ AMDACs, expresses at least 2 log less PCR-amplified mRNA for OCT-4 at, *e.g.,* 20 cycles, than a population of NTERA-2 cells having an equivalent number of cells and at an equivalent number of RNA amplification cycles. In another specific embodiment, said OCT-4⁻ AMDACs are CD90⁺, CD105⁺, or CD117⁻, as determinable by immunolocalization. In a more specific embodiment, said OCT-4⁻ AMDACs are CD90⁺, CD105⁺, and CD117⁻, as determinable by immunolocalization. In another specific embodiment, the AMDACs are OCT-4⁻ or HLA-G⁻, and are additionally CD49f⁺, CD90⁺, CD105⁺, and CD117⁻, as determinable by immunolocalization. In a more specific embodiment, the AMDACs are OCT-4⁻, HLA-G⁻, CD49f⁺, CD90⁺, CD105⁺, and CD117⁻, as determinable by immunolocalization. In another specific embodiment, the OCT-4⁻ AMDACs do not express SOX2, *e.g.,* as determinable by RT-PCR for 30 cycles. In a specific embodiment, therefore, the AMDACs are OCT-4⁻, CD49f⁺, CD90⁺, CD105⁺, and CD117⁻, as determinable by immunolocalization, and SOX2⁻, as determinable by RT-PCR, *e.g.,* for 30 cycles.

In another embodiment, said OCT-4⁻ AMDACs are one or more of CD29⁺, CD73⁺, ABC-p⁺, and CD38⁻, as determinable by immunolocalization. In a more specific embodiment, said OCT-4⁻ AMDACs are CD29⁺, CD73⁺, ABC-p⁺, and CD38⁻, as determinable by immunolocalization.

In another specific embodiment, for example, OCT-4⁻ AMDACs can additionally be one or more of CD9⁺, CD10⁺, CD44⁺, CD54⁺, CD98⁺, TEM-7⁺ (tumor endothelial marker 7), CD31⁻, CD34⁻, CD45⁻, CD133⁻, CD143⁻ (angiotensin-I-converting enzyme, ACE), CD146⁻ (melanoma cell adhesion molecule), or CXCR4⁻ (chemokine (C-X-C motif) receptor 4) as determinable by immunolocalization, or HLA-G⁻ as determinable by RT-PCR. In a more specific embodiment, said cell is CD9⁺, CD10⁺, CD44⁺, CD54⁺, CD98⁺, Tie-2⁺, TEM-7⁺, CD31⁻, CD34⁻, CD45⁻, CD133⁻, CD143⁻, CD146⁻, and CXCR4⁻ as determinable by immunolocalization, and HLA-G⁻ as determinable by RT-PCR. In certain embodiments, the AMDACs are one or more of CD31⁻, CD34⁻, CD45⁻, and/or CD133⁻. In a specific embodiment, the AMDACs are OCT-4⁻, as determinable by RT-PCR; VEGFR1/Flt-1⁺ and/or VEGFR2/KDR⁺, as determinable by immunolocalization; and one or more, or all, of CD31⁻, CD34⁻, CD45⁻, and/or CD133⁻.

In another specific embodiment, said AMDACs are additionally VE-cadherin as determinable by immunolocalization. In another specific embodiment, said AMDACs are additionally positive for CD105⁺ and CD200⁺ as determinable by immunolocalization. In another specific embodiment, said AMDACs do not express CD34 as detected by immunolocalization after exposure to 1 to 100 ng/mL VEGF for 4 to 21 days. In more specific embodiments, said AMDACs do not express CD34 as detected by immunolocalization after exposure to 25 to 75 ng/mL VEGF for 4 to 21 days, or to 50 ng/mL VEGF for 4 to 21 days. In even more specific embodiments, said AMDACs do not express CD34 as detected by immunolocalization after exposure to 1, 2.5, 5, 10, 25, 50, 75 or 100 ng/mL VEGF for 4 to 21 days, *e.g.,* in culture. In yet more specific embodiments, said AMDACs do not express CD34 as detected by immunolocalization after exposure to 1 to 100 ng/mL VEGF for 7 to 14, *e.g.,* 7, days, *e.g.,* in culture.

In specific embodiments, the AMDACs are OCT-4⁻, as determined by RT-PCR, and one or more of VE-cadherin⁻, VEGFR2/KDR⁺, CD9⁺, CD54⁺, CD105⁺, and/or CD200⁺ as determinable by immunolocalization. In a specific embodiment, the AMDACs are OCT-4⁻, as determinable by RT-PCR, and VE-cadherin⁻, VEGFR2/KDR⁺, CD9⁺, CD54⁺, CD105⁺, and CD200⁺ as determinable by immunolocalization. In another specific embodiment, said AMDACs do not express CD34, as detected by immunolocalization, *e.g.,* after exposure to 1 to 100 ng/mL VEGF for 4 to 21 days.

In another embodiment, the AMDACs are OCT-4⁻, CD49f⁺, HLA-G⁻, CD90⁺, CD105⁺, and CD117⁻, as determinable by immunolocalization. In a more specific embodiment, said AMDACs are one or more of CD9⁺, CD10⁺, CD44⁺, CD54⁺, CD98⁺, Tie-2⁺, TEM-7⁺, CD31⁻, CD34⁻, CD45⁻, CD133⁻, CD143⁻, CD146⁻, or CXCR4⁻, as determinable by immunolocalization. In a more specific embodiment, said AMDACs are CD9⁺, CD10⁺, CD44⁺, CD54⁺, CD98⁺, Tie-2⁺, TEM-7⁺, CD31⁻, CD34⁻, CD45⁻, CD133⁻, CD143⁻, CD146⁻, and CXCR4⁻ as determinable by immunolocalization. In another specific embodiment, said AMDACs are additionally VEGFR1/Flt-1⁺ and/or VEGFR2/KDR⁺, as determinable by immunolocalization; and one or more of CD31⁻, CD34⁻, CD45⁻, CD133⁻, and/or Tie-2⁻ as determinable by immunolocalization. In another specific embodiment, said AMDACs are additionally VEGFR1/Flt-1⁺, VEGFR2/KDR⁺, CD31⁻, CD34⁻, CD45⁻, CD133⁻, and Tie-2⁻ as determinable by immunolocalization.

In another embodiment, the OCT-4⁻ AMDACs are additionally one or more, or all, of CD9⁺, CD10⁺, CD44⁺, CD49f⁺, CD54⁺, CD90⁺, CD98⁺, CD105⁺, CD200⁺, Tie-2⁺, TEM-7⁺, VEGFR1/Flt-1⁺, and/or VEGFR2/KDR⁺ (CD309⁺), as determinable by immunolocalization; or additionally one or more, or all, of CD31⁻, CD34⁻, CD38⁻, CD45⁻, CD117⁻, CD133⁻, CD143⁻, CD144⁻, CD146⁻, CD271⁻, CXCR4⁻, HLA-G⁻, and/or VE-cadherin⁻, as determinable by immunolocalization, or SOX2⁻, as determinable by RT-PCR.

In certain embodiments, the AMDACs are CD49f⁺. In a specific embodiment, said CD49f⁺ AMDACs are additionally one or more, or all, of CD9⁺, CD10⁺, CD44⁺, CD54⁺, CD90⁺, CD98⁺, CD105⁺, CD200⁺, Tie-2⁺, TEM-7⁺, VEGFR1/Flt-1⁺, and/or VEGFR2/KDR⁺ (CD309⁺), as determinable by immunolocalization; or additionally one or more, or all, of CD31⁻, CD34⁻, CD38⁻, CD45⁻, CD117⁻, CD133⁻, CD143⁻, CD144⁻, CD146⁻, CD271⁻, CXCR4⁻, HLA-G⁻, OCT-4⁻ and/or VE-cadherin⁻, as determinable by immunolocalization, or SOX2⁻, as determinable by RT-PCR.

In certain other embodiments, the AMDACs are HLA-G⁻, CD90⁺, and CD117⁻. In a specific embodiment, said HLA-G⁻, CD90⁺, and CD117⁻ cells are additionally one or more, or all, of CD9⁺, CD10⁺, CD44⁺, CD49f⁺, CD54⁺, CD98⁺, CD105⁺, CD200⁺, Tie-2⁺, TEM-7⁺, VEGFR1/Flt-1⁺, and/or VEGFR2/KDR⁺ (CD309⁺), as determinable by immunolocalization; or additionally one or more, or all, of CD31⁻, CD34⁻, CD38⁻, CD45⁻,CD133⁻, CD143⁻, CD144⁻, CD146⁻, CD271⁻, CXCR4⁻, OCT-4⁻ and/or VE-cadherin, as determinable by immunolocalization, or SOX2⁻, as determinable by RT-PCR.

In another embodiment, the AMDACs do not constitutively express mRNA for fibroblast growth factor 4 (FGF4), interferon γ (IFNG), chemokine (C-X-C motif) ligand 10 (CXCL10), angiopoietin 4 (ANGPT4), angiopoietin-like 3 (ANGPTL3), fibrinogen α chain (FGA), leptin (LEP), prolactin (PRL), prokineticin 1 (PROK1), tenomodulin (TNMD), FMS-like tyrosine kinase 3 (FLT3), extracellular link domain containing 1 (XLKD1), cadherin 5, type 2 (CDH5), leukocyte cell derived chemotaxin 1 (LECT1), plasminogen (PLG), telomerase reverse transcriptase (TERT), (sex determining region Y)-box 2 (SOX2), NANOG, matrix metalloprotease 13 (MMP-13), distal-less homeobox 5 (DLX5), and/or bone gamma-carboxyglutamate (gla) protein (BGLAP), as determinable by RT-PCR, *e.g.,* for 30 cycles, after said cells have been cultured under standard culture conditions. In other embodiments, the AMDACs express mRNA for (ARNT2), nerve growth factor (NGF), brain-derived neurotrophic factor (BDNF), glial-derived neurotrophic factor (GDNF), neurotrophin 3 (NT-3), NT-5, hypoxia-Inducible Factor 1α (HIF1A), hypoxia-inducible protein 2 (HIG2), heme oxygenase (decycling) 1 (HMOX1), Extracellular superoxide dismutase [Cu-Zn] (SOD3), catalase (CAT), transforming growth factor β1 (TGFB1), transforming growth factor β1 receptor (TGFB1R), and hepatoycte growth factor receptor (HGFR/c-met), as determinable by RT-PCR, *e.g.,* for 30 cycles, after said cells have been cultured under standard culture conditions.

In another aspect, provided herein are isolated populations of cells comprising the AMDACs described herein, wherein said isolated population of cells is not an amnion or amniotic membrane. The populations of cells can be homogeneous populations, *e.g.,* a population of cells, at least about 90%, 95%, 98% or 99% of which are AMDACs, *e.g.,* the AMDACs as described by any combination of markers as described herein. In certain other embodiments, the populations of cells can be heterogeneous, *e.g.,* a population of cells wherein at most about 10%, 20%, 30%, 40%, 50%, 60%, 70% or 80% of the cells in the population are AMDACs. The isolated populations of cells are not, however, tissue, i.e., amniotic membrane or a portion thereof.

In one embodiment, the isolated AMDACs are provided, *e.g.,* administered, in an isolated population of cells comprising the AMDACs, *e.g.,* a population of cells substantially homogeneous for AMDACs, wherein said AMDACs are adherent to a tissue culture surface, *e.g.,* tissue culture plastic, and wherein said AMDACs are OCT-4⁻, as determinable by RT-PCR. In a specific embodiment, the AMDACs are CD49f⁺ or HLA-G⁺, *e.g.,* as determinable by immunolocalization or RT-PCR. In another specific embodiment, said population of AMDACs is VEGFR1/Flt-1⁺ and/or VEGFR2/KDR⁺ as determinable by immunolocalization. In a more specific embodiment, the AMDACs are OCT-4⁻, and/or HLA-G⁻ as determinable by RT-PCR, and VEGFR1/Flt-1⁺ and/or VEGFR2/KDR⁺ as determinable by immunolocalization. In another specific embodiment, said AMDACs are CD90⁺, CD105⁺, or CD117⁻, as determinable by immunolocalization. In a more specific embodiment, said AMDACs are CD90⁺, CD105⁺, and CD117⁻, as determinable by immunolocalization. In a more specific embodiment, the AMDACs are OCT-4⁻, CD49f⁺, CD90⁺, CD105⁺, and CD117⁻, as determinable by immunolocalization. In another specific embodiment, the AMDACs do not express SOX2, *e.g.,* as determinable by RT-PCR for 30 cycles. In an even more specific embodiment, the population comprises AMDACs, wherein said AMDACs are OCT-4⁻, HLA-G⁻, CD49f⁺, CD90⁺, CD105⁺, and CD117⁻, as determinable by immunolocalization, and SOX2⁻, *e.g.,* as determinable by RT-PCR for 30 cycles

In another specific embodiment, said AMDACs in said population of cells are CD90⁺, CD105⁺, or CD117⁻, as determinable by immunolocalization. In a more specific embodiment, the AMDACs are CD90⁺, CD105⁺, and CD117⁻, as determinable by immunolocalization. In a more specific embodiment, the AMDACs are OCT-4⁻ or HLA-G⁻, *e.g.,* as determinable by RT-PCR, and are additionally CD49f⁺, CD90⁺, CD105⁺, and CD117⁻ as determinable by immunolocalization. In a more specific embodiment, the AMDACs in said population of cells are OCT-4⁻, HLA-G⁻, CD49f⁺, CD90⁺, CD105⁺, and CD117⁻. In another specific embodiment, the AMDACs do not express SOX2, *e.g.,* as determinable by RT-PCR for 30 cycles. In a more specific embodiment, therefore, the AMDACs are OCT-4⁻, CD49f⁺, CD90⁺, CD105⁺, and CD117⁻, as determinable by immunolocalization, and SOX2⁻, as determinable by RT-PCR, *e.g.,* for 30 cycles. In an even more specific embodiment, the AMDACs are OCT-4⁻ or HLA-G⁻, and are additionally CD49f⁺, CD90⁺, CD105⁺, and CD117⁻, as determinable by immunolocalization. In a more specific embodiment, the AMDACs are OCT-4⁻, HLA-G⁻, CD49f⁺, CD90⁺, CD105⁺, and CD117⁻, as determinable by immunolocalization.

In another embodiment, the AMDACs in said population of cells are adherent to tissue culture plastic, OCT-4⁻ as determinable by RT-PCR, and VEGFR1/Flt-1⁺ and/or VEGFR2/KDR⁺ as determinable by immunolocalization, and are additionally one or more of CD9⁺, CD10⁺, CD44⁺, CD54⁺, CD98⁺, Tie-2⁺, TEM-7⁺, CD31⁻, CD34⁻, CD45⁻, CD133⁻, CD143⁻, CD146⁻, or CXCR4⁻, as determinable by immunolocalization, or HLA-G⁻ as determinable by RT-PCR. In another embodiment, the isolated population of cells comprises AMDACs, wherein said AMDACs are adherent to tissue culture plastic, wherein said AMDACs are OCT-4⁻ as determinable by RT-PCR, and VEGFR1/Flt-1⁺ and/or VEGFR2/KDR⁺ as determinable by immunolocalization, wherein said AMDACs do not express CD34 as detected by immunolocalization after exposure to 1 to 100 ng/mL VEGF for 4 to 21 days, and wherein said isolated population of cells is not an amnion or a portion thereof. In a specific embodiment of any of the above embodiments, at least about 50%, 60%, 70%, 80%, 90%, 95%, 98% or 99% of cells in said population are said AMDACs.

In another embodiment, any of the above populations of cells comprising AMDACs forms sprouts or tube-like structures when cultured in the presence of an extracellular matrix protein, *e.g.,* like collagen type I and IV, or an angiogenic factor, *e.g.,* like vascular endothelial growth factor (VEGF), epithelial growth factor (EGF), platelet derived growth factor (PDGF) or basic fibroblast growth factor (bFGF), *e.g.,* in or on a substrate such as placental collagen, *e.g.,* or MATRIGEL™ for at least 4 days and up to 14 days.

AMDACs display characteristic expression of angiogenesis-related or cardiomyogenesis-related genes. In certain embodiments, the AMDACs express RNA for one or more of, or all of, ACTA2 (actin, alpha 2, smooth muscle, aorta), ADAMTS1 (ADAM metallopeptidase with thrombospondin type 1 motif, 1), AMOT (angiomotin), ANG (angiogenin), ANGPT1 (angiopoietin 1), ANGPT2, ANGPTL1 (angiopoietin-like 1), ANGPTL2, ANGPTL4, BAI1 (brain-specific angiogenesis inhibitor 1), CD44, CD200, CEACAM1 (carcinoembryonic antigen-related cell adhesion molecule 1), CHGA (chromogranin A), COL15A1 (collagen, type XV, alpha 1), COL18A1 (collagen, type XVIII, alpha 1), COL4A1 (collagen, type IV, alpha 1), COL4A2 (collagen, type IV, alpha 2), COL4A3 (collagen, type IV, alpha 3), CSF3 (colony stimulating factor 3 (granulocyte), CTGF (connective tissue growth factor), CXCL12 (chemokine (CXC motif) ligand 12 (stromal cell-derived factor 1)), CXCL2, DNMT3B (DNA (cytosine-5-)-methyltransferase 3 beta), ECGF1 (thymidine phosphorylase), EDG1 (endothelial cell differentiation gene 1), EDIL3 (EGF-like repeats and discoidin I-like domains 3), ENPP2 (ectonucleotide pyrophosphatase/phosphodiesterase 2), EPHB2 (EPH receptor B2), FBLN5 (FIBULIN 5), F2 (coagulation factor II (thrombin)), FGF1 (acidic fibroblast growth factor), FGF2 (basic fibroblast growth factor), FIGF (c-fos induced growth factor (vascular endothelial growth factor D)), FLT4 (fms-related tyrosine kinase 4), FN1 (fibronectin 1), FST (follistatin), FOXC2 (forkhead box C2 (MFH-1, mesenchyme forkhead 1)), GRN (granulin), HGF (hepatocyte growth factor), HEY1 (hairy/enhancer-of-split related with YRPW motif 1), HSPG2 (heparan sulfate proteoglycan 2), IFNB1 (interferon, beta 1, fibroblast), IL8 (interleukin 8), IL12A, ITGA4 (integrin, alpha 4; CD49d), ITGAV (integrin, alpha V), ITGB3 (integrin, beta 3), MDK (midkine), MMP2 (matrix metalloprotease 2), MYOZ2 (myozenin 2), NRP1 (neuropilin 1), NRP2, PDGFB (platelet-derived growth factor β), PDGFRA (platelet-derived growth factor receptor α), PDGFRB, PECAM1 (platelet/endothelial cell adhesion molecule), PF4 (platelet factor 4), PGK1 (phosphoglycerate kinase 1), PROX1 (prospero homeobox 1), PTN (pleiotrophin), SEMA3F (semophorin 3F), SERPINB5 (serpin peptidase inhibitor, clade B (ovalbumin), member 5), SERPINC1, SERPINF1, TIMP2 (tissue inhibitor of metalloproteinases 2), TIMP3, TGFA (transforming growth factor, alpha), TGFB1, THBS1 (thrombospondin 1), THBS2, TIE1 (tyrosine kinase with immunoglobulin-like and EGF-like domains 1), TIE2/TEK, TNF (tumor necrosis factor), TNNI1 (troponin I, type 1), TNFSF15 (tumor necrosis factor (ligand) superfamily, member 15), VASH1 (vasohibin 1), VEGF (vascular endothelial growth factor), VEGFB, VEGFC, VEGFR1/FLT1 (vascular endothelial growth factor receptor 1), and/or VEGFR2/KDR.

When human cells are used, the gene designations throughout refer to human sequences, and, as is well known to persons of skill in the art, representative sequences can be found in literature, or in GenBank. Probes to the sequences can be determined by sequences that are publicly-available, or through commercial sources, *e.g.,* specific TAQMAN® probes or TAQMAN® Angiogenesis Array (Applied Biosystems, part no. 4378710).

AMDACs display characteristic expression of (*e.g*., production of) angiogenesis-related proteins. In certain embodiments, the AMDACs express CD49d, Connexin-43, HLA-ABC, Beta 2-microglobulin, CD349, CD318, PDL1, CD106, Galectin-1, ADAM 17 precursor (A disintegrin and metalloproteinase domain 17) (TNF-alpha converting enzyme) (TNF-alpha convertase), Angiotensinogen precursor, Filamin A (Alpha-filamin) (Filamin 1) (Endothelial actin-binding protein) (ABP-280) (Nonmuscle filamin), Alpha-actinin 1 (Alpha-actinin cytoskeletal isoform) (Non-muscle alpha-actinin 1) (F-actin cross linking protein), Low-density lipoprotein receptor-related protein 2 precursor (Megalin) (Glycoprotein 330) (gp330), Macrophage scavenger receptor types I and II (Macrophage acetylated LDL receptor I and II), Activin receptor type IIB precursor (ACTR-IIB), Wnt-9 protein, Glial fibrillary acidic protein, astrocyte (GFAP), Myosin-binding protein C, cardiac-type (Cardiac MyBP-C) (C-protein, cardiac muscle isoform), and/or Myosin heavy chain, nonmuscle type A (Cellular myosin heavy chain, type A) (Nonmuscle myosin heavy chain-A) (NMMHC-A).

AMDACs further secrete proteins that promote angiogenesis, *e.g.,* in populations of endothelial cells, endothelial progenitor cells, or the like. In certain embodiments, the AMDACs or population of cells comprising AMDACs, *e.g.,* wherein at least about 50%, 60%, 70%, 80%, 90%, 95% or 98% of cells in said isolated population of cells are AMDACs, secrete one or more, or all, of VEGF, HGF, IL-8, MCP-3, FGF2, Follistatin, G-CSF, EGF, ENA-78, GRO, IL-6, MCP-1, PDGF-BB, TIMP-2, uPAR, Galectin-1, *e.g.,* into culture medium in which the AMDACs are grown.

In another embodiment, any of the above AMDACs can cause the formation of sprouts or tube-like structures in a population of endothelial cells in contact with said AMDACs. In a specific embodiment, the AMDACs can be co-cultured with human endothelial cells, forming sprouts or tube-like structures, or supporting the endothelial cell sprouts, *e.g.,* when cultured in the presence of extracellular matrix proteins such as collagen type I and IV, and/or angiogenic factors such as vascular endothelial growth factor (VEGF), epithelial growth factor (EGF), platelet derived growth factor (PDGF) or basic fibroblast growth factor (bFGF), *e.g.,* in or on a substrate such as placental collagen or MATRIGEL™ for at least 4 days and/or up to 14 days.

In another embodiment, any of the above AMDACs or populations of cells comprising AMDACs secrete angiogenic factors such as vascular endothelial growth factor (VEGF), epithelial growth factor (EGF), platelet derived growth factor (PDGF), basic fibroblast growth factor (bFGF), or Interleukin-8 (IL-8), and thereby can induce human endothelial cells to form sprouts or tube-like structures, *e.g.,* when cultured in the presence of extracellular matrix proteins such as collagen type I and IV *e.g.,* in or on a substrate such as placental collagen or MATRIGEL™.

In another embodiment, AMDACs for use according to the invention are a population of AMDACs, or a population of cells wherein at least about 50%, 60%, 70%, 80%, 90%, 95% or 98% of cells in said isolated population of cells are AMDACs that express angiogenic micro RNAs (miRNAs) at a higher level than bone marrow-derived mesenchymal stem cells, wherein said miRNAs comprise one or more, or all of, miR-17-3p, miR-18a, miR-18b, miR-19b, miR-92, and/or miR-296. In another embodiment, a population of AMDACs, or a population of cells wherein at least about 50%, 60%, 70%, 80%, 90%, 95% or 98% of cells in said isolated population of cells are AMDACs that express one or more of, or all of, angiogenic micro RNAs (miRNAs) at a lower level than bone marrow-derived mesenchymal stem cells, wherein said miRNAs comprise one or more, or all of, miR-20a, miR-20b, miR-221, miR-222, miR-15b, and/or miR-16. In certain embodiments, AMDACs, or populations of AMDACs, express one or more, or all, of the angiogenic miRNAs miR-17-3p, miR-18a, miR-18b, miR-19b, miR-92, miR-20a, miR-20b, (members of the of the angiogenic miRNA cluster 17-92), miR-296, miR-221, miR-222, miR-15b, and/or miR-16.

In certain embodiments, the AMDACs useful in the treatment of an individual having pain are adherent to tissue culture plastic, and wherein said AMDACs are OCT-4⁻, as determinable by RT-PCR, and CD49f⁺, HLA-G⁻, CD90⁺, CD105⁺, and CD117⁻, as determinable by immunolocalization, and wherein said AMDACs: (a) express one or more of CD9, CD10, CD44, CD54, CD98, CD200, Tie-2, TEM-7, VEGFR1/Flt-1, or VEGFR2/KDR (CD309), as determinable by immunolocalization; (b) lack expression of CD31, CD34, CD38, CD45, CD133, CD143, CD144, CD146, CD271, CXCR4, HLA-G, or VE-cadherin, as determinable by immunolocalization, or lack expression of SOX2, as determinable by RT-PCR; (c) express mRNA for ACTA2, ADAMTS1, AMOT, ANG, ANGPT1, ANGPT2, ANGPTL1, ANGPTL2, ANGPTL4, BAI1, CD44, CD200, CEACAM1, CHGA, COL15A1, COL18A1, COL4A1, COL4A2, COL4A3, CSF3, CTGF, CXCL12, CXCL2, DNMT3B, ECGF1, EDG1, EDIL3, ENPP2, EPHB2, FBLN5, F2, FGF1, FGF2, FIGF, FLT4, FN1, FST, FOXC2, GRN, HGF, HEY1, HSPG2, IFNB1, IL8, IL12A, ITGA4, ITGAV, ITGB3, MDK, MMP2, MYOZ2, NRP1, NRP2, PDGFB, PDGFRA, PDGFRB, PECAM1, PF4, PGK1, PROX1, PTN, SEMA3F, SERPINB5, SERPINC1, SERPINF1, TIMP2, TIMP3, TGFA, TGFB1, THBS1, THBS2, TIE1, TIE2/TEK, TNF, TNNI1, TNFSF15, VASH1, VEGF, VEGFB, VEGFC, VEGFR1/FLT1, or VEGFR2/KDR; (d) express one or more of the proteins CD49d, Connexin-43, HLA-ABC, Beta 2-microglobulin, CD349, CD318, PDL1, CD106, Galectin-1, ADAM 17, angiotensinogen precursor, filamin A, alpha-actinin 1, megalin, macrophage acetylated LDL receptor I and II, activin receptor type IIB precursor, Wnt-9 protein, glial fibrillary acidic protein, astrocyte, myosin-binding protein C, or myosin heavy chain, nonmuscle type A; (e) secrete VEGF, HGF, IL-8, MCP-3, FGF2, Follistatin, G-CSF, EGF, ENA-78, GRO, IL-6, MCP-1, PDGF-BB, TIMP-2, uPAR, or galectin-1 into culture medium in which the cell grows; (f) express micro RNAs miR-17-3p, miR-18a, miR-18b, miR-19b, miR-92, or miR-296 at a higher level than an equivalent number of bone marrow-derived mesenchymal stem cells; (g) express micro RNAs miR-20a, miR-20b, miR-221, miR-222, miR-15b, or miR-16 at a lower level than an equivalent number of bone marrow-derived mesenchymal stem cells; (h) express miRNAs miR-17-3p, miR-18a, miR-18b, miR-19b, miR-92, miR-20a, miR-20b, miR-296, miR-221, miR-222, miR-15b, or miR-16; and/or (i) express increased levels of CD202b, IL-8 or VEGF when cultured in less than about 5% O₂, compared to expression of CD202b, IL-8 or VEGF under 21% O₂.

In a specific embodiment, the AMDACs are OCT-4⁻, as determinable by RT-PCR, and CD49f⁺, HLA-G⁻, CD90⁺, CD105⁺, and CD117⁻, as determinable by immunolocalization, and (a) express CD9, CD10, CD44, CD54, CD90, CD98, CD200, Tie-2, TEM-7, VEGFR1/Flt-1, and/or VEGFR2/KDR (CD309), as determinable by immunolocalization; (b) lack expression of CD31, CD34, CD38, CD45, CD133, CD143, CD144, CD146, CD271, CXCR4, HLA-G, and/or VE-cadherin, as determinable by immunolocalization, or lacks expression of SOX2, as determinable by RT-PCR; (c) express mRNA for ACTA2, ADAMTS1, AMOT, ANG, ANGPT1, ANGPT2, ANGPTL1, ANGPTL2, ANGPTL4, BAI1, CD44, CD200, CEACAM1, CHGA, COL15A1, COL18A1, COL4A1, COL4A2, COL4A3, CSF3, CTGF, CXCL12, CXCL2, DNMT3B, ECGF1, EDG1, EDIL3, ENPP2, EPHB2, FBLN5, F2, FGF1, FGF2, FIGF, FLT4, FN1, FST, FOXC2, GRN, HGF, HEY1, HSPG2, IFNB1, IL8, IL12A, ITGA4, ITGAV, ITGB3, MDK, MMP2, MYOZ2, NRP1, NRP2, PDGFB, PDGFRA, PDGFRB, PECAM1, PF4, PGK1, PROX1, PTN, SEMA3F, SERPINB5, SERPINC1, SERPINF1, TIMP2, TIMP3, TGFA, TGFB1, THBS1, THBS2, TIE1, TIE2/TEK, TNF, TNNI1, TNFSF15, VASH1, VEGF, VEGFB, VEGFC, VEGFR1/FLT1, and/or VEGFR2/KDR; (d) express one or more of CD49d, Connexin-43, HLA-ABC, Beta 2-microglobulin, CD349, CD318, PDL1, CD106, Galectin-1, ADAM 17, angiotensinogen precursor, filamin A, alpha-actinin 1, megalin, macrophage acetylated LDL receptor I and II, activin receptor type IIB precursor, Wnt-9 protein, glial fibrillary acidic protein, astrocyte, myosin-binding protein C, and/or myosin heavy chain, nonmuscle type A; (e) secrete VEGF, HGF, IL-8, MCP-3, FGF2, Follistatin, G-CSF, EGF, ENA-78, GRO, IL-6, MCP-1, PDGF-BB, TIMP-2, uPAR, and/or Galectin-1, *e.g.,* into culture medium in which the cell grows; (f) express micro RNAs miR-17-3p, miR-18a, miR-18b, miR-19b, miR-92, and/or miR-296 at a higher level than an equivalent number of bone marrow-derived mesenchymal stem cells; (g) express micro RNAs miR-20a, miR-20b, miR-221, miR-222, miR-15b, and/or miR-16 at a lower level than an equivalent number of bone marrow-derived mesenchymal stem cells; (h) express miRNAs miR-17-3p, miR-18a, miR-18b, miR-19b, miR-92, miR-20a, miR-20b, miR-296, miR-221, miR-222, miR-15b, and/or miR-16; and/or (i) express increased levels of CD202b, IL-8 and/or VEGF when cultured in less than about 5% O₂, compared to expression of CD202b, IL-8 and/or VEGF under 21% O₂. Further provided herein are populations of cells comprising AMDACs, *e.g.* populations of AMDACs, having one or more of the above-recited characteristics.

In another embodiment, any of the above AMDACs, or populations of cells comprising AMDACs, secretes angiogenic factors. In specific embodiments, the AMDACs or population of cells comprising AMDACs secrete vascular endothelial growth factor (VEGF), epithelial growth factor (EGF), platelet derived growth factor (PDGF), basic fibroblast growth factor (bFGF), and/or interleukin-8 (IL-8). In other specific embodiments, the AMDACs or population of cells comprising AMDACs secretes one or more angiogenic factors and thereby induces human endothelial cells to migrate in an *in vitro* wound healing assay. In other specific embodiments, the AMDACs or population of cells comprising AMDACs induces maturation, differentiation or proliferation of human endothelial cells, endothelial progenitors, myocytes or myoblasts.

In another embodiment, any of the above AMDACs or populations of cells comprising AMDACs take up acetylated low density lipoprotein (LDL) when cultured in the presence of extracellular matrix proteins, *e.g.,* collagen type I or IV, and/or one or more angiogenic factors, *e.g.,* VEGF, EGF, PDGF, or bFGF, *e.g.,* on a substrate such as placental collagen or MATRIGEL™.

In another embodiment, a population of cells useful for treating pain, comprises AMDACs, wherein said AMDACs are adherent to tissue culture plastic, and wherein said cells are OCT-4⁻, as determinable by RT-PCR, and VEGFR2/KDR⁺, CD9⁺, CD54⁺, CD105⁺, CD200⁺, or VE-cadherin⁻, as determinable by immunolocalization. In specific embodiments, at least 10%, 20%, 30%, 40%, 50%, 60%, 70%, 80%, 90%, 95%, 98% or 99% of the cells in said population of cells are AMDACs that are OCT-4⁻, as determinable by RT-PCR, and VEGFR2/KDR⁺, CD9⁺, CD54⁺, CD105⁺, CD200⁺, or VE-cadherin⁻, as determinable by immunolocalization. In another specific embodiment, at least 10%, 20%, 30%, 40%, 50%, 60%, 70%, 80%, 90%, 95%, 98% or 99% of the cells in said population are AMDACs that are OCT-4⁻, as determinable by RT-PCR, and VEGFR2/KDR⁺, CD9⁺, CD54⁺, CD105⁺, CD200⁺, and VE-cadherin⁻, as determinable by immunolocalization. In another specific embodiment, said AMDACs that are OCT-4⁻, as determinable by RT-PCR, and VEGFR2/KDR⁺, CD9⁺, CD54⁺, CD105⁺, CD200⁺, or VE-cadherin⁻, as determinable by immunolocalization, do not express CD34, as detected by immunolocalization, after exposure to 1 to 100 ng/mL VEGF for 4 to 21 days. In another specific embodiment, said cells are also VE-cadherin⁻.

The populations of cells comprising AMDACs, are able to form sprouts or tube-like structures resembling vessels or vasculature. In one embodiment, the populations of cells comprising AMDACs form sprouts or tube-like structures when cultured in the presence of an angiogenic moiety, *e.g.,* VEGF, EGF, PDGF or bFGF. In a more specific embodiment, said AMDACs that are OCT-4⁻, as determinable by RT-PCR, and VEGFR2/KDR⁺, CD9⁺, CD54⁺, CD105⁺, CD200⁺, or VE-cadherin , as determinable by immunolocalization, form sprouts or tube-like structures when said population of cells is cultured in the presence of vascular endothelial growth factor (VEGF).

The AMDACs described herein display the above characteristics, *e.g.,* combinations of cell surface markers and/or gene expression profiles, and/or angiogenic potency and function, in primary culture, or during proliferation in medium suitable for the culture of stem cells. Such medium includes, for example, medium comprising 1 to 100% DMEM-LG (Gibco), 1 to 100% MCDB-201(Sigma), 1 to 10% fetal calf serum (FCS) (Hyclone Laboratories), 0.1 to 5x insulin-transferrin-selenium (ITS, Sigma), 0.1 to 5x linolenic-acid-bovine-serum-albumin (LA-BSA, Sigma), 10⁻⁵ to 10⁻¹⁵ M dexamethasone (Sigma), 10⁻² to 10⁻¹⁰ M ascorbic acid 2-phosphate (Sigma), 1 to 50 ng/mL epidermal growth factor (EGF), (R&D Systems), 1 to 50 ng/mL platelet derived-growth factor (PDGF-BB) (R&D Systems), and 100U penicillin/1000U streptomycin. In a specific embodiment, the medium comprises 60% DMEM-LG (Gibco), 40% MCDB-201(Sigma), 2% fetal calf serum (FCS) (Hyclone Laboratories), 1x insulin-transferrin-selenium (ITS), 1x linolenic-acid-bovine-serum-albumin (LA-BSA), 10⁻⁹ M dexamethasone (Sigma), 10⁻⁴M ascorbic acid 2-phosphate (Sigma), epidermal growth factor (EGF)10 ng/ml (R&D Systems), platelet derived-growth factor (PDGF-BB) 10 ng/ml (R&D Systems), and 100U penicillin/1000U streptomycin. Other suitable media are described below.

Isolated populations of AMDACs, suitable for treating individuals having pain, can comprise about, at least about, or no more than about, 1 x 10⁵, 5 x 10⁵, 1 x 10⁶, 5 x 10⁶, 1 x 10⁷, 5 x 10⁷, 1 x 10⁸, 5 x 10⁸, 1 x 10⁹, 5 x 10⁹, 1 x 10¹⁰, 5 x 10¹⁰, 1 x 10¹¹ or more AMDACs, *e.g.,* in a container. In various embodiments, at least 10%, 20%, 30%, 40%, 50%, 60%, 70%, 80%, 90%, 95%, or 99% of the cells in the isolated cell populations provided herein are AMDACs. That is, a population of isolated AMDACs can comprise, *e.g.,* as much as 1%, 5%, 10%, 20%, 30%, 40%, 50%, 60%, 70%, 80%, 90% non-stem cells.

The AMDACs can be cultured on a substrate. In various embodiments, the substrate can be any surface on which culture and/or selection of amnion derived adherent cells, can be accomplished. Typically, the substrate is plastic, *e.g.,* tissue culture dish or multiwell plate plastic. Tissue culture plastic can be treated, coated or imprinted with a biomolecule or synthetic mimetic agent, *e.g.,* CELLSTART™, MESENCULT™ ACF-substrate, ornithine, or polylysine, or an extracellular matrix protein, *e.g.,* collagen, laminin, fibronectin, vitronectin, or the like.

AMDACs can be isolated from one or more placentas. Isolated AMDACs can be cultured and expanded to produce populations of AMDACs. Populations of placental cells comprising amnion derived adherent cells can also be cultured and expanded to produce populations of amnion derived adherent cells.

In certain embodiments, AMDACs displaying any of the above marker and/or gene expression characteristics have been passaged at least 1, 2, 3, 4, 5, 6, 7, 8, 9, 10, 11, 12, 13, 14, 15, 16, 17, 18, 19 or 20 times, or more. In certain other embodiments, AMDACs displaying any of the above marker and/or gene expression characteristics have been doubled in culture at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or at least 50 times, or more.

In preparation for administration to an individual who is experiencing pain, the AMDACs can be cultured for a plurality of passages. The growth of the AMDACs described herein, as for any mammalian cell, depends in part upon the particular medium selected for growth. Under optimum conditions, AMDACs typically double in number in approximately 24 hours. During culture, the AMDACs described herein adhere to a substrate in culture, e.g. the surface of a tissue culture container (e.g., tissue culture dish plastic, fibronectin-coated plastic, and the like) and form a monolayer. Typically, the cells establish in culture within 2-7 days after digestion of the amnion. They typically proliferate at approximately 0.4 to 1.2 population doublings per day and can undergo at least 30 to 50 population doublings. The cells display a mesenchymal/fibroblastic cell-like phenotype during subconfluence and expansion, and a cuboidal/cobblestone-like appearance at confluence, and proliferation in culture is strongly contact-inhibited. Populations of AMDACs can form embryoid bodies during expansion in culture.

### 5.4 METHODS OF OBTAINING AMDACS

The amnion derived adherent cells, and populations of cells comprising the AMDACs, useful for treating individuals experiencing pain, can be produced, *e.g.,* isolated from other cells or cell populations, for example, through particular methods of digestion of amnion tissue, optionally followed by assessment of the resulting cells or cell population for the presence or absence of markers, or combinations of markers, characteristic of AMDACs, or by obtaining amnion cells and selecting on the basis of markers characteristic of AMDACs, as described above.

The AMDACs, and isolated populations of cells comprising the AMDACs, can be produced by, *e.g.,* specific methods of digestion of amnion tissue followed by selection for adherent cells. AMDACs are preferably isolated using a two-step digestion method, *e.g.,* the method described in detail in Section 4.3.3, below. In one embodiment, for instance, isolated AMDACs, or an isolated population of cells comprising AMDACs, can be produced by (1) digesting amnion tissue with a first enzyme to dissociate cells from the epithelial layer of the amnion from cells from the mesenchymal layer of the amnion; (2) subsequently digesting the mesenchymal layer of the amnion with a second enzyme to form a single-cell suspension; (3) culturing cells in said single-cell suspension on a tissue culture surface, *e.g.,* tissue culture plastic; and (4) selecting cells that adhere to said surface after a change of medium, thereby producing an isolated population of cells comprising amnion derived adherent cells, *e.g.,* a population of AMDACs. In a specific embodiment, said first enzyme is trypsin. In a more specific embodiment, said trypsin is used at a concentration of 0.25% trypsin (w/v), in 5-20 mL, *e.g.,* 10 milliliters solution per gram of amnion tissue to be digested. In another more specific embodiment, said digesting with trypsin is allowed to proceed for about 15 minutes at 37°C and is repeated up to three times. In another specific embodiment, said second enzyme is collagenase. In a more specific embodiment, said collagenase is used at a concentration between 50 and 500 U/L in 5 mL per gram of amnion tissue to be digested. In another more specific embodiment, said digesting with collagenase is allowed to proceed for about 45-60 minutes at 37°C. In another specific embodiment, the single-cell suspension formed after digestion with collagenase is filtered through, *e.g.,* a 75 µM - 150 µM filter between step (2) and step (3). In another specific embodiment, said first enzyme is trypsin, and said second enzyme is collagenase. The resulting cell population is an amnion cell population comprising AMDACs, *e.g.,* a population of AMDACs.

An isolated population of cells comprising amnion derived adherent cells can, in another embodiment, be obtained by selecting cells from amnion, *e.g.,* cells obtained by digesting amnion tissue as described elsewhere herein, that display one or more characteristics of an amnion derived adherent cell (AMDAC), and optionally separating such cells from other amnion cells, to produce a population of AMDACs. In one embodiment, for example, a cell population is produced by a method comprising selecting amnion cells that are (a) negative for OCT-4, as determinable by RT-PCR, and (b) positive for one or more of VEGFR2/KDR, CD9, CD54, CD105, CD200, as determinable by flow cytometry or flow cytometry or immunolocalization, and optionally separating such cells from other amnion cells, to produce a population of AMDACs. In a specific embodiment, said amnion cells are additionally VE-cadherin⁻. In a specific embodiment, a cell population is produced by selecting placental cells that are (a) negative for OCT-4, as determinable by RT-PCR, and VE-cadherin, as determinable by flow cytometry or immunolocalization, and (b) positive for each of VEGFR2/KDR, CD9, CD54, CD105, CD200, as determinable by flow cytometry or immunolocalization, and optionally separating such cells from other amnion cells, to produce a population of AMDACs. In certain embodiments, said selection by flow cytometry or immunolocalization is performed before said selection by RT-PCR. In another specific embodiment, said selecting comprises selecting cells that do not express cellular marker CD34 after culture for 4 to 21 days in the presence of 1 to 100 ng/mL VEGF.

In another embodiment, for example, a cell population is produced by a method comprising selecting amnion cells that are adherent to tissue culture plastic and are OCT-4⁻, as determinable by RT-PCR, and VEGFR1/Flt-1⁺ and VEGFR2/KDR⁺, as determinable by flow cytometry or immunolocalization, and isolating said cells from other cells to form a cell population, *e.g.,* a population of AMDACs. In a specific embodiment, a cell population is produced by a method comprising selecting amnion cells that are OCT-4⁻, as determinable by RT-PCR, and VEGFR1/Flt-1⁺, VEGFR2/KDR ⁺, and HLA-G⁻, as determinable by flow cytometry or immunolocalization, and isolating said cells from other cells to form a cell population, *e.g.,* a population of AMDACs. In another specific embodiment, said cell population is produced by selecting amnion cells that are additionally one or more, or all, of CD9⁺, CD10⁺, CD44⁺, CD54⁺, CD98⁺, Tie-2⁺, TEM-7⁺, CD31⁻, CD34⁻, CD45⁻, CD133⁻, CD143⁻, CD146⁻, and/or CXCR4⁻ (chemokine (C-X-C motif) receptor 4) as determinable by flow cytometry or immunolocalization, and isolating the cells from other cells to form a population of AMDACs. In another specific embodiment, said cell population, *e.g.,* population of AMDACs, is produced by selecting amnion cells that are additionally VE-cadherin⁻ as determinable by flow cytometry or immunolocalization, and isolating the cells from cells that are VE-cadherin⁺. In another specific embodiment, said cell population is produced by selecting amnion cells that are additionally CD105⁺ and CD200⁺ as determinable by immunolocalization, and isolating the cells from cells that are CD105⁻ or CD200⁻. In another specific embodiment, said cell does not express CD34 as detected by immunolocalization after exposure to 1 to 100 ng/mL VEGF for 4 to 21 days.

In the selection of cells, it is not necessary to test an entire population of cells for characteristics specific to AMDACs. Instead, one or more aliquots of cells (*e.g.*, about 0.5% - 2%) of a population of cells may be tested for such characteristics, and the results can be attributed to the remaining cells in the population.

Selected cells can be confirmed to be the amnion derived adherent cells provided herein by culturing a sample of the cells (*e.g.,* about 10⁴ to about 10⁵ cells) on a substrate, *e.g.,* MATRIGEL™, for 4 to 14, *e.g.,* 7, days in the presence of VEGF (*e.g.,* about 50 ng/mL), and visually inspecting the cells for the appearance of sprouts and/or cellular networks.

AMDACs can be selected by the above markers using any method known in the art of cell selection. For example, AMDACs can be selected using an antibody or antibodies to one or more cell surface markers, for example, in immunolocalization, *e.g.,* flow cytometry or FACS. Selection can be accomplished using antibodies in conjunction with magnetic beads. Antibodies that are specific for certain markers are known in the art and are available commercially, *e.g.,* antibodies to CD9 (Abcam); CD54 (Abcam); CD105 (Abcam; BioDesign International, Saco, ME, *etc.*); CD200 (Abcam) cytokeratin (SigmaAldrich). Antibodies to other markers are also available commercially, *e.g.,* CD34, CD38 and CD45 are available from, *e.g.,* StemCell Technologies or BioDesign International. Primers to OCT-4 sequences suitable for RT-PCR can be obtained commercially, *e.g.,* from Millipore or Invitrogen, or can be readily derived from the human sequence in GenBank Accession No. DQ486513.

Detailed methods of obtaining placenta and amnion tissue, and treating such tissue in order to obtain AMDACs, are provided below.

### 5.4.1 Cell Collection Composition

AMDACs can be obtained from amnion from a mammalian placenta, *e.g.,* a human placenta, using a physiologically-acceptable solution, *e.g.,* a cell collection composition. Preferably, the cell collection composition prevents or suppresses apoptosis, prevents or suppresses cell death, lysis, decomposition and the like. A cell collection composition is described in detail in related U.S. Patent Application Publication No. 2007/0190042, entitled "Improved Medium for Collecting Placental Stem Cells and Preserving Organs." The cell collection composition can comprise any physiologically-acceptable solution suitable for the collection and/or culture of AMDACs, for example, a saline solution (*e.g*., phosphate-buffered saline, Kreb's solution, modified Kreb's solution, Eagle's solution, 0.9% NaCl. *etc*.), a culture medium (*e.g.,* DMEM, H.DMEM, *etc.*), and the like, with or without the addition of a buffering component, *e.g.,* 4-(2-hydroxyethyl)-1-piperazineethanesulfonic acid (HEPES).

The cell collection composition can comprise one or more components that tend to preserve cells, *e.g.,* AMDACs, that is, prevent the cells from dying, or delay the death of the cells, reduce the number of cells in a population of cells that die, or the like, from the time of collection to the time of culturing. Such components can be, *e.g.,* an apoptosis inhibitor (*e.g.,* a caspase inhibitor or JNK inhibitor); a vasodilator (*e.g*., magnesium sulfate, an antihypertensive drug, atrial natriuretic peptide (ANP), adrenocorticotropin, corticotropin-releasing hormone, sodium nitroprusside, hydralazine, adenosine triphosphate, adenosine, indomethacin or magnesium sulfate, a phosphodiesterase inhibitor, *etc*.); a necrosis inhibitor (*e.g.,* 2-(1H-Indol-3-yl)-3-pentylamino-maleimide, pyrrolidine dithiocarbamate, or clonazepam); a TNF-α inhibitor; and/or an oxygen-carrying perfluorocarbon (*e.g.*, perfluorooctyl bromide, perfluorodecyl bromide, *etc.*).

The cell collection composition can comprise a bacteriocidally or bacteriostatically effective amount of an antibiotic. In certain non-limiting embodiments, the antibiotic is a macrolide (*e.g.,* tobramycin), a cephalosporin (*e.g.,* cephalexin, cephradine, cefuroxime, cefprozil, cefaclor, cefixime or cefadroxil), a clarithromycin, an erythromycin, a penicillin (*e.g*., penicillin V) or a quinolone (*e.g*., ofloxacin, ciprofloxacin or norfloxacin), a tetracycline, a streptomycin, *etc.* In a particular embodiment, the antibiotic is active against Gram(+) and/or Gram(-) bacteria, *e.g., Pseudomonas aeruginosa, Staphylococcus aureus,* and the like.

The cell collection composition can also comprise one or more of the following compounds: adenosine (about 1 mM to about 50 mM); D-glucose (about 20 mM to about 100 mM); magnesium ions (about 1 mM to about 50 mM); a macromolecule of molecular weight greater than 20,000 daltons, in one embodiment, present in an amount sufficient to maintain endothelial integrity and cellular viability (*e.g*., a synthetic or naturally occurring colloid, a polysaccharide such as dextran or a polyethylene glycol present at about 25 g/l to about 100 g/l, or about 40 g/l to about 60 g/l); an antioxidant (*e.g*., butylated hydroxyanisole, butylated hydroxytoluene, glutathione, vitamin C or vitamin E present at about 25 µM to about 100 µM); a reducing agent (*e.g*., N-acetylcysteine present at about 0.1 mM to about 5 mM); an agent that prevents calcium entry into cells (*e.g*., verapamil present at about 2 µM to about 25 µM); nitroglycerin (*e.g*., about 0.05 g/L to about 0.2 g/L); an anticoagulant, in one embodiment, present in an amount sufficient to help prevent clotting of residual blood (*e.g*., heparin or hirudin present at a concentration of about 1000 units/l to about 100,000 units/l); or an amiloride containing compound (*e.g*., amiloride, ethyl isopropyl amiloride, hexamethylene amiloride, dimethyl amiloride or isobutyl amiloride present at about 1.0 µM to about 5 µM).

The AMDACs described herein can also be collected, *e.g.,* during and after digestion as described below, into a simple physiologically-acceptable buffer, *e.g.,* phosphate-buffered saline, a 0.9% NaCl solution, cell culture medium, or the like.

### 5.4.2 Collection and Handling of Placenta

Generally, a human placenta is recovered shortly after its expulsion after birth, or after, *e.g.,* Caesarian section. Preferably, the placenta is recovered from a patient after informed consent and after a complete medical history of the patient is obtained and is associated with the placenta. Preferably, the medical history continues after delivery. Such a medical history can be used to coordinate subsequent use of the placenta or cells harvested therefrom. For example, AMDACs can be used, in light of the medical history, for personalized medicine for the infant, or a close relative, associated with the placenta, or for parents, siblings, or other relatives of the infant. In other embodiments, the AMDACs may be used for autologous recipients, optionally based in their medical histories.

Prior to recovery of AMDACs from the amnion, umbilical cord blood and placental blood are preferably removed from the placenta comprising the amnion. In certain embodiments, after delivery, the cord blood in the placenta is recovered. The placenta can be subjected to a conventional cord blood recovery process. Typically a needle or cannula is used, with the aid of gravity, to exsanguinate the placenta (*see, e.g.,* Anderson, U.S. Patent No. 5,372,581; Hessel et al., U.S. Patent No. 5,415,665). The needle or cannula is usually placed in the umbilical vein and the placenta can be gently massaged to aid in draining cord blood from the placenta. Such cord blood recovery may be performed commercially, *e.g*., LifeBank USA, Cedar Knolls, N.J., ViaCord, Cord Blood Registry and Cryocell. Preferably, the placenta is gravity drained without further manipulation so as to minimize tissue disruption during cord blood recovery.

Typically, a placenta is transported from the delivery or birthing room to another location, *e.g.,* a laboratory, for recovery of cord blood and collection of cells by, *e.g.,* perfusion or tissue dissociation. The placenta is preferably transported in a sterile, thermally insulated transport device (maintaining the temperature of the placenta between 20-28°C), for example, by placing the placenta, with clamped proximal umbilical cord, in a sterile zip-lock plastic bag, which is then placed in an insulated container. In another embodiment, the placenta is transported in a cord blood collection kit substantially as described in United States Patent No. 7,147,626. Preferably, the placenta is delivered to the laboratory four to twenty-four hours following delivery. In certain instances, the proximal umbilical cord is clamped, preferably within 4-5 cm (centimeter) of the insertion into the placental disc prior to cord blood recovery. In other instances, the proximal umbilical cord is clamped after cord blood recovery but prior to further processing of the placenta.

The placenta, prior to cell collection, can be stored under sterile conditions and at a temperature of, *e.g.,* 4 to 25°C (centigrade), *e.g.,* at room temperature. The placenta may be stored for, *e.g.,* a period of for zero to twenty-four hours, up to forty-eight hours, or longer than forty eight hours, prior to perfusing the placenta to remove any residual cord blood. In one embodiment, the placenta is harvested from between about zero hours to about two hours post-expulsion. The placenta can be stored in an anticoagulant solution at a temperature of, *e.g.,* 4 to 25°C (centigrade). Suitable anticoagulant solutions are well known in the art. For example, a solution of sodium citrate, heparin or warfarin sodium can be used. Preferably, the anticoagulant solution comprises a solution of heparin (*e.g.*, 1% w/w in 1:1000 solution). The exsanguinated placenta is preferably stored for no more than 36 hours before cells are collected.

### 5.4.3 Physical Disruption and Enzymatic Digestion of Amnion Tissue

The amnion is separated from the rest of the placenta, *e.g.,* by blunt dissection, *e.g.,* using the fingers. The amnion can be dissected, *e.g.,* into parts or tissue segments, prior to enzymatic digestion and adherent cell recovery. AMDACs can be obtained from a whole amnion, or from a small segment of amnion, *e.g*., a segment of amnion that is about 1, 2, 3, 4, 5, 6, 7, 8, 9, 10, 20, 30, 40, 50, 60, 70, 80, 90, 100, 200, 300, 400, 500, 600, 700, 800, 900 or about 1000 square millimeters in area.

AMDACs can generally be collected from a placental amnion or a portion thereof, at any time within about the first three days post-expulsion, but preferably between about 0 hours and 48 hours after expulsion, or about 8 hours and about 18 hours post-expulsion.

AMDACs may be extracted from amnion tissue by enzymatic digestion using one or more tissue-digesting enzymes, preferably the sequential combination of trypsin and collagenase as outlined above. The amnion, or a portion thereof, may, *e.g.,* be digested with one or more enzymes dissolved or mixed into a cell collection composition as described above. In one instance, for example, the amnion tissue is digested three times with trypsin and then once with collagenase.

Typical concentrations for tissue digestion enzymes include, *e.g.,* 50-200 U/mL for collagenase I and collagenase IV, 1-10 U/mL for dispase, and 10-100 U/mL for elastase. Proteases can be used in combination, that is, two or more proteases in the same digestion reaction, or can be used sequentially in order to isolate amnion derived adherent cells. For example, amnion tissue, or part thereof, is digested first with an appropriate amount of trypsin, at a concentration of about 0.25%, for, e.g., 15 minutes, at 37°C, followed by collagenase I at about 1 to about 2 mg/ml for, *e.g.,* 45 minutes.

In one example, amnion derived adherent cells are obtainable as follows. The amniotic membrane is cut into segments approximately 0.1" x 0.1" to about 5" x 5", *e.g.,* 2" x 2", in size. The epithelial monolayer is removed from the fetal side of the amniotic membrane by triple trypsinization as follows. The segments of amniotic membrane are placed into a container with warm (*e.g.*, about 20°C to about 37°C) trypsin-EDTA solution (0.25%). The volume of trypsin can range from about 5 mL per gram of amnion to about 50 mL per gram of amnion. The container is agitated for about 5 minutes to about 30 minutes, *e.g.,* 15 minutes, while maintaining the temperature constant. The segments of amniotic membrane are then separated from the trypsin solution by any appropriate method, such as manually removing the amnion segments, or by filtration. The trypsinization step can be repeated at least one more time.

Upon completion of the final trypsinization, the segments of amniotic membrane are placed back into the container filled with warm trypsin neutralization solution, such as phosphate-buffered saline (PBS)/10% FBS, PBS/5% FBS or PBS/3% FBS. The container is agitated for about 5 seconds to about 30 minutes, *e.g.,* 5 minutes. The segments of amniotic membrane are then separated from the trypsin neutralization solution as described above, and the segments of amniotic membrane are placed into the container filled with warm PBS, pH 7.2. The container is agitated for about 5 seconds to about 30 minutes, and the amniotic membrane segments are then separated from the PBS as described above.

The segments of amniotic membrane are then placed into the container filled with warm (e.g., about 20°C to about 37°C) digestion solution. The volume of digestion solution can range from about 5 mL per gram of amnion to about 50 mL per gram of amnion. Digestion solutions comprise digestion enzymes in an appropriate culture medium, such as DMEM. Typical digestion solutions include collagenase type I (about 50 U/mL to about 500 U/mL); collagenase type I (about 50 U/mL to about 500 U/mL) plus dispase (about 5 U/mL to about 100 U/mL); and collagenase type I (about 50 U/mL to about 500 U/mL), dispase (about 2 U/mL to about 50 U/mL) and hyaluronidase (about 3 U/mL to about 10 U/ mL). The container is agitated at 37°C until amnion digestion is substantially complete (approximately 10 minutes to about 90 minutes). Warm PBS/5% FBS is then added to the container at a ratio of about 1 mL per gram of amniotic tissue to about 50 mL per gram of amniotic tissue. The container is agitated for about 2 minutes to about 5 minutes. The cell suspension is then filtered to remove any un-digested tissue using a 40 µm to 100 µm filter. The cells are suspended in warm PBS (about 1 mL to about 500 mL), and then centrifuged at 200 x *g* to about 400 x *g* for about 5 minutes to about 30 minutes, *e.g*. 300 x g for about 15 minutes at 20°C. After centrifugation, the supernatant is removed and the cells are resuspended in a suitable culture medium. The cell suspension can be filtered (40 µm to 70 µm filter) to remove any remaining undigested tissue, yielding a single cell suspension.

In this instance, cells in suspension are collected and cultured as described elsewhere herein to produce isolated amnion derived adherent cells, and populations of such cells. The remaining undigested amnion, in this embodiment, can be discarded. The cells released from the amnion tissue can be, *e.g.,* collected, *e.g.,* by centrifugation, and cultured in standard cell culture medium.

In any of the digestion protocols herein, the cell suspension obtained by digestion can be filtered, *e.g*., through a filter comprising pores from about 50 µm to about 150 µm, *e.g.,* from about 75 µm to about 125 µm. In a more specific case, the cell suspension can be filtered through two or more filters, *e.g.,* a 125 µm filter and a 75 µm filter.

In conjunction with any of the methods described herein, AMDACs can be isolated from the cells released during digestion by selecting cells that express one or more markers characteristic of AMDACs, as described in Section 5.5, above.

AMDACs can also, for example, be isolated using a specific two-step isolation method comprising digestion with trypsin followed by digestion with collagenase. Thus, a method of isolating AMDACs comprises digesting an amniotic membrane or portion thereof with trypsin such that epithelial cells are released from said amniotic membrane; removing the amniotic membrane or portion thereof from said epithelial cells; further digesting the amniotic membrane or portion thereof with collagenase such that amnion derived adherent cells are released from said amniotic membrane or portion thereof; and separating said amnion derived adherent cells from said amniotic membrane. Digestion of the amniotic membrane or portion thereof may be repeated at least once. Alternatively, digestion of the amniotic membrane or portion thereof with collagenase is repeated at least once. The trypsin may be at about 0.1%-1.0% (final concentration). More specifically, the trypsin is at about 0.25% (final concentration). The collagenase may be at about 50 U/mL to about 1000 U/mL (final concentration). More specifically, the collagenase is at about 125 U/mL (final concentration). The method of isolation may additionally comprises culturing said AMDACs in cell culture and separating said AMDACs from non-adherent cells in said culture to produce an enriched population of AMDACs. For example, at least 50%, 55%, 60%, 65%, 70%, 75%, 80%, 85%, 90%, 95%, 98% or 99% of cells in said enriched population of AMDACs are said AMDACs.

The amnion derived adherent cells for use according to the invention are negative for OCT-4, as determinable by RT-PCRT, and one or more of HLA-G⁺, CD90⁺, CD105⁺, and CD117⁻, as determinable by immunolocalization, and/or have any of the other characteristics listed in Section 4.3.2, above.

### 5.4.4 Isolation, Sorting, and Characterization of Amnion Derived Adherent Cells

Cell pellets can be resuspended in fresh cell collection composition, as described above, or a medium suitable for cell maintenance, *e.g*., Dulbecco's Modified Eagle's Medium (DMEM); Iscove's Modified Dulbecco's Medium (IMDM), *e.g.* IMDM serum-free medium containing 2U/mL heparin and 2 mM EDTA (GibcoBRL, NY); a mixture of buffer (*e.g*. PBS, HBSS) with FBS (*e.g.* 2% v/v); or the like.

AMDACs that have been cultured, *e.g.,* on a surface, *e.g.,* on tissue culture plastic, with or without additional extracellular matrix coating such as fibronectin, can be passaged or isolated by differential adherence. For example, a cell suspension obtained from collagenase digestion of amnion tissue, performed as described above, can be cultured, *e.g*., for 3-7 days in culture medium on tissue culture plastic. During culture, a plurality of cells in the suspension adhere to the culture surface, and, after continued culture, give rise to AMDACs. Nonadherent cells, which do not give rise to the AMDACs, are removed during medium exchange.

The number and type of cells collected from amnion can be monitored, for example, by measuring changes in morphology and cell surface markers using standard cell detection techniques such as immunolocalization, *e.g*., flow cytometry, cell sorting, immunocytochemistry (*e.g*., staining with tissue specific or cell-marker specific antibodies) fluorescence activated cell sorting (FACS), magnetic activated cell sorting (MACS), by examination of the morphology of cells using light or confocal microscopy, and/or by measuring changes in gene expression using techniques well known in the art, such as PCR and gene expression profiling. These techniques can be used, too, to identify cells that are positive for one or more particular markers. For example, using one or more antibodies to CD34, one can determine, using the techniques above, whether a cell comprises a detectable amount of CD34; if so, the cell is CD34⁺.

Amnion-derived cells, *e.g.,* cells that have been isolated by Ficoll separation, differential adherence, or a combination of both, can be sorted, *e.g*., further isolated, using a fluorescence activated cell sorter (FACS). Fluorescence activated cell sorting (FACS) is a well-known method for separating particles, including cells, based on the fluorescent properties of the particles *(see, e.g.,* Kamarch, 1987, Methods Enzymol, 151:150-165). Laser excitation of fluorescent moieties in the individual particles results in a small electrical charge allowing electromagnetic separation of positive and negative particles from a mixture. In one embodiment, cell surface marker-specific antibodies or ligands are labeled with distinct fluorescent labels. Cells are processed through the cell sorter, allowing separation of cells based on their ability to bind to the antibodies used. FACS sorted particles may be directly deposited into individual wells of 96-well or 384-well plates to facilitate separation and cloning.

In one sorting scheme, cells from placenta, *e.g*., AMDACs, can be sorted on the basis of expression of the markers CD49f, VEGFR2/KDR, and/or Flt-1/VEGFR1. Preferably the cells are identified as being OCT-4⁻, *e.g.,* by determining the expression of OCT-4 by RT-PCR in a sample of the cells, wherein the cells are OCT-4⁻ if the cells in the sample fail to show detectable production of mRNA for OCT-4 after 30 cycles. For example, cells from amnion that are VEGFR2/KDR⁺ and VEGFR1/Flt-1⁺ can be sorted from cells that are one or more of VEGFR2/KDR⁻, and VEGFR1/Flt-1⁺, CD9⁺, CD54⁺, CD105⁺, CD200⁺, and/or VE-cadherin⁻. In a specific embodiment, amnion-derived, tissue culture plastic-adherent cells that are one or more of CD49f⁺, VEGFR2/KDR⁺, CD9⁺, CD54⁺, CD105⁺, CD200⁺, and/or VE-cadherin⁻, or cells that are VEGFR2/KDR⁺, CD9⁺, CD54⁺, CD105⁺, CD200⁺, and VE-cadherin⁻, are sorted away from cells not expressing one or more of such marker(s), and selected. In another specific embodiment, CD49f⁺, VEGFR2/KDR⁺, VEGFR1/Flt-1⁺ cells that are additionally one or more, or all, of CD31⁺, CD34⁺, CD45⁺, CD133⁻, and/or Tie-2⁺ are sorted from cells that do not display one or more, or any, of such characteristics. In another specific embodiment, VEGFR2/KDR⁺, VEGFR1/Flt-1⁺ cells that are additionally one or more, or all, of CD9⁺, CD10⁺, CD44⁺, CD54⁺, CD98⁺, Tie-2⁺, TEM-7⁺, CD31⁻, CD34⁻, CD45⁻, CD133⁻, CD143⁻, CD146⁻, and/or CXCR4⁻, are sorted from cells that do not display one or more, or any, of such characteristics.

Selection for AMDACs can be performed on a cell suspension resulting from digestion, or on isolated cells collected from digestate, *e.g*., by centrifugation or separation using flow cytometry. Selection by expressed markers can be accomplished alone or, *e.g*., in connection with procedures to select cells on the basis of their adherence properties in culture. For example, an adherence selection can be accomplished before or after sorting on the basis of marker expression.

With respect to antibody-mediated detection and sorting of amnion cells, *e.g*. AMDACs, any antibody, specific for a particular marker, can be used, in combination with any fluorophore or other label suitable for the detection and sorting of cells (*e.g*., fluorescence-activated cell sorting). Antibody/fluorophore combinations to specific markers include, but are not limited to, fluorescein isothiocyanate (FITC) conjugated monoclonal antibodies against CD105 (available from R&D Systems Inc., Minneapolis, Minnesota); phycoerythrin (PE) conjugated monoclonal antibodies against CD200 (BD Biosciences Pharmingen); VEGFR2/KDR-Biotin (CD309, Abcam), and the like. Antibodies to any of the markers disclosed herein can be labeled with any standard label for antibodies that facilitates detection of the antibodies, including, *e.g*., horseradish peroxidase, alkaline phosphatase, β-galactosidase, acetylcholinesterase streptavidin/biotin, avidin/biotin, umbelliferone, fluorescein, fluorescein isothiocyanate (FITC), rhodamine, dichlorotriazinylamine fluorescein, dansyl chloride or phycoerythrin (PE), luminol, luciferase, luciferin, and aequorin, and examples of suitable radioactive material include ¹²⁵I, ¹³¹I, ³⁵S or ³H.

AMDACs can be labeled with an antibody to a single marker and detected and/sorted based on the single marker, or can be simultaneously labeled with multiple antibodies to a plurality of different markers and sorted based on the plurality of markers.

In another embodiment, magnetic beads can be used to separate cells, *e.g.,* to separate the amnion derived adherent cells described herein from other amnion cells. The cells may be sorted using a magnetic activated cell sorting (MACS) technique, a method for separating particles based on their ability to bind magnetic beads (0.5-100 µm diameter). A variety of useful modifications can be performed on the magnetic microspheres, including covalent addition of antibody that specifically recognizes a particular cell surface molecule or hapten. The beads are then mixed with the cells to allow binding. Cells are then passed through a magnetic field to separate out cells having the specific cell surface marker. In one embodiment, these cells can then isolated and re-mixed with magnetic beads coupled to an antibody against additional cell surface markers. The cells are again passed through a magnetic field, isolating cells that bound both the antibodies. Such cells can then be diluted into separate dishes, such as microtiter dishes for clonal isolation.

AMDACs can be assessed for viability, proliferation potential, and longevity using standard techniques known in the art, such as trypan blue exclusion assay, fluorescein diacetate uptake assay, propidium iodide uptake assay (to assess viability); and thymidine uptake assay or MTT cell proliferation assay (to assess proliferation). Longevity may be determined by methods well known in the art, such as by determining the maximum number of population doubling in an extended culture.

AMDACs can also be separated from other placental cells using other techniques known in the art, *e.g*., selective growth of desired cells (positive selection), selective destruction of unwanted cells (negative selection); separation based upon differential cell agglutinability in the mixed population as, for example, with soybean agglutinin; freeze-thaw procedures; filtration; conventional and zonal centrifugation; centrifugal elutriation (counter-streaming centrifugation); unit gravity separation; countercurrent distribution; electrophoresis; and the like.

### 5.5 CULTURE OF AMDACS

### 5.5.1 Culture Media

Isolated AMDACs, or populations of such cells, can be used to initiate, or seed, cell cultures. Cells are generally transferred to sterile tissue culture vessels either uncoated or coated with extracellular matrix or biomolecules such as laminin, collagen (*e.g*., native or denatured), gelatin, fibronectin, ornithine, vitronectin, and extracellular membrane protein (*e.g*., MATRIGEL™ (BD Discovery Labware, Bedford, Mass.)).

AMDACs can, for example, be established in media suitable for the culture of stem cells, Establishment media can, for example, include EGM-2 medium (Lonza), DMEM + 10% FBS, or medium comprising 60% DMEM-LG (Gibco), 40% MCDB-201(Sigma), 2% fetal calf serum (FCS) (Hyclone Laboratories), IX insulin-transferrin-selenium (ITS), IX lenolenic-acid-bovine-serum-albumin (LA-BSA), 10⁻⁹ M dexamethasone (Sigma), 10⁻⁴M ascorbic acid 2-phosphate (Sigma), epidermal growth factor (EGF) 10 ng/ml (R&D Systems), platelet derived-growth factor (PDGF-BB) 10 ng/ml (R&D Systems), and 100 U penicillin/1000 U streptomycin (referred to herein as "standard medium").

AMDACs can be cultured in any medium, and under any conditions, recognized in the art as acceptable for the culture of cells, *e.g*., adherent placental stem cells. Preferably, the culture medium comprises serum. In various embodiments, media for the culture or subculture of AMDACs includes STEMPRO® (Invitrogen), MSCM-sf (ScienCell, Carlsbad, CA), MESENCULT®-ACF medium (StemCell Technologies, Vancouver, Canada), standard medium, standard medium lacking EGF, standard medium lacking PDGF, DMEM + 10% FBS, EGM-2 (Lonza), EGM-2MV (Lonza), 2%, 10% and 20% ES media, ES-SSR medium, or α-MEM-20%FBS. Medium acceptable for the culture of amnion derived adherent cells includes, *e.g*., DMEM, IMDM, DMEM (high or low glucose), Eagle's basal medium, Ham's F10 medium (F10), Ham's F-12 medium (F12), Iscove's modified Dulbecco's medium, Mesenchymal Stem Cell Growth Medium (MSCGM Lonza), ADVANCESTEM™ Medium (Hyclone), KNOCKOUT™ DMEM (Invitrogen), Leibovitz's L-15 medium, MCDB, DMEM/F12, RPMI 1640, advanced DMEM (Gibco), DMEM/MCDB201 (Sigma), and CELL-GRO FREE, or the like. In various embodiments, for example, DMEM-LG (Dulbecco's Modified Essential Medium, low glucose)/MCDB 201 (chick fibroblast basal medium) containing ITS (insulin-transferrin-selenium), LA+BSA (linoleic acid-bovine serum albumin), dextrose, L-ascorbic acid, PDGF, EGF, IGF-1, and penicillin/streptomycin; DMEM-HG (high glucose) comprising about 2 to about 20%, *e.g.,* about 10%, fetal bovine serum (FBS; *e.g.* defined fetal bovine serum, Hyclone, Logan Utah); DMEM-HG comprising about 2 to about 20%, *e.g.,* about 15%, FBS; IMDM (Iscove's modified Dulbecco's medium) comprising about 2 to about 20%, *e.g.,* about 10%, FBS, about 2 to about 20%, *e.g.,* about 10%, horse serum, and hydrocortisone; M199 comprising about 2 to about 20%, *e.g.,* about 10%, FBS, EGF, and heparin; α-MEM (minimal essential medium) comprising about 2 to about 20%, *e.g.,* about 10%, FBS, GLUTAMAX™ and gentamicin; DMEM comprising 10% FBS, GLUTAMAX™ and gentamicin; DMEM-LG comprising about 2 to about 20%, *e.g.,* about 15%, (v/v) fetal bovine serum (*e.g*., defined fetal bovine serum, Hyclone, Logan Utah), antibiotics/antimycotics (*e.g*., penicillin at about 100 Units/milliliter, streptomycin at 100 micrograms/milliliter, and/or amphotericin B at 0.25 micrograms/milliliter (Invitrogen, Carlsbad, Calif.)), and 0.001% (v/v) β-mercaptoethanol (Sigma, St. Louis Mo.); KNOCKOUT™-DMEM basal medium supplemented with 2 to 20% FBS, non-essential amino acid (Invitrogen), beta-mercaptoethanol, KNOCKOUT™ basal medium supplemented with KNOCKOUT™ Serum Replacement, alpha-MEM comprising 2 to 20% FBS, FBM2™ basal medium supplemented with EGF, VEGF, bFGF, R3-IGF-1, hydrocortisone, heparin, ascorbic acid, FBS, gentamicin), or the like.

The culture medium can be supplemented with one or more components including, for example, serum (*e.g.,* FCS or FBS, *e.g.,* about 2-20% (v/v); equine (horse) serum (ES); human serum (HS)); beta-mercaptoethanol (BME), preferably about 0.001% (v/v); one or more growth factors, for example, platelet-derived growth factor (PDGF), epidermal growth factor (EGF), basic fibroblast growth factor (bFGF), insulin-like growth factor-1 (IGF-1), leukemia inhibitory factor (LIF), vascular endothelial growth factor (VEGF), and erythropoietin (EPO); amino acids, including L-valine; and one or more antibiotic and/or antimycotic agents to control microbial contamination, such as, for example, penicillin G, streptomycin sulfate, amphotericin B, gentamicin, and nystatin, either alone or in combination.

AMDACs can be cultured in standard tissue culture conditions, *e.g*., in tissue culture dishes or multiwell plates. The cells can also be cultured using a hanging drop method. In this method, the cells are suspended at about 1 x 10⁴ cells per mL in about 5 mL of medium, and one or more drops of the medium are placed on the inside of the lid of a tissue culture container, *e.g*., a 100 mL Petri dish. The drops can be, *e.g.,* single drops, or multiple drops from, *e.g.,* a multichannel pipetter. The lid is carefully inverted and placed on top of the bottom of the dish, which contains a volume of liquid, *e.g*., sterile PBS sufficient to maintain the moisture content in the dish atmosphere, and the cells are cultured. AMDACs can also be cultured in standard or high-volume or high-throughput culture systems, such as T-flasks, Corning HYPERFLASK®, Cell Factories (Nunc), 1-, 2-, 4-, 10 or 40-Tray Cell stacks, and the like.

In one instance, AMDACs are cultured in the presence of a compound that acts to maintain an undifferentiated phenotype in the cells. In a specific instance, the compound is a substituted 3,4-dihydropyridimol[4,5-d]pyrimidine. In a more specific instance, the compound is a compound having the following chemical structure:

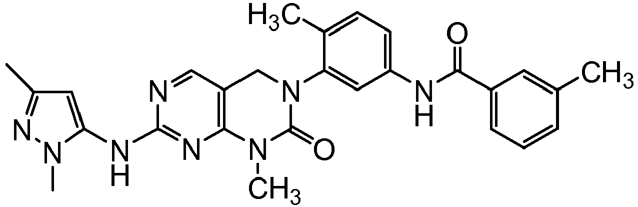

The compound can be contacted with an amnion derived adherent cell, or population of such cells, at a concentration of, for example, between about 1 µM to about 10 µM.

### 5.5.2 Expansion and Proliferation of Amnion Derived Adherent Cells

Once AMDACs, or a population of such cells (*e.g*., AMDACs, or population of AMDACs separated from at least 50% of the amnion cells with which the cell or population of cells is normally associated *in vivo*) are isolated, the cells can be proliferated and expanded *in vitro.* For example, a population of adherent cells or amnion derived adherent cells can be cultured in tissue culture containers, *e.g*., dishes, flasks, multiwell plates, or the like, for a sufficient time for the cells to proliferate to 40-70% confluence, that is, until the cells and their progeny occupy 40-70% of the culturing surface area of the tissue culture container.

AMDACs can be seeded in culture vessels at a density that allows cell growth. For example, the cells may be seeded at low density (*e.g*., about 400 to about 6,000 cells/cm²) to high density (*e.g*., about 20,000 or more cells/cm²). It is preferred that the cells are cultured at about 0% to about 5% by volume CO₂ in air. In some cases, the cells are cultured at about 0.1% to about 25% O₂ in air, preferably about 5% to about 20% O₂ in air. The cells are preferably cultured at about 25°C to about 40°C, preferably at about 37°C.

The cells are preferably cultured in an incubator. During culture, the culture medium can be static or can be agitated, for example, during culture using a bioreactor. Amnion derived adherent cells preferably are grown under low oxidative stress (*e.g*., with addition of glutathione, ascorbic acid, catalase, tocopherol, N-acetylcysteine, or the like).

Although the AMDACs may be grown to confluence, the cells are preferably not grown to confluence. For example, once 40%-70% confluence is obtained, the cells may be passaged. For example, the cells can be enzymatically treated, *e.g*., trypsinized, using techniques well-known in the art, to separate them from the tissue culture surface. After removing the cells by pipetting and counting the cells, about 20,000-100,000 cells, preferably about 50,000 cells, or about 400 to about 6,000 cells/cm², can be passaged to a new culture container containing fresh culture medium. Typically, the new medium is the same type of medium from which the cells were removed. The AMDACs can be passaged at least 1, 2, 3, 4, 5, 6, 7, 8, 9, 10, 11, 12, 13, 14, 15, 16, 17, 18, 19 or 20 times, or more. AMDACs can be doubled in culture at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or at least 50 times, or more.

### 5.6 PRESERVATION OF AMDACS

Amnion derived adherent cells can be preserved, that is, placed under conditions that allow for long-term storage, or conditions that inhibit cell death by, *e.g*., apoptosis or necrosis, *e.g.,* during collection or prior to production of the compositions described herein, *e.g.,* using the methods described herein.

AMDACs can be preserved using, *e.g.,* a composition comprising an apoptosis inhibitor, necrosis inhibitor and/or an oxygen-carrying perfluorocarbon, as described in U.S. Application Publication No. 2007/0190042. A method of preserving such cells, or a population of such cells, may comprise contacting said cells or population of cells with a cell collection composition comprising an inhibitor of apoptosis and an oxygen-carrying perfluorocarbon, wherein said inhibitor of apoptosis is present in an amount and for a time sufficient to reduce or prevent apoptosis in the population of cells, as compared to a population of cells not contacted with the inhibitor of apoptosis. Said inhibitor of apoptosis may be a caspase inhibitor,or a JNK inhibitor. In a more specific case, said JNK inhibitor does not modulate differentiation or proliferation of amnion derived adherent cells. Alternatively, said cell collection composition comprises said inhibitor of apoptosis and said oxygen-carrying perfluorocarbon in separate phases. Alternatively, said cell collection composition comprises said inhibitor of apoptosis and said oxygen-carrying perfluorocarbon in an emulsion. The cell collection composition may additionally comprise an emulsifier, *e.g.*, lecithin. Said apoptosis inhibitor and said perfluorocarbon may be between about 0°C and about 25°C at the time of contacting the cells. More specifically, said apoptosis inhibitor and said perfluorocarbon are between about 2°C and 10°C, or between about 2°C and about 5°C, at the time of contacting the cells. Said contacting may be performed during transport of said population of cells. Altenating, said contacting is performed during freezing and thawing of said population of cells.

Populations of AMDACs can be preserved, *e.g*., by a method comprising contacting a population of said cells with an inhibitor of apoptosis and an organ-preserving compound, wherein said inhibitor of apoptosis is present in an amount and for a time sufficient to reduce or prevent apoptosis in the population of cells, as compared to a population of cells not contacted with the inhibitor of apoptosis. In a specific embodiment, the organ-preserving compound is UW solution (described in U.S. Patent No. 4,798,824; also known as ViaSpan; *see also* Southard et al., Transplantation 49(2):251-257 (1990)) or a solution described in Stern et al., U.S. Patent No. 5,552,267. Said organ-preserving compound may be hydroxyethyl starch, lactobionic acid, raffinose, or a combination thereof. The cell collection composition may additionally comprises an oxygen-carrying perfluorocarbon, either in two phases or as an emulsion.

In another example, amnion derived adherent cells are contacted with a cell collection composition comprising an apoptosis inhibitor and oxygen-carrying perfluorocarbon, organ-preserving compound, or combination thereof, during perfusion. Alternatively, the amnion derived adherent cells are contacted with such a cell collection composition during a process of tissue disruption, *e.g*., enzymatic digestion of amnion tissue. Alternatively, amnion derived adherent cells are contacted with said cell collection compound after collection by tissue disruption, *e.g*., enzymatic digestion of amnion tissue.

Typically, during collection of AMDACs, enrichment and isolation, it is preferable to minimize or eliminate cell stress due to hypoxia and mechanical stress. In another embodiment of the method, therefore, AMDACs, or population of cells comprising the AMDACs, is exposed to a hypoxic condition during collection, enrichment or isolation for less than six hours during said preservation, wherein a hypoxic condition is a concentration of oxygen that is, *e.g.,* less than normal atmospheric oxygen concentration; less than normal blood oxygen concentration; or the like. More specifically, said cells or population of said cells is exposed to said hypoxic condition for less than two hours during said preservation. In another case, said cells or population of said cells is exposed to said hypoxic condition for less than one hour, or less than thirty minutes, or is not exposed to a hypoxic condition, during collection, enrichment or isolation. In another case, said population of cells is not exposed to shear stress during collection, enrichment or isolation.

AMDACs can be cryopreserved, in general or by the specific methods disclosed herein, *e.g.,* in cryopreservation medium in small containers, *e.g.,* ampoules. Suitable cryopreservation medium includes, but is not limited to, culture medium including, *e.g*., growth medium, or cell freezing medium, for example commercially available cell freezing medium, *e.g*., cell freezing medium identified by SigmaAldrich catalog numbers C2695, C2639 (Cell Freezing Medium-Serum-free IX, not containing DMSO) or C6039 (Cell Freezing Medium-Glycerol 1 X containing Minimum Essential Medium, glycerol, calf serum and bovine serum), Lonza PROFREEZE™ 2x Medium, methylcellulose, dextran, human serum albumin, fetal bovine serum, fetal calf serum, or Plasmalyte. Cryopreservation medium preferably comprises DMSO (dimethylsulfoxide) or glycerol, at a concentration of, *e.g.,* about 1% to about 20%, *e*.g., about 5% to 10% (v/v), optionally including fetal bovine serum or human serum. Cryopreservation medium may comprise additional agents, for example, methylcellulose and/or glycerol. Isolated amnion derived adherent cells are preferably cooled at about 1°C/min during cryopreservation. A preferred cryopreservation temperature is about -80°C to about -180°C, preferably about - 125°C to about -140°C. Cryopreserved cells can be transferred to vapor phase of liquid nitrogen prior to thawing for use. In some embodiments, for example, once the ampoules have reached about -80°C, they are transferred to a liquid nitrogen storage area. Cryopreservation can also be done using a controlled-rate freezer. Cryopreserved cells preferably are thawed at a temperature of about 25°C to about 40°C, preferably to a temperature of about 37°C.

### 5.7 COMPOSITIONS COMPRISING AMNION DERIVED ADHERENT CELLS

Treating individuals experiencing pain according to the invention encompasses the use of compositions comprising AMDACs, *e.g.,* liquids, solids *(e.g.,* matrices), or a combination of both (*e.g*., hydrogels). In certain embodiments, AMDACs are contained within, or are components of, a pharmaceutical composition.

The cells can be prepared in a form that is easily administrable to an individual, *e.g*., AMDACs in solution suitable for, *e.g*., intravenous administration, that are contained within a container that is suitable for medical use. Such a container can be, for example, a syringe, sterile plastic bag, flask, jar, or other container from which the AMDACs can be easily dispensed. For example, the container can be a blood bag or other plastic, medically-acceptable bag suitable for the intravenous administration of a liquid to a recipient. The container, in certain examples, is one that allows for cryopreservation of the cells. The cells in the compositions, *e.g*., pharmaceutical compositions, can comprise amnion derived adherent cells derived from a single donor, or from multiple donors. The cells can be completely HLA-matched to an intended recipient, or partially or completely HLA-mismatched, *e.g*., can be completely autologous, partially allogeneic, or completely allogeneic.

Thus, in one embodiment, AMDACs in the compositions are administered to an individual in need thereof in the form of a composition comprising AMDACs in a container. In another specific embodiment, the container is a bag, flask, or jar. In more specific embodiment, said bag is a sterile plastic bag. More specifically, said bag is suitable for, allows or facilitates intravenous administration of said AMDACs, *e.g*., by intravenous infusion, bolus injection, or the like. The bag can comprise multiple lumens or compartments that are interconnected to allow mixing of the cells and one or more other solutions, *e.g*., a drug, prior to, or during, administration. In another specific case, prior to cryopreservation, the solution comprising the AMDACs comprises one or more compounds that facilitate cryopreservation of the cells. In another specific case, said AMDACs are contained within a physiologically-acceptable aqueous solution. In a more specific case, said physiologically-acceptable aqueous solution is a 0.9% NaCl solution. In another specific embodiment, said AMDACs are, or comprise cells that are, HLA-matched to a recipient of said cells. In another specific embodiment, said AMDACs are, or comprise cells that are, at least partially HLA-mismatched to a recipient of said cells. In another specific embodiment, said AMDACs are derived from a plurality of donors. In various specific embodiments, said container comprises about, at least, or at most 1 x 10⁶ said cells, 5 x 10⁶ said cells, 1 x 10⁷ said stem cells, 5 x 10⁷ said cells, 1 x 10⁸ said cells, 5 x 10⁸ said cells, 1 x 10⁹ said cells, 5 x 10⁹ said cells, or 1 x 10¹⁰ said cells. In other specific embodiments of any of the foregoing cryopreserved populations, said cells have been passaged about, at least, or no more than 5 times, no more than 10 times, no more than 15 times, or no more than 20 times. In another specific embodiment of any of the foregoing cryopreserved cells, said cells have been expanded within said container. In specific embodiments, a single unit dose of AMDACs can comprise, in various embodiments, about, at least, or no more than 1 x 10⁵, 5 x 10⁵, 1 x 10⁶, 5 x 10⁶, 1 x 10⁷, 5 x 10⁷, 1 x 10⁸, 5 x 10⁸, 1 x 10⁹, 5 x 10⁹, 1 x 10¹⁰, 5 x 10¹⁰, 1 x 10¹¹ or more AMDACs.

In certain embodiments, the pharmaceutical compositions for use according to the invention comprises populations of AMDACs, that comprise 50% viable cells or more (that is, at least 50% of the cells in the population are functional or living). Preferably, at least 60% of the cells in the population are viable. More preferably, at least 70%, 80%, 90%, 95%, or 99% of the cells in the population in the pharmaceutical composition are viable.

### 5.7.1 Pharmaceutical Compositions Comprising AMDACs

Populations of isolated AMDACs, or populations of cells comprising the isolated AMDACs, can be formulated into pharmaceutical compositions for use *in vivo, e.g.,* in the methods of treatment of pain. Such pharmaceutical compositions comprise AMDACs, or a population of cells comprising isolated AMDACs, in a pharmaceutically-acceptable carrier, *e.g.,* a saline solution or other accepted physiologically-acceptable solution for *in vivo* administration. Pharmaceutical compositions comprising the isolated AMDACs described herein can comprise any, or any combination, of the isolated AMDACs populations, or isolated AMDACs, described elsewhere herein. The pharmaceutical compositions can comprise fetal, maternal, or both fetal and maternal isolated cells. The pharmaceutical compositions provided herein can further comprise isolated AMDACs obtained from a single individual, *i.e.,* a single amnion, or from a plurality of individuals. Any of the AMDACs, described elsewhere herein, can be formulated into pharmaceutical composition, as described below.

The pharmaceutical compositions can comprise any number of isolated AMDACs. For example, a single unit dose of isolated AMDACs can comprise, in various embodiments, about, at least, or no more than 1 x 10⁵, 5 x 10⁵, 1 x 10⁶, 5 x 10⁶, 1 x 10⁷, 5 x 10⁷, 1 x 10⁸, 5 x 10⁸, 1 x 10⁹, 5 x 10⁹, 1 x 10¹⁰, 5 x 10¹⁰, 1 x 10¹¹ or more isolated cells.

The pharmaceutical compositions comprise populations of cells that comprise 50% viable cells or more (that is, at least 50% of the cells in the population are functional or living). Preferably, at least 60% of the cells in the population are viable. More preferably, at least 70%, 80%, 90%, 95%, or 99% of the cells in the population in the pharmaceutical composition are viable.

The pharmaceutical compositions can comprise one or more compounds that, *e.g.,* facilitate engraftment (*e.g.,* anti-T-cell receptor antibodies, an immunosuppressant, or the like); stabilizers such as albumin, dextran 40, gelatin, hydroxyethyl starch, plasmalyte, and the like.

When formulated as an injectable solution, in one embodiment, the pharmaceutical composition comprises about 1% to 1.5% HSA and about 2.5% dextran. In a preferred embodiment, the pharmaceutical composition comprises from about 5 x 10⁶ cells per milliliter to about 2 x 10⁷ cells per milliliter in a solution comprising 5% HSA and 10% dextran, optionally comprising an immunosuppressant, *e.g.,* cyclosporine A at, *e.g.,* 10 mg/kg.

In other embodiments, the pharmaceutical composition, *e.g*., a solution, comprises a plurality of cells, *e.g.,* isolated AMDACs, wherein said pharmaceutical composition comprises between about 1.0 ±0.3 x 10⁶ cells per milliliter to about 5.0 ± 1.5 x 10⁶ cells per milliliter. In other embodiments, the pharmaceutical composition comprises between about 1.5 x 10⁶ cells per milliliter to about 3.75 x 10⁶ cells per milliliter. In other embodiments, the pharmaceutical composition comprises between about 1 x 10⁶ cells/mL to about 50 x 10⁶ cells/mL, about 1 x 10⁶ cells/mL to about 40 x 10⁶ cells/mL, about 1 x 10⁶ cells/mL to about 30 x 10⁶ cells/mL, about 1 x 10⁶ cells/mL to about 20 x 10⁶ cells/mL, about 1 x 10⁶ cells/mL to about 15 x 10⁶ cells/mL, or about 1 x 10⁶ cells/mL to about 10 x 10⁶ cells/mL. In certain embodiments, the pharmaceutical composition comprises no visible cell clumps (i.e., no macro cell clumps), or substantially no such visible clumps. As used herein, "macro cell clumps" means an aggregation of cells visible without magnification, *e.g*., visible to the naked eye, and generally refers to a cell aggregation larger than about 150 microns. In some embodiments, the pharmaceutical composition comprises about 2.5%, 3.0%, 3.5%, 4.0%, 4.5%, 5.0%, 5.5%, 6.0%, 6.5%, 7.0%, 7.5% 8.0%, 8.5%, 9.0%, 9.5% or 10% dextran, *e.g*., dextran-40. In a specific embodiment, said composition comprises about 7.5% to about 9% dextran-40. In a specific embodiment, said composition comprises about 5.5 % dextran-40. In certain embodiments, the pharmaceutical composition comprises from about 1% to about 15% human serum albumin (HSA). In specific embodiments, the pharmaceutical composition comprises about 1%, 2%, 3%, 4%, 5%, 65, 75, 8%, 9%, 10%, 11%, 12%, 13%, 14% or 15% HSA. In a specific embodiment, said cells have been cryopreserved and thawed. In another specific embodiment, said cells have been filtered through a 70 µM to 100 µM filter. In another specific embodiment, said composition comprises no visible cell clumps. In another specific embodiment, said composition comprises fewer than about 200 cell clumps per 10⁶ cells, wherein said cell clumps are visible only under a microscope, *e.g*., a light microscope. In another specific embodiment, said composition comprises fewer than about 150 cell clumps per 10⁶ cells, wherein said cell clumps are visible only under a microscope, *e.g*., a light microscope. In another specific embodiment, said composition comprises fewer than about 100 cell clumps per 10⁶ cells, wherein said cell clumps are visible only under a microscope, *e.g*., a light microscope.

In a specific embodiment, the pharmaceutical composition comprises about 1.0 ±0.3 x 10⁶ cells per milliliter, about 5.5% dextran-40 (w/v), about 10% HSA (w/v), and about 5% DMSO (v/v).

In other embodiments, the pharmaceutical composition comprises a plurality of cells, *e.g.,* a plurality of isolated AMDACs in a solution comprising 10% dextran-40, wherein the pharmaceutical composition comprises between about 1.0 ± 0.3 x 10⁶ cells per milliliter to about 5.0 ±1.5 x 10⁶ cells per milliliter, and wherein said composition comprises no cell clumps visible with the unaided eye (i.e., comprises no macro cell clumps). In some embodiments, the pharmaceutical composition comprises between about 1.5 x 10⁶ cells per milliliter to about 3.75 x 10⁶ cells per milliliter. In a specific embodiment, said cells have been cryopreserved and thawed. In another specific embodiment, said cells have been filtered through a 70 µM to 100 µM filter. In another specific embodiment, said composition comprises fewer than about 200 micro cell clumps (that is, cell clumps visible only with magnification) per 10⁶ cells. In another specific embodiment, the pharmaceutical composition comprises fewer than about 150 micro cell clumps per 10⁶ cells. In another specific embodiment, the pharmaceutical composition comprises fewer than about 100 micro cell clumps per 10⁶ cells. In another specific embodiment, the pharmaceutical composition comprises less than 15%, 14%, 13%, 12%, 11%, 10%, 9%, 8%, 7%, 6%, 5%, 4%, 3%, or 2% DMSO, or less than 1%, 0.9%, 0.8%, 0.7%, 0.6%, 0.5%, 0.4%, 0.3%, 0.2%, or 0.1% DMSO.

Compositions comprising cells for use according to the invention may be produced by one of the methods disclosed herein. For example, in one embodiment, the pharmaceutical composition comprises cells, wherein the pharmaceutical composition is produced by a method comprising filtering a solution comprising AMDACs to form a filtered cell-containing solution; diluting the filtered cell-containing solution with a first solution to about 1 to 50 x 10⁶, 1 to 40 x 10⁶, 1 to 30 x 10⁶, 1 to 20 x 10⁶, 1 to 15 x 10⁶, or 1 to 10 x 10⁶ cells per milliliter, *e.g*., prior to cryopreservation; and diluting the resulting filtered cell-containing solution with a second solution comprising dextran, but not comprising human serum albumin (HSA) to produce said composition. In certain embodiments, said diluting is to no more than about 15 x 10⁶ cells per milliliter. In certain embodiments, said diluting is to no more than about 10 ± 3 x 10⁶ cells per milliliter. In certain embodiments, said diluting is to no more than about 7.5 x 10⁶ cells per milliliter. In other certain embodiments, if the filtered cell-containing solution, prior to the dilution, comprises less than about 15 x 10⁶ cells per milliliter, filtration is optional. In other certain embodiments, if the filtered cell-containing solution, prior to the dilution, comprises less than about 10 ± 3 x 10⁶ cells per milliliter, filtration is optional. In other certain embodiments, if the filtered cell-containing solution, prior to the dilution, comprises less than about 7.5 x 10⁶ cells per milliliter, filtration is optional.

In a specific embodiment, the cells are cryopreserved between said diluting with a first dilution solution and said diluting with said second dilution solution. In another specific embodiment, the first dilution solution comprises dextran and HSA. The dextran in the first dilution solution or second dilution solution can be dextran of any molecular weight, *e.g*., dextran having a molecular weight of from about 10 kDa to about 150 kDa. In some embodiments, said dextran in said first dilution solution or said second solution is about 2.5%, 3.0%, 3.5%, 4.0%, 4.5%, 5.0%, 5.5%, 6.0%, 6.5%, 7.0%, 7.5% 8.0%, 8.5%, 9.0%, 9.5% or 10% dextran. In another specific embodiment, the dextran in said first dilution solution or said second dilution solution is dextran-40. In another specific embodiment, the dextran in said first dilution solution and said second dilution solution is dextran-40. In another specific embodiment, said dextran-40 in said first dilution solution is 5.0% dextran-40. In another specific embodiment, said dextran-40 in said first dilution solution is 5.5% dextran-40. In another specific embodiment, said dextran-40 in said second dilution solution is 10% dextran-40. In another specific embodiment, said HSA in said solution comprising HSA is 1 to 15 % HSA. In another specific embodiment, said HSA in said solution comprising HSA is about 1%, 2%, 3%, 4%, 5%, 6%, 7%, 8%, 9%, 10%, 11%, 12%, 13%, 14% or 15 % HSA. In another specific embodiment, said HSA in said solution comprising HSA is 10% HSA. In another specific embodiment, said first dilution solution comprises HSA. In a more specific embodiment, said HSA in said first dilution solution is 10% HSA. In another specific embodiment, said first dilution solution comprises a cryoprotectant. In a more specific embodiment, said cryoprotectant is DMSO. In another specific embodiment, said dextran-40 in said second dilution solution is about 10% dextran-40. In another specific embodiment, said composition comprising cells comprises about 7.5% to about 9% dextran. In another specific embodiment, the pharmaceutical composition comprises from about 1.0 ±0.3 x 10⁶ cells per milliliter to about 5.0 ±1.5 x 10⁶ cells per milliliter. In another specific embodiment, the pharmaceutical composition comprises from about 1.5 x 10⁶ cells per milliliter to about 3.75 x 10⁶ cells per milliliter.

In another embodiment, the pharmaceutical composition is made by a method comprising (a) filtering a cell-containing solution comprising AMDACs prior to cryopreservation to produce a filtered cell-containing solution; (b) cryopreserving the cells in the filtered cell-containing solution at about 1 to 50 x 10⁶, 1 to 40 x 10⁶, 1 to 30 x 10⁶, 1 to 20 x 10⁶, 1 to 15 x 10⁶, or 1 to 10 x 10⁶ cells per milliliter; (c) thawing the cells; and (d) diluting the filtered cell-containing solution about 1:1 to about 1:11 (v/v) with a dextran-40 solution. In certain embodiments, if the number of cells is less than about 10 ± 3 x 10⁶ cells per milliliter prior to step (a), filtration is optional. In a more specific embodiment, the cells in step (b) are cryopreserved at about 10 ± 3 x 10⁶ cells per milliliter. In a more specific embodiment, the cells in step (b) are cryopreserved in a solution comprising about 5% to about 10% dextran-40 and HSA. In certain embodiments, said diluting in step (b) is to no more than about 15 x 10⁶ cells per milliliter.

In another embodiment, the pharmaceutical composition is made by a method comprising: (a) suspending AMDACs in a 5.5% dextran-40 solution that comprises 10% HSA to form a cell-containing solution; (b) filtering the cell-containing solution through a 70 µM filter; (c) diluting the cell-containing solution with a solution comprising 5.5% dextran-40, 10% HSA, and 5% DMSO to about 1 to 50 x 10⁶, 1 to 40 x 10⁶, 1 to 30 x 10⁶, 1 to 20 x 10⁶, 1 to 15 x 10⁶, or 1 to 10 x 10⁶ cells per milliliter; (d) cryopreserving the cells; (e) thawing the cells; and (f) diluting the cell-containing solution 1:1 to 1:11 (v/v) with 10% dextran-40. In certain embodiments, said diluting in step (c) is to no more than about 15 x 10⁶ cells per milliliter. In certain embodiments, said diluting in step (c) is to no more than about 10 ± 3 x 10⁶ cells/mL. In certain embodiments, said diluting in step (c) is to no more than about 7.5 x 10⁶ cells/mL.

In another embodiment, the composition comprising cells is made by a method comprising: (a) centrifuging a plurality of AMDACs to collect the cells; (b) resuspending the cells in 5.5% dextran-40; (c) centrifuging the cells to collect the cells; (d) resuspending the cells in a 5.5% dextran-40 solution that comprises 10% HSA; (e) filtering the cells through a 70 µM filter; (f) diluting the cells in 5.5% dextran-40, 10% HSA, and 5% DMSO to about 1 to 50 x 10⁶, 1 to 40 x 10⁶, 1 to 30 x 10⁶, 1 to 20 x 10⁶, 1 to 15 x 10⁶, or 1 to 10 x 10⁶ cells per milliliter; (g) cryopreserving the cells; (h) thawing the cells; and (i) diluting the cells 1:1 to 1:11 (v/v) with 10% dextran-40. In certain embodiments, said diluting in step (f) is to no more than about 15 x 10⁶ cells per milliliter. In certain embodiments, said diluting in step (f) is to no more than about 10 ± 3 x 10⁶ cells/mL. In certain embodiments, said diluting in step (f) is to no more than about 7.5 x 10⁶ cells/mL. In other certain embodiments, if the number of cells is less than about 10 ± 3 x 10⁶ cells per milliliter, filtration is optional.

The compositions, *e.g*., pharmaceutical compositions comprising the AMDACs, described herein can comprise any of the isolated AMDACs described herein.

Other injectable formulations, suitable for the administration of cellular products, may be used.

In certain embodiments, the AMDACs can be encapsulated in, *e.g*., alginate, either before or after cryopreservation. In certain other embodiments, the AMDACs can be combined with platelet-rich plasma, *e.g*., for local injection or local administration applications. In specific embodiments, the platelet rich plasma is autologous platelet rich plasma, *e.g*., autologous to the individual having pain to whom the AMDACs are administered. In other specific embodiments, the platelet-rich plasma is allogeneic to the individual having pain to whom the AMDACs are administered. In another specific embodiment, said platelet rich plasma is derived from placental perfusate. In other specific embodiments, the volume to volume ratio of AMDACs to platelet rich plasma in the composition, or the ratio between numbers of AMDACs and numbers of platelets, is between about 10:1 and 1:10; between about 100:1 and 1:100; or is about 1:1.

In one embodiment, the pharmaceutical composition comprises isolated AMDACs that are substantially, or completely, non-maternal in origin, that is, have the fetal genotype; *e.g.,* at least about 90%, 95%, 98%, 99% or about 100% are non-maternal in origin.

In a specific embodiment, the pharmaceutical composition additionally comprises stem cells that are not obtained from a placenta. Isolated AMDACs in the compositions, *e.g*., pharmaceutical compositions, provided herein, can comprise AMDACs derived from a single donor, or from multiple donors. The isolated AMDACs can be completely HLA-matched to an intended recipient, or partially or completely HLA-mismatched.

### 5.7.2 Matrices Comprising AMDACs

In certain embodiments AMDACs for use in the methods of treating individuals experienceing pain, presented herein, the AMDACs are administered in compositions comprising matrices, hydrogels, scaffolds, and the like. Such compositions can be used in the place of, or in addition to, such cells in liquid suspension.

The matrix can be, *e.g.,* a permanent or degradable decellularized tissue, *e.g.,* a decellularized amniotic membrane, or a synthetic matrix. The matrix can be a three-dimensional scaffold. In a more specific embodiment, said matrix comprises collagen, gelatin, laminin, fibronectin, pectin, ornithine, or vitronectin. In another more specific embodiment, the matrix is an amniotic membrane or an amniotic membrane-derived biomaterial. In another more specific embodiment, said matrix comprises an extracellular membrane protein. In another more specific embodiment, said matrix comprises a synthetic compound. In another more specific embodiment, said matrix comprises a bioactive compound. In another more specific embodiment, said bioactive compound is a growth factor, a cytokine, an antibody, or an organic molecule of less than 5,000 daltons.

The AMDACs described herein can be seeded onto a natural matrix, e.g., a placental biomaterial such as an amniotic membrane material. Such an amniotic membrane material can be, *e.g*., amniotic membrane dissected directly from a mammalian placenta; fixed or heat-treated amniotic membrane, substantially dry (*i.e.,* <20% H₂O) amniotic membrane, chorionic membrane, substantially dry chorionic membrane, substantially dry amniotic and chorionic membrane, and the like. Preferred placental biomaterials on which the amnion derived adherent cells provided herein can be seeded are described in Hariri, U.S. Application Publication No. 2004/0048796.

In another specific embodiment, the matrix is a composition comprising an extracellular matrix. In a more specific embodiment, said composition is MATRIGEL™ (BD Biosciences).

The isolated amnion derived adherent cells described herein can be suspended in a hydrogel solution suitable for, *e.g.,* injection. The hydrogel is, *e.g.,* an organic polymer (natural or synthetic) that is cross-linked via covalent, ionic, or hydrogen bonds to create a three-dimensional open-lattice structure that entraps water molecules to form a gel. Suitable hydrogels for such compositions include self-assembling peptides, such as RAD16. In one embodiment, a hydrogel solution comprising the cells can be allowed to harden, for instance in a mold, to form a matrix having cells dispersed therein for implantation. The amnion derived adherent cells in such a matrix can also be cultured so that the cells are mitotically expanded, *e.g*., prior to implantation. Hydrogel-forming materials include polysaccharides such as alginate and salts thereof, peptides, polyphosphazines, and polyacrylates, which are crosslinked ionically, or block polymers such as polyethylene oxide-polypropylene glycol block copolymers which are crosslinked by temperature or pH, respectively. In some embodiments, the hydrogel or matrix is biodegradable.

In certain embodiments, the AMDACs can be encapsulated in, *e.g*., alginate, either before or after cryopreservation. In certain other embodiments, the AMDACs can be combined with platelet-rich plasma, *e.g*., for local injection or local administration applications. In specific embodiments, the platelet rich plasma is autologous platelet rich plasma, *e.g*., autologous to the individual having pain to whom the AMDACs are administered. In other specific embodiments, the platelet-rich plasma is allogeneic to the individual having pain to whom the AMDACs are administered. In another specific embodiment, said platelet rich plasma is derived from placental perfusate. In other specific embodiments, the volume to volume ratio of AMDACs to platelet rich plasma in the composition, or the ratio between numbers of AMDACs and numbers of platelets, is between about 10:1 and 1:10; between about 100:1 and 1:100; or is about 1:1.

In certain embodiments, the compositions comprising cells, provided herein, comprise an *in situ* polymerizable gel (*see, e.g.,* U.S. Patent Application Publication 2002/0022676; Anseth et al., J. Control Release, 78(1-3):199-209 (2002); Wang et al., Biomaterials, 24(22):3969-80 (2003). In some embodiments, the polymers are at least partially soluble in aqueous solutions, such as water, buffered salt solutions, or aqueous alcohol solutions that have charged side groups, or a monovalent ionic salt thereof. Examples of polymers having acidic side groups that can be reacted with cations are poly(phosphazenes), poly(acrylic acids), poly(methacrylic acids), copolymers of acrylic acid and methacrylic acid, poly(vinyl acetate), and sulfonated polymers, such as sulfonated polystyrene. Copolymers having acidic side groups formed by reaction of acrylic or methacrylic acid and vinyl ether monomers or polymers can also be used. Examples of acidic groups are carboxylic acid groups, sulfonic acid groups, halogenated (preferably fluorinated) alcohol groups, phenolic OH groups, and acidic OH groups.

In a specific embodiment, the matrix is a felt, which can be composed of a multifilament yarn made from a bioabsorbable material, *e.g*., PGA, PLA, PCL copolymers or blends, or hyaluronic acid. The yarn is made into a felt using standard textile processing techniques consisting of crimping, cutting, carding and needling. In another preferred embodiment the cells of the invention are seeded onto foam scaffolds that may be composite structures. In addition, the three-dimensional framework may be molded into a useful shape, such as a specific structure in the body to be repaired, replaced, or augmented. Other examples of scaffolds that can be used include nonwoven mats, porous foams, or self assembling peptides. Nonwoven mats can be formed using fibers comprised of a synthetic absorbable copolymer of glycolic and lactic acids (*e.g.,* PGA/PLA) (VICRYL, Ethicon, Inc., Somerville, N.J.). Foams, composed of, *e.g.,* poly(ε-caprolactone)/poly(glycolic acid) (PCL/PGA) copolymer, formed by processes such as freeze-drying, or lyophilization (*see, e.g.,* U.S. Pat. No. 6,355,699), can also be used as scaffolds.

The AMDACs described herein can be seeded onto a three-dimensional framework or scaffold and implanted *in vivo.* Such a framework can be implanted in combination with any one or more growth factors, cells, drugs or other components that, *e.g*., stimulate tissue formation, *e.g*., formation of vasculature.

The AMDACs provided herein can, in another embodiment, be seeded onto foam scaffolds that may be composite structures. Such foam scaffolds can be molded into a useful shape, such as that of a portion of a specific structure in the body to be repaired, replaced or augmented. In some embodiments, the framework is treated, *e.g*., with 0.1M acetic acid followed by incubation in polylysine, PBS, and/or collagen, prior to inoculation of the cells in order to enhance cell attachment. External surfaces of a matrix may be modified to improve the attachment or growth of cells and differentiation of tissue, such as by plasma-coating the matrix, or addition of one or more proteins (*e.g*., collagens, elastic fibers, reticular fibers), glycoproteins, glycosaminoglycans (*e.g*., heparin sulfate, chondroitin-4-sulfate, chondroitin-6-sulfate, dermatan sulfate, keratin sulfate, *etc.*), a cellular matrix, and/or other materials such as, but not limited to, gelatin, alginates, agar, agarose, and plant gums, and the like.

### 5.7.3 Media Conditioned by Amnion Derived Adherent Cells

Further disclosed herein is medium that has been conditioned by AMDACs, that is, medium comprising one or more biomolecules secreted or excreted by the AMDACs, which may, itself, be used to treat individuals having pain. In various examples, the conditioned medium comprises medium in which the cells have grown for at least 1, 2, 3, 4, 5, 6, 7, 8, 9, 10, 11, 12, 13, 14 or more days, or for at least 1, 2, 3, 4, 5, 6, 7, 8, 9, 10, 11, 12, 13, 14, 15, 16, 17, 18, 19, or 20 population doublings, or more. In other examples, the conditioned medium comprises medium in which amnion derived adherent cells have grown to at least 30%, 40%, 50%, 60%, 70%, 80%, 90% confluence, or up to 100% confluence. In another examples, the conditioned medium comprises medium in which AMDACs, and cells that are not AMDACs, have been cultured together.

The conditioned medium can comprise the adherent cells provided herein. Thus, disclosed herein is a cell culture comprising AMDACs. In a specific examples, the conditioned medium comprises a plurality, *e.g.,* a population, of amnion derived adherent cells.

### 6. EXAMPLES

### 6.1 EXAMPLE 1. TREATMENT OF PAIN USING AMDACS

This example demonstrates the effect of AMDACs on pain using a rat neuritis model. In this model, perineural inflammation is induced in the sciatic nerve vicinity; pain develops in the nerve target organ (hind-paw) within 2-3 days and resolves within approximately 8 days (Eliav, et al., Pain (1999) 83:169-182; and Noma et al., Neuroscience Letters (2011) 493:86-91).

This study evaluated the effect of AMDACs provided systemically (intravenous (I.V.)), or to muscle (intramuscular (I.M.)) on neuropathic pain in this model.

### 6.1.1 Methods

***Animals:*** The study was performed on male Sprague Dawley rats, 3 months old, approximately 250-300 g Male at study initiation; the minimum and maximum weights of the group were within a range of ±20 % of group mean weight.

***Anesthesia:*** For surgical procedures, rats were anaesthetized with ketamine (50 mg/kg) and xylazine (7.5 mg/kg) solution that was administered intraperitoneally. Following verification of the anesthesia, the area of surgery was shaved and subsequently sterilized with betadine and alcohol wipe. The rats' eyes were lubricated.

***Perineural inflammation surgical procedure:*** The surgery was performed based on the original description (Eliav et al. 1999,*supra*). In brief, the common sciatic nerve was exposed at the mid-thigh level by blunt dissection through the biceps femoris and gently separated from adjacent tissue. The nerve was wrapped in a band (approx. 3 mm wide and 25 mm long) of sterile hemostatic oxidized cellulose (Surgicel™; 'cotton' type; Ethicon, J&J, NJ, USA). Surgicel was applied by passing curved forceps beneath the nerve (taking particular care to avoid stretching the nerve), grasping one end of the band and pulling it under the nerve. The end that was grasped was then gently folded over the nerve; the other end was folded over in the opposite direction. The Surgicel was wrapped loosely around the nerve and did not cause any nerve constriction; it was intended to act as a sponge. Carrageenan (200 cc) was then injected into the Surgicel band. Carrageenan induces a local inflammatory reaction and has been one of the primary immunological adjuvants used in antigen - adjuvant emulsions for immunological studies.

***Behavior test:*** Tactile allodynia was measured by assessing the withdrawal response to calibrated Von Frey fibers. Three fibers according to force application - low (8g), medium (16g) and high force fiber (26g) were employed in this study. Each fiber was applied to the paw five times and a percentage score of responses was calculated (Flatters, S.J. & Bennett, G. J., Pain (2004) 109: 150-161). The most reliable and repeatable score at baseline level were the response to 26g; therefore statistical analysis was performed on the 26g data.

***Data Analysis:*** Data were tabulated and analyzed using StatView software version 5.0 (SAS Institute Inc., San Francisco, California, USA). Alpha (two tailed) for significance in all analyses was set at 0.05. Behavioral statistics were calculated only for rats with data at all time points. The pain behavior data was analyzed with a repeated measurements analysis of variance (ANOVA) followed by post hoc test.

### 6.1.2 RESULTS

### The Effect of IV Administered ADMAC cells

The objective of these experiments was to assess the effect of AMDACs administered IV on pain induced by the rat neuritis model described herein. Briefly, cells in various doses or vehicle were provided to the tail vein on day 3 following the induction of perineural inflammation. Pain levels were assessed at baseline, 3 days following the procedure (prior to cell administration), and 4, 6 and 8 days following the procedure. The AMDACs in all the tested doses reduced pain significantly on days 4, 6 and 8 compared to the vehicle and to the pain level assessed on day 3.

In particular, cells frozen in freezing medium were thawed and diluted in Plasmalyte A to the concentrations specified below. Vehicle was a mixture of freezing medium and Plasmalyte A; amounts were determined based on cell dilution volumes. On the 3rd day following exposure of the left sciatic nerve to perineural inflammation the rats were randomly assigned to one of the following treatment groups, wherein cells were administered to the tail vein: 4x10⁶ AMDAC cells, 4 x10⁵ AMDAC cells, 4 x10⁴ AMDAC cells or vehicle in similar volumes. Pain levels were assessed on days 0 (baseline), 3, 4, 6 and 8.

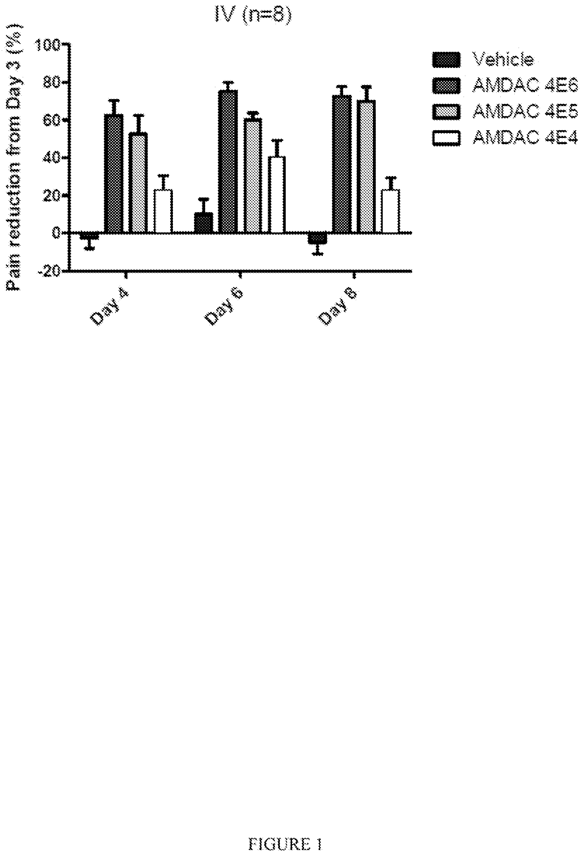

Pain was developed on the 3rd day following the procedure compared to the baseline. While the vehicle had no effect on the pain levels, the AMDAC cells reduced the pain significantly on days 4, 6 and 8 compared to the 3rd day pain level and compared to the vehicle treated group (Figure 1). Higher doses of AMDACs (e.g., 4 x10⁶ cells) demonstrated greater activity than the lowest dose (0.4 x106 cells). The lowest dose of AMDACs (4 x10⁴) had only a limited and shorter lasting effect. No significant effect was demonstrated on the contralateral paw.

Thus, AMDACs reduced neuropathic pain induced by perineural inflammation when administrated I.V. The effect was dose dependent, began within 24 hours following I.V. administration, and lasted at least 5 days following administration. This result with AMDACs is in contrast to drugs (*e.g*., non-steroidal anti-inflammatory drugs (NSAIDS) or corticosteroids) used to treat inflammation, whose use in treating pain associated with neuritis (*e.g*., sciatic nerve pain) is often ineffective. *See,* Pinto, et al., BMJ (2012) 344:e497).

### The effect of IM Administered ADMAC Cells

The objective of this experiment was to assess the effect of AMDACs administered I.M. on pain induced by the rat neuritis model described herein. The cells were injected to the affected side, contralateral or the forelimb muscles. The AMDACs reduced pain significantly when injected to the affected and contralateral posterior limb muscles. Forearm injection induced a short-lasting, limited effect.

In particular, on the 3rd day following exposure of the left sciatic nerve to perineural inflammation the rats were randomly assigned to one of the following treatments: 1x10⁵ AMDACs injected to the muscle ipsilateral to the affected sciatic nerve, 1 x10⁵ AMDACs injected to the muscle contralateral to the affected sciatic nerve, 1x10⁵ AMDACs injected to the forelimb muscle on the same side of the affected nerve, 1x10⁴ AMDACs injected to the muscle ipsilateral to the affected sciatic nerve, or similar volume of vehicle injected to the muscle ipsilateral to affected sciatic nerve. Pain levels were assessed on days 0 (baseline), 3, 4, 6 and 8.

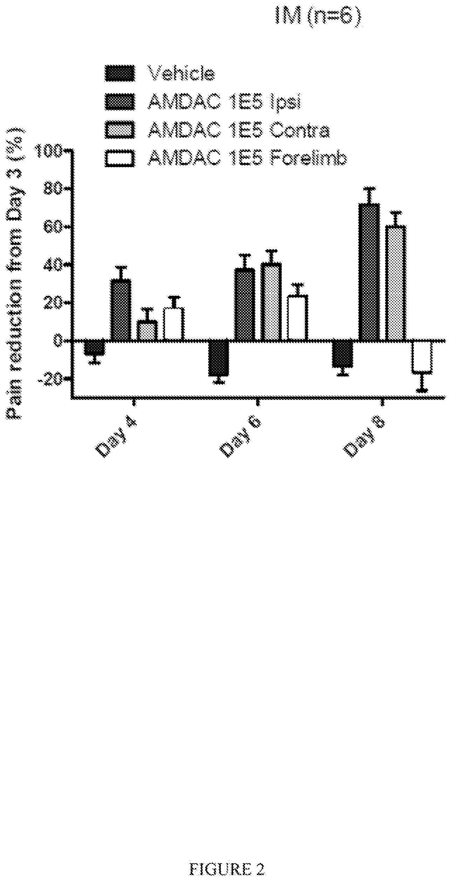

Pain was developed on the 3rd day following the procedure compared to the baseline. While the vehicle had no effect on the pain levels, the administration of 1x10⁵ ADMAC cells to the posterior limbs (ipsilateral and contralateral) reduced the pain significantly on days 4, 6 and 8 compared to the vehicle treated group (Figure 2). Administration of 1 x10⁵ AMDAC cells to the forelimb (contralateral side to the affected sciatic nerve) or administration of 1 x10⁴ AMDAC cells to the muscle ipsilateral to the affected sciatic nerve had a limited and short lasting anti-nociceptive effect. No significant effect was demonstrated on the contralateral paw.

Thus, AMDACs reduced neuropathic pain induced by perineural inflammation in rats when administrated I.M. The effect was significant when the cells were administrated to the posterior limbs but not when administrated to the forelimbs; the contralateral administration was as effective as the ipsilateral administration. This result with AMDACs, too, is in contrast to drugs (*e.g.*, non-steroidal anti-inflammatory drugs (NSAIDS) or corticosteroids) used to treat inflammation, whose use in treating pain associated with neuritis (*e.g*., sciatic nerve pain) is often ineffective. *See,* Pinto, et al., BMJ (2012) 344:e497).

### 6.2 EXAMPLE 2: TREATMENT OF NEUROPATHIC PAIN USING AMDACS

### 6.2.1 Intravenous administration

### Case 1

An individual presents with neuropathic pain in the extremities related to administration of paclitaxel. The attending oncologist indicates that maintenance of paclitaxel therapy is strongly indicated. An assessment of pain is performed using the Pain Quality Assessment Scale, with the quality of pain indicated on a scale of 0-10 for each indicated type of pain. An aggregate score is also recorded. After pain assessment, the individual is administered 1 x 10⁹ OCT-4⁻ amnion derived adherent cells (AMDACs) in normal saline by intravenous infusion. The individual is monitored for the following seven days for any adverse events, and is reassessed for pain on day 7 following administration. The scores for each individual pain quality, and overall aggregate pain score, are compared to the scores prior to administration. If the majority of the pain quality scores, or the aggregate score, is not reduced after administration, the individual is optionally provided a second administration of 1 x 10⁹ AMDACs in normal saline by intravenous infusion. The individual is then monitored over the course of paclitaxel therapy, and optionally for six months afterwards; administration of the AMDACs is repeated at any time paclitaxel-related pain is determined to increase by the Pain Quality Assessment Scale.

### Case 2

A 78-year old diabetic individual presents with diabetic neuropathy experienced primarily in the legs, with apparent sciatic nerve involvement, making walking difficult. The individual's pain is assessed using the Numeric Pain Assessment Scale, both while the individual is seated, and while the individual is walking. After pain assessment, the individual is administered 1 x 10⁹ OCT-4⁻ amnion derived adherent cells (AMDACs) in normal saline by intravenous infusion. The individual is monitored for the following seven days for any adverse events, and is reassessed for pain on day 7 following administration, again while the individual is seated and while the individual is walking. The administration is considered successful if the individual indicates are reduction in pain while seated, while walking, or both. Optionally, if the individual indicates improvement while seated, or while walking, but not both, the individual may be administered a second dose of AMDACs equivalent to the first. The individual is then monitored for the following six months every 1-2 weeks, and follow-up administration(s) take place whenever pain according to the Numeric Pain Assessment Scale is determined to worsen.

### Case 3

A 62-year old individual presents with postherpetic neuralgia. The individual's medical records confirm a previous case of shingles with accompanying rash and herpetic pustules on the individual's right dorsal area. Pain associated with the shingles, however, has not resolved after one month after healing of the rash and pustules. The individual's pain is assessed using the Numeric Pain Assessment Scale. After pain assessment, the individual is administered 1 x 10⁹ OCT-4 AMDACs in normal saline by intravenous infusion. The individual is monitored for the following seven days for any adverse events, and is reassessed for pain on day 7 following administration.

### 6.2.2 Local Administration

A 62-year old individual presents with postherpetic neuralgia. The individual's medical records confirm a previous case of shingles with accompanying rash and herpetic pustules. Pain associated with the shingles, however, has not resolved after one month after healing of the rash and pustules. The individual's pain is assessed using the Numeric Pain Assessment Scale. After pain assessment, the individual is administered 3 x 10⁷ OCT-4⁻AMDACs in a solution of platelet-rich plasma in a series of 10 injections adjacent to the nerve trunk servicing the area of the individual affected by the shingles. The individual is monitored for the following seven days for any adverse events, and is reassessed for pain on day 7 following administration.

### 6.3 EXAMPLE 3: TREATMENT OF VULVODYNIA USING AMDACS

A 41-year old female individual presents with vulvodynia. Prior to treatment, the exact pain sites are determined with cotton tips and gentle digital palpation. The individual's pain is assessed using the Numeric Pain Assessment Scale. After pain assessment, the individual is given 1 x 10⁹ OCT-4⁻AMDACs in a solution of platelet-rich plasma via intravaginal administration to the pain sites. The individual is monitored for the following seven days for any adverse events, and is reassessed for pain on day 7 following administration.

### 6.4 EXAMPLE 4: TREATMENT OF INTERSTITIAL CYSTITIS USING AMDACS

A 29-year old female individual is diagnosed with interstitial cystitis. The individual's pain is assessed using the Numeric Pain Assessment Scale. After pain assessment, the individual is given 1 x 10⁹ OCT-4AMDACs in a solution of platelet-rich plasma via intravesical route, on either side of the bladder neck, and other pelvic sites that the individual has identified as tender during the examination. The individual is monitored for the following seven days for any adverse events, and is reassessed for pain on day 7 following administration. The individual is also assessed by urinalysis and biomarkers for interstitial cystitis on day 7 following administration.

## Claims

1. A composition comprising OCT-4⁻ tissue culture surface-adherent amnion-derived adherent cells (AMDACs) for use in a method of treating pain in an individual, wherein said pain is unresponsive to steroid therapy, or nonsteroidal anti-inflammatory therapy, wherein the method comprises administering a therapeutically effective amount of AMDACs to the individual, and wherein the therapeutically effective amount is an amount sufficient to cause a detectable improvement in said pain.

2. The composition for use of claim 1, wherein said method additionally comprises determining one or more first levels of pain in said individual prior to administration of said AMDACs, and determining one or more second levels of pain in said individual after administration of said AMDACs, wherein said method of treating pain comprises reducing said one or more second levels of said pain as compared to said one or more first levels of pain,
preferably wherein a first level of pain in said individual prior to administration of said AMDACs, and a second level of pain in said individual after administration of said AMDACs, are determined using a pain assessment scale, preferably wherein said pain assessment scale is the Numeric Pain Intensity Scale; the Pain Quality Assessment Scale; the Simple Descriptive Pain Intensity Scale; the Visual Analog Scale; the Wong-Baker FACES Pain Rating Scale; the FLACC scale; the CRIES scale; the COMFORT scale; or evoked pain measure induced by subjecting the patient to cold, heat or mechanical stimuli.

3. The composition for use of claim 1 or claim 2, wherein said method additionally comprises determining a first level of one or more physiological indicia of pain in said individual prior to administration of said AMDACs, and determining a second level of one or more physiological indicia of pain in said individual after administration of said AMDACs, wherein said method of treating pain comprises reducing said second level as compared to said first level.

4. The composition for use of claim 3, wherein said physiological indicium of pain is:
(i) heart rate in the individual, preferably wherein said heart rate in said individual is lower after said administration compared to said heart rate in said individual before said administration;
(ii) the systolic of said individual, preferably wherein said systolic of said individual is lower after said administration compared to said systolic in said individual before said administration; or
(iii) the diastolic of said individual, preferably wherein said diastolic of said individual is lower after said administration compared to said diastolic in said individual before said administration.

5. The composition for use of claim 4, wherein said AMDACs are:
(a) HLA-G⁻, as determinable by RT-PCR and/or CD49f⁺, as determinable by flow cytometry;
(b) CD90⁺, CD105⁺, and/or CD117⁻ as determinable by flow cytometry, preferably wherein said AMDACs are OCT-4⁻ and HLA-G⁻, as determinable by RT-PCR, and CD49f⁺, CD90⁺, CD105⁺, and CD117⁻ as determinable by flow cytometry;
(c) VEGFR1/Flt-1⁺ (vascular endothelial growth factor receptor 1) and VEGFR2/KDR⁺ (vascular endothelial growth factor receptor 2), as determinable by immunolocalization;
(d) one or more of CD9⁺, CD10⁺, CD44⁺, CD54⁺, CD98⁺, Tie-2⁺ (angiopoietin receptor), TEM-7⁺ (tumor endothelial marker 7), CD31⁻, CD34⁻, CD45⁻, CD133⁻, CD143⁻ (angiotensin-I-converting enzyme, ACE), CD146⁻ (melanoma cell adhesion molecule), and/or CXCR4⁻ (chemokine (C-X-C motif) receptor 4) as determinable by immunolocalization;
(e) VE-cadherin⁻ as determinable by immunolocalization; or
(f) CD105⁺ and CD200⁺ as determinable by immunolocalization.

6. The composition for use of any one of claims 1 to 5, wherein said AMDACs do not express CD34 as determinable by immunolocalization after exposure to 50 ng/mL VEGF for 7 days.

7. The composition for use of any one of claims 1 to 6, wherein said AMDACs are comprised within an isolated population of cells, and wherein at least 50% of the cells in said population are said AMDACs,
preferably wherein at least 80%, or at least 90% of the cells in said population are said AMDACs.

8. The composition for use of claim 7, wherein said population further comprises an isolated second type of cells, and wherein said population is not an amnion, portion of an amnion, or homogenate of an amnion,
preferably wherein said second type of cells are embryonic stem cells, blood cells, stem cells isolated from peripheral blood, stem cells isolated from placental blood, stem cells isolated from placental perfusate, stem cells isolated from placental tissue, stem cells isolated from umbilical cord blood, umbilical cord stem cells, bone marrow-derived mesenchymal stem cells, bone marrow-derived mesenchymal stromal cells, hematopoietic stem cells, somatic stem cells, chondrocytes, fibroblasts, muscle cells, endothelial cells, angioblasts, endothelial progenitor cells, pericytes, cardiomyocytes, myocytes, cardiomyoblasts, myoblasts, or cells manipulated to resemble embryonic stem cells,
more preferably wherein said second type of cells comprises at least 10% of cells or at least 25% of cells in said population.

9. The composition for use of claim 8, wherein said second type of cells is hematopoietic stem or progenitor cells, preferably wherein said hematopoietic stem or progenitor cells are CD34⁺ cells.

10. The composition for use of any one of claims 1 to 5, wherein said AMDACs are OCT-4⁻, as determinable by RT-PCR, and CD49f⁺, HLA-G⁻, CD90⁺, CD105⁺, and CD117⁻, as determinable by immunolocalization, and wherein said AMDACs:
(a) express one or more of CD9, CD10, CD44, CD54, CD98, CD200, Tie-2, TEM-7, VEGFR1/Flt-1, and/or VEGFR2/KDR (CD309), as determinable by immunolocalization;
(b) lack expression of CD31, CD34, CD38, CD45, CD133, CD143, CD144, CD146, CD271, CXCR4, HLA-G, and/or VE-cadherin, as determinable by immunolocalization, and/or lack expression of SOX2, as determinable by RT-PCR;
(c) express mRNA for ACTA2, ADAMTS1, AMOT, ANG, ANGPT1, ANGPT2, ANGPTL1, ANGPTL2, ANGPTL4, BAI1, CD44, CD200, CEACAM1, CHGA, COL15A1, COL18A1, COL4A1, COL4A2, COL4A3, CSF3, CTGF, CXCL12, CXCL2, DNMT3B, ECGF1, EDG1, EDIL3, ENPP2, EPHB2, FBLN5, F2, FGF1, FGF2, FIGF, FLT4, FN1, FST, FOXC2, GRN, HGF, HEY1, HSPG2, IFNB1, IL8, IL12A, ITGA4, ITGAV, ITGB3, MDK, MMP2, MYOZ2, NRP1, NRP2, PDGFB, PDGFRA, PDGFRB, PECAM1, PF4, PGK1, PROX1, PTN, SEMA3F, SERPINB5, SERPINC1, SERPINF1, TIMP2, TIMP3, TGFA, TGFB1, THBS1, THBS2, TIE1, TIE2/TEK, TNF, TNNI1, TNFSF15, VASH1, VEGF, VEGFB, VEGFC, VEGFR1/FLT1, and/or VEGFR2/KDR;
(d) express one or more of CD49d, Connexin-43, HLA-ABC, Beta 2-microglobulin, CD349, CD318, PDL1, CD106, Galectin-1, ADAM 17, angiotensinogen precursor, filamin A, alpha-actinin 1, megalin, macrophage acetylated LDL receptor I and II, activin receptor type IIB precursor, Wnt-9 protein, glial fibrillary acidic protein, astrocyte, myosin-binding protein C, and/or myosin heavy chain, nonmuscle type A;
(e) secrete one or more of VEGF, HGF, IL-8, MCP-3, FGF2, Follistatin, G-CSF, EGF, ENA-78, GRO, IL-6, MCP-1, PDGF-BB, TIMP-2, uPAR, and/or Galectin-1 into culture medium in which the AMDACs are cultured;
(f) express micro RNAs miR-17-3p, miR-18a, miR-18b, miR-19b, miR-92, and/or miR-296 at a higher level than an equivalent number of bone marrow-derived mesenchymal stem cells;
(g) express micro RNAs miR-20a, miR-20b, miR-221, miR-222, miR-15b, and/or miR-16 at a lower level than an equivalent number of bone marrow-derived mesenchymal stem cells;
(h) express miRNAs miR-17-3p, miR-18a, miR-18b, miR-19b, miR-92, miR-20a, miR-20b, miR-296, miR-221, miR-222, miR-15b, and/or miR-16; or
(i) express increased levels of CD202b, IL-8 and/or VEGF when cultured in less than about 5% O₂, compared to expression of CD202b, IL-8 and/or VEGF under 21% O₂.

11. The composition for use of claim 1, wherein said pain is:
(a) neuropathic pain;
(b) inflammatory pain;
(c) bone pain; or
(d) caused by cancer.

12. The composition for use of claim 11, wherein said neuropathic pain is caused by
(i) diabetic neuropathy;
(ii) injury to a nerve in said individual; or
(iii) a drug.

13. The composition for use of claim 12, wherein said drug is or comprises a platinum-containing anticancer drug,
preferably wherein said platinum-containing anticancer drug is or comprises oxaliplatin, carboplatin or cisplatin; or said drug is or comprises paclitaxel.

14. The composition for use of claim 11, wherein said bone pain is associated with or caused by cancer.

15. The composition for use of any one of claims 1 to 14, wherein said AMDACs are formulated to be administered locally; or
wherein said AMDACs are formulated to be administered systemically, intravenously or intraarterially.

## Patentansprüche

1. Zusammensetzung, die auf einer OCT-4⁻-Gewebskulturoberfläche anhaftende Amnion-abgeleitete adhärente Zellen (AMDACs) umfasst, zur Verwendung in einem Verfahren zur Schmerzbehandlung bei einem Individuum, wobei der Schmerz nicht auf Steroidtherapie oder nichtsteroidale Antiphlogistikatherapie anspricht, wobei das Verfahren das Verabreichen einer therapeutisch wirksamen Menge von AMDACs an das Individuum umfasst und wobei die therapeutisch wirksame Menge eine Menge darstellt, die ausreicht, um eine detektierbare Verbesserung des Schmerzes zu bewirken.

2. Zusammensetzung zur Verwendung nach Anspruch 1, wobei das Verfahren außerdem das Bestimmen von einem oder mehreren ersten Schmerzausmaßen bei dem Individuum vor der Verabreichung der AMDACs und das Bestimmen von einem oder mehreren zweiten Schmerzausmaßen bei dem Individuum nach der Verabreichung der AMDACs umfasst, wobei das Verfahren zur Schmerzbehandlung das Verringern des einen oder der mehreren zweiten Schmerzausmaße im Vergleich zu dem einen oder den mehreren ersten Schmerzausmaßen umfasst,
wobei ein erstes Schmerzausmaß bei dem Individuum vor der Verabreichung der AMDACs und ein zweites Schmerzausmaß bei dem Individuum nach der Verabreichung der AMDACs vorzugsweise unter Verwendung einer Schmerzbeurteilungsskala bestimmt werden, wobei die Schmerzbeurteilungsskala vorzugsweise die numerische Schmerzintensitätsskala; die Schmerzqualitätsbeurteilungsskala; die einfache beschreibende Schmerzintensitätsskala; die visuelle Analogskala; die Wong-Baker-FACES-Schmerzbeurteilungsskala; die FLACC-Skala; die CRIES-Skala; die COMFORT-Skala; oder Messung des hervorgerufenen Schmerzes ist, der durch Aussetzen des Patienten gegenüber Kälte, Hitze oder mechanische Stimuli verursacht wird.

3. Zusammensetzung zur Verwendung nach Anspruch 1 oder Anspruch 2, wobei das Verfahren außerdem das Bestimmen eines ersten Ausmaßes von einem oder mehreren physiologischen Anzeichen für Schmerz bei dem Individuum vor der Verabreichung der AMDACs und das Bestimmen eines zweiten Ausmaßes von einem oder mehreren physiologischen Anzeichen für Schmerz bei dem Individuum nach der Verabreichung der AMDACs umfasst, wobei das Verfahren zur Schmerzbehandlung das Verringern des zweiten Ausmaßes im Vergleich zum ersten Ausmaß umfasst.

4. Zusammensetzung zur Verwendung nach Anspruch 3, wobei das physiologische Anzeichen für Schmerz Folgendes ist:
(i) Herzfrequenz des Individuums, wobei die Herzfrequenz des Individuums nach der Verabreichung im Vergleich zu der Herzfrequenz des Individuums vor der Verabreichung vorzugsweise niedriger ist;
(ii) systolischer Blutdruck des Individuums, wobei der systolische Blutdruck des Individuums nach der Verabreichung im Vergleich zu dem systolischen Blutdruck des Individuums vor der Verabreichung vorzugsweise niedriger ist; oder
(iii) diastolischer Blutdruck des Individuums, wobei der diastolische Blutdruck des Individuums nach der Verabreichung im Vergleich zu dem diastolischen Blutdruck des Individuums vor der Verabreichung vorzugsweise niedriger ist.

5. Zusammensetzung zur Verwendung nach Anspruch 4, wobei die AMDACs Folgendes sind:
(a) HLA-G⁻, wie mittels RT-PCR bestimmt werden kann, und/oder CD49f⁺, wie mittels Durchflusszytometrie bestimmt werden kann;
(b) CD90⁺, CD105⁺ und/oder CD117⁻, wie mittels Durchflusszytometrie bestimmt werden kann, wobei die AMDACs vorzugsweise OCT-4⁻ und HLA-G⁻, wie mittels RT-PCR bestimmt werden kann, und/oder CD49f⁺, CD90⁺, CD105⁺ und CD117⁻, wie mittels Durchflusszytometrie bestimmt werden kann, sind;
(c) VEGFR1/Flt-1⁺ (Rezeptor 1 des Gefäßendothelwachstumsfaktors) und VEGFR2/KDR⁺ (Rezeptor 2 des Gefäßendothelwachstumsfaktors), wie mittels Immunlokalisierung bestimmt werden kann;
(d) eines oder mehrere aus CD9⁺, CD10⁺, CD44⁺, CD54⁺, CD98⁺, Tie-2⁺ (Angiopoietin-Rezeptor), TEM-7⁺ (endothelialer Tumormarker 7), CD31⁻, CD34⁻, CD45⁻, CD133⁻, CD143⁻ (Angiotensin-I-konvertierendes Enzym, ACE), CD146⁻ (Melanomzellenadhäsionsmolekül) und/oder CXCR4⁻ (Chemokin-(C-X-C-Motiv)-Rezeptor 4), wie mittels Immunlokalisierung bestimmt werden kann;
(e) VE-Cadherin⁻, wie mittels Immunlokalisierung bestimmt werden kann; oder
(f) CD105⁺ und CD200⁺, wie mittels Immunlokalisierung bestimmt werden kann.

6. Zusammensetzung zur Verwendung nach einem der Ansprüche 1 bis 5, wobei die AMDACs CD34 nicht exprimieren, wie mittels Immunlokalisierung nach einer 7-tägigen Exposition gegenüber 50 ng/ml VEGF bestimmt werden kann.

7. Zusammensetzung zur Verwendung nach einem der Ansprüche 1 bis 6, wobei die AMDACs in einer isolierten Zellpopulation umfasst sind und wobei zumindest 50 % der Zellen in der Population AMDACs sind,
wobei vorzugsweise zumindest 80 % oder zumindest 90 % der Zellen in der Population AMDACs sind.

8. Zusammensetzung zur Verwendung nach Anspruch 7, wobei die Population außerdem einen zweiten isolierten Zelltyp umfasst und wobei die Population nicht Amnion, ein Teil von Amnion oder ein Homogenat von Amnion ist,
wobei der zweite Zelltyp vorzugsweise embryonale Stammzellen, Blutzellen, aus peripherem Blut isolierte Stammzellen, aus Plazentablut isolierte Stammzellen, aus Plazentaperfusat isolierte Stammzellen, aus Plazentagewebe isolierte Stammzellen, aus Nabelschnurblut isolierte Stammzellen, Nabelschnur-Stammzellen, aus dem Knochenmark stammende mesenchymale Stammzellen, aus dem Knochenmark stammende mesenchymale Stromazellen, hämatopoietische Stammzellen, somatische Stammzellen, Chondrozyten, Fibroblasten, Muskelzellen, Endothelzellen, Angioblasten, endotheliale Vorläuferzellen, Perizyten, Kardiomyozyten, Myozyten, Kardiomyoblasten, Myoblasten oder Zellen, die manipuliert wurden, um embryonalen Stammzellen zu ähneln, umfasst,
wobei der zweite Zelltyp vorzugsweise zumindest 10 % der Zellen oder zumindest 25 % der Zellen in der Population ausmacht.

9. Zusammensetzung zur Verwendung nach Anspruch 8, wobei der zweite Zelltyp hämatopoietische Stamm- oder Vorläuferzellen umfasst, wobei die hämatopoietische Stamm- oder Vorläuferzellen CD34⁺-Zellen sind.

10. Zusammensetzung zur Verwendung nach einem der Ansprüche 1 bis 5, wobei die AMDACs OCT-4⁻, wie mittels RT-PCR bestimmt werden kann, und CD49f⁺, HLA-G⁻, CD90⁺, CD105⁺ und CD117⁻, wie mittels Immunlokalisierung bestimmt werden kann, sind; und wobei die AMDACs:
(a) eines oder mehrere aus CD9, CD10, CD44, CD54, CD98, CD200, Tie-2, TEM-7, VEGFR1/Flt-1 oder VEGFR2/KDR (CD309), wie mittels Immunlokalisierung bestimmt werden kann, exprimieren;
(b) keine Expression von CD31, CD34, CD38, CD45, CD133, CD143, CD144, CD146, CD271, CXCR4, HLA-G oder VE-Cadherin zeigen, wie mittels Immunlokalisierung bestimmt werden kann, oder keine Expression von SOX2 zeigen, wie mittels RT-PCR bestimmt werden kann;
(c) mRNA für ACTA2, ADAMTS1, AMOT, ANG, ANGPT1, ANGPT2, ANGPTL1, ANGPTL2, ANGPTL4, BAI1, CD44, CD200, CEACAM1, CHGA, COL15A1, COL18A1, COL4A1, COL4A2, COL4A3, CSF3, CTGF, CXCL12, CXCL2, DNMT3B, ECGF1, EDG1, EDIL3, ENPP2, EPHB2, FBLN5, F2, FGF1, FGF2, FIGF, FLT4, FN1, FST, FOXC2, GRN, HGF, HEY1, HSPG2, IFNB1, IL8, IL12A, ITGA4, ITGAV, ITGB3, MDK, MMP2, MYOZ2, NRP1, NRP2, PDGFB, PDGFRA, PDGFRB, PECAM1, PF4, PGK1, PROX1, PTN, SEMA3F, SERPINB5, SERPINC1, SERPINF1, TIMP2, TIMP3, TGFA, TGFB1, THBS1, THBS2, TIE1, TIE2/TEK, TNF, TNNI1, TNFSF15, VASH1, VEGF, VEGFB, VEGFC, VEGFR1/FLT1 und/oder VEGFR2/KDR exprimieren;
(d) eines oder mehrere der Proteine CD49d, Connexin-43, HLA-ABC, beta-2- Mikroglobulin, CD349, CD318, PDL1, CD106, Galectin-1, ADAM 17, Angiotensinogen-Vorläufer, Filamin A, alpha-Actinin 1, Megalin, Makrophage-acetylierter LDL-Rezeptor I und II, Vorläufer des Activin-Rezeptors Typ IIB, Wnt-9-Protein, saures Gliafaserprotein, Astrozyt, Myosinbindendes Protein C und/oder Myosin-Schwerkette, Nichtmuskel-Typ A exprimieren;
(e) eines oder mehrere aus VEGF, HGF, IL-8, MCP-3, FGF2, Follistatin, G-CSF, EGF, ENA-78, GRO, IL-6, MCP-1, PDGF-BB, TIMP-2, uPAR und/oder Galectin-1 in Kulturmedium, in dem die AMDACs kultiviert werden, sekretieren;
(f) mikroRNAs miR-17-3p, miR-18a, miR-18b, miR-19b, miR-92 und/oder miR-296 in einem höheren Ausmaß als eine äquivalente Anzahl von aus dem Knochenmark stammenden mesenchymalen Stammzellen exprimieren;
(g) mikroRNAs miR-20a, miR-20b, miR-221, miR-222, miR-15b und/oder miR-16 in einem geringeren Ausmaß als eine äquivalente Anzahl von aus dem Knochenmark stammenden mesenchymalen Stammzellen exprimieren;
(h) miRNAs miR-17-3p, miR-18a, miR-18b, miR-19b, miR-92, miR-20a, miR-20b, miR-296, miR-221, miR-222, miR-15b und/oder miR-16 exprimieren; oder
(i) erhöhte Ausmaße von CD202b, IL-8 und/oder VEGF exprimieren, wenn sie in weniger als etwa 5 % O₂ kultiviert werden, verglichen mit der Expression von CD202b, IL-8 und/oder VEGF unter 21 % O₂.

11. Zusammensetzung zur Verwendung nach Anspruch 1, wobei der Schmerz folgender ist:
(a) neuropathischer Schmerz;
(b) Entzündungsschmerz;
(c) Knochenschmerz; oder
(d) durch Krebs verursacht wird.

12. Zusammensetzung zur Verwendung nach Anspruch 11, wobei der neuropathische Schmerz durch Folgendes verursacht wird:
(i) diabetische Neuropathie;
(ii) Verletzung eines Nervs des Individuums; oder
(iii) ein Arzneimittel.

13. Zusammensetzung zur Verwendung nach Anspruch 12, wobei das Arzneimittel ein platinhältiges Antikrebs-Arzneimittel ist oder umfasst,
wobei das platinhältige Antikrebs-Arzneimittel vorzugsweise Oxaliplatin, Carboplatin oder Cisplatin ist oder umfasst; oder das Arzneimittel Paclitaxel ist oder umfasst.

14. Zusammensetzung zur Verwendung nach Anspruch 11, wobei der Knochenschmerz mit einer Krebserkrankung verbunden oder von dieser verursacht ist.

15. Zusammensetzung zur Verwendung nach einem der Ansprüche 1 bis 14, wobei die AMDACs für eine lokale Verabreichung formuliert sind; oder
wobei die AMDACs zur systemischen, intravenösen oder intraarteriellen Verabreichung formuliert sind.

## Revendications

1. Composition comprenant des cellules adhérentes dérivées de l'amnios (AMDAC) qui adhérent à la surface d'une culture tissulaire OCT-4⁻ pour une utilisation dans une méthode de traitement de la douleur chez un individu, dans laquelle ladite douleur ne répond pas à une thérapie par stéroïde, ou à une thérapie anti-inflammatoire non stéroïdienne, dans laquelle la méthode comprend l'administration d'une quantité thérapeutiquement efficace d'AMDAC à l'individu, et dans laquelle la quantité thérapeutiquement efficace est une quantité suffisante pour provoquer une amélioration détectable de ladite douleur.

2. Composition pour une utilisation selon la revendication 1, dans laquelle ladite méthode comprend en outre la détermination d'un ou plusieurs premiers niveaux de douleur chez ledit individu avant l'administration desdites AMDAC, et la détermination d'un ou plusieurs seconds niveaux de douleur chez ledit individu après l'administration desdites AMDAC, dans laquelle ladite méthode de traitement de la douleur comprend la réduction dudit ou desdits seconds niveaux de ladite douleur par comparaison audit ou auxdits premiers niveaux de douleur,
de préférence dans laquelle un premier niveau de douleur chez ledit individu avant l'administration desdites AMDAC, et un second niveau de douleur chez l'individu après l'administration desdites AMDAC sont déterminés en utilisant une échelle d'évaluation de la douleur, de préférence dans laquelle ladite échelle d'évaluation de la douleur est l'échelle numérique d'intensité de la douleur ; l'échelle d'évaluation qualitative de la douleur ; l'échelle d'intensité de la douleur descriptive simple ; l'échelle visuelle analogique ; l'échelle d'évaluation de la douleur FACES de Wong-Baker ; l'échelle FLACC ; l'échelle CRIES ; l'échelle CONFORT ; ou une mesure de douleur suscitée induite en soumettant le patient à des stimuli froids, chauds ou mécaniques.

3. Composition pour une utilisation selon la revendication 1 ou la revendication 2, dans laquelle ladite méthode comprend en outre la détermination d'un premier niveau d'un ou plusieurs indices physiologiques de la douleur chez ledit individu avant l'administration desdites AMDAC et la détermination d'un second niveau d'un ou plusieurs indices physiologiques de la douleur chez ledit individu après l'administration desdites AMDAC, dans laquelle ladite méthode de traitement de la douleur comprend la réduction dudit second niveau par comparaison audit premier niveau.

4. Composition pour une utilisation selon la revendication 3, dans laquelle ledit indice physiologique de la douleur est :
(i) le rythme cardiaque chez l'individu, de préférence dans laquelle ledit rythme cardiaque chez ledit individu est inférieur après ladite administration par comparaison audit rythme cardiaque chez ledit individu avant ladite administration ;
(ii) la pression systolique dudit individu, de préférence dans laquelle ladite pression systolique dudit individu est inférieure après ladite administration par comparaison à ladite pression systolique dudit individu avant ladite administration ; ou
(iii) la pression diastolique dudit individu, de préférence dans laquelle ladite pression diastolique dudit individu est inférieure après ladite administration par comparaison à ladite pression diastolique dudit individu avant ladite administration.

5. Composition pour une utilisation selon la revendication 4, dans laquelle lesdites AMDAC sont :
(a) HLA-G⁻, comme pouvant être déterminé par RT-PCR et/ou CD49f⁺, comme pouvant être déterminé par cytométrie en flux ;
(b) CD90⁺, CD105⁺ et/ou CD117⁻ comme pouvant être déterminé par cytométrie en flux, de préférence dans laquelle lesdites AMDAC sont OCT-4⁻ et HLA-G⁻, comme pouvant être déterminé par RT-PCR, et CD49f⁺, CD90⁺, CD105⁺ et CD117⁻ comme pouvant être déterminé par cytométrie en flux ;
(c) VEGFR1/Ftl-1⁺ (récepteur du facteur de croissance de l'endothélium vasculaire 1) et VEGFR2/KDR⁺ (récepteur du facteur de croissance de l'endothélium vasculaire 2), comme pouvant être déterminé par immunolocalisation ;
(d) un ou plusieurs de CD9⁺, CD10⁺, CD44⁺, CD54⁺, CD98⁺, Tie-2⁺ (récepteur de l'angiopoïétine), TEM-7⁺ (marqueur endothélial tumoral 7), CD31⁻, CD34⁻, CD45⁻, CD133⁻, CD143⁻ (enzyme convertissant l'angiotensine I, ACE), CD146⁻ (molécule d'adhésion cellulaire de mélanome) et/ou CXCR4⁻ (récepteur de la chimiokine 4 (motif C-X-C)) comme pouvant être déterminé par immunolocalisation ;
(e) la VE-cadhérine⁻ comme pouvant être déterminé par immunolocalisation ; ou
(f) CD105⁺ et CD200⁺ comme pouvant être déterminé par immunolocalisation.

6. Composition pour une utilisation selon l'une quelconque des revendications 1 à 5, dans laquelle lesdites AMDAC n'expriment pas CD34 comme pouvant être déterminé par immunolocalisation après une exposition à 50 ng/ml de VEGF pendant 7 jours.

7. Composition pour une utilisation selon l'une quelconque des revendications 1 à 6, dans laquelle lesdites AMDAC sont comprises au sein d'une population isolée de cellules, et dans laquelle au moins 50 % des cellules dans ladite population sont lesdites AMDAC,
de préférence dans laquelle au moins 80 %, ou au moins 90 % des cellules dans ladite population sont lesdites AMDAC.

8. Composition pour une utilisation selon la revendication 7, dans laquelle ladite population comprend en outre un second type isolé de cellules, et dans laquelle ladite population n'est pas un amnios, une partie d'un amnios ou un homogénat d'un amnios,
de préférence dans laquelle ledit second type de cellules est des cellules souches embryonnaires, des cellules sanguines, des cellules souches isolées de sang périphérique, des cellules souches isolées de sang placentaire, des cellules souches isolées de perfusat placentaire, des cellules souches isolées de tissu placentaire, des cellules souches isolées de sang de cordon ombilical, des cellules souches de cordon ombilical, des cellules souches mésenchymateuses issues de la moelle osseuse, des cellules stromales mésenchymateuses issues de la moelle osseuse, des cellules souches hématopoïétiques, des cellules souches somatiques, des chondrocytes, des fibroblastes, des cellules musculaires, des cellules endothéliales, des angioblastes, des cellules progénitrices endothéliales, des péricytes, des cardiomyocytes, des myocytes, des cardiomyoblastes, des myoblastes ou des cellules manipulées pour ressembler à des cellules souches embryonnaires,
plus préférablement dans laquelle ledit second type de cellules comprend au moins 10 % des cellules ou au moins 25 % des cellules dans ladite population.

9. Composition pour une utilisation selon la revendication 8, dans laquelle ledit second type de cellules est des cellules souches hématopoïétiques ou progénitrices, de préférence dans laquelle lesdites cellules souches hématopoïétiques ou progénitrices sont des cellules CD34⁺.

10. Composition pour une utilisation selon l'une quelconque des revendications 1 à 5, dans laquelle lesdites AMDAC sont OCT-4⁻, comme pouvant être déterminé par RT-PCR et CD49f⁺, HLA-G⁻, CD90⁺, CD105⁺ et CD117⁻, comme pouvant être déterminé par immunolocalisation, et dans laquelle lesdites AMDAC :
(a) expriment un ou plusieurs de CD9, CD10, CD44, CD54, CD98, CD200, Tie-2, TEM-7, VEGFR1/Flt-1 et/ou VEGFR2/KDR (CD309), comme pouvant être déterminé par immunolocalisation ;
(b) sont exemptes de l'expression de CD31, CD34, CD38, CD45, CD133, CD143, CD144, CD146, CD271, CXCR4, HLA-G et/ou VE-cadhérine, comme pouvant être déterminé par immunolocalisation, et/ou sont exemptes de l'expression de SOX2, comme pouvant être déterminé par RT-PCR ;
(c) expriment l'ARNm pour ACTA2, ADAMTS1, AMOT, ANG, ANGPT1, ANGPT2, ANGPTL1, ANGPTL2, ANGPTL4, BAI1, CD44, CD200, CEACAM1, CHGA, COL15A1, COL18A1, COL4A1, COL4A2, COL4A3, CSF3, CTGF, CXCL12, CXCL2, DNMT3B, ECGF1, EDG1, EDIL3, ENPP2, EPHB2, FBLN5, F2, FGF1, FGF2, FIGF, FLT4, FN1, FST, FOXC2, GRN, HGF, HEY1, HSPG2, IFNB1, IL8, IL12A, ITGA4, ITGAV, ITGB3, MDK, MMP2, MYOZ2, NRP1, NRP2, PDGFB, PDGFRA, PDGFRB, PECAM1, PF4, PGK1, PROX1, PTN, SEMA3F, SERPINB5, SERPINC1, SERPINF1, TIMP2, TIMP3, TGFA, TGFB1, THBS1, THBS2, TIE1, TIE2/TEK, TNF, TNNI1, TNFSF15, VASH1, VEGF, VEGFB, VEGFC, VEGFR1/FLT1 et/ou VEGFR2/KDR ;
(d) expriment une ou plusieurs de CD49d, la connexine-43, HLA-ABC, la 2-microglobuline bêta, CD349, CD318, PDL1, CD106, la galectine-1, ADAM 17, un précurseur de l'angiotensinogène, la filamine A, l'alpha-actinine 1, la mégaline, le récepteur de LDL acétylé macrophage de types I et II, le précurseur du récepteur de l'activine de type IIB, la protéine Wnt-9, la protéine acide fibrillaire gliale, l'astrocyte, la protéine C de liaison à la myosine et/ou une chaîne lourde de myosine non musculaire de type A ;
(e) sécrètent un ou plusieurs de VEGF, HGF, IL-8, MCP-3, FGF2, la follistatine, G-CSF, EGF, ENA-78, GRO, IL-6, MCP-1, PDGF-BB, TIMP-2, uPAR, et/ou la galectine-1 dans un milieu de culture dans lequel les AMDAC sont cultivées ;
(f) expriment les micro-ARN miR-17-3p, miR-18a, miR-18b, miR-19b, miR-92 et/ou miR-296 à un niveau supérieur à un nombre équivalent de cellules souches mésenchymateuses issues de la moelle osseuse ;
(g) expriment les micro-ARN miR-20a, miR-20b, miR-221, miR-222, miR-15b et/ou miR-16 à un niveau inférieur à un nombre équivalent de cellules souches mésenchymateuses issues de la moelle osseuse ;
(h) expriment les micro-ARN miR-17-3p, miR-18a, miR-18b, miR-19b, miR-92, miR-20a, miR-20b, miR-296, miR-221, miR-222, miR-15b et/ou miR-16 ; ou
(i) expriment des taux accrus de CD202b, IL-8 ou VEGF lorsqu'elles sont cultivées dans moins d'environ 5 % d'O₂, par comparaison à l'expression de CD202b, IL-8 et/ou VEGF sous 21 % d'O₂.

11. Composition pour une utilisation selon la revendication 1, dans laquelle ladite douleur est :
(a) une douleur neuropathique ;
(b) une douleur inflammatoire ;
(c) une douleur osseuse ; ou
(d) provoquée par un cancer.

12. Composition pour une utilisation selon la revendication 11, dans laquelle ladite douleur neuropathique est provoquée par
(i) une neuropathie diabétique ;
(ii) une lésion d'un nerf chez ledit individu ; ou
(iii) un médicament.

13. Composition pour une utilisation selon la revendication 12, dans laquelle ledit médicament est ou comprend un médicament anticancéreux contenant du platine,
de préférence dans laquelle ledit médicament anticancéreux contenant du platine est ou comprend de l'oxaliplatine, du carboplatine ou du cisplatine ; ou ledit médicament est ou comprend du paclitaxel.

14. Composition pour une utilisation selon la revendication 11, dans laquelle ladite maladie osseuse est associée à ou causée par un cancer.

15. Composition pour une utilisation selon l'une quelconque des revendications 1 à 14, dans laquelle lesdites AMDAC sont formulées pour une administration par voie locale ; ou
dans laquelle lesdites AMDAC sont formulées pour une administration par voie systémique, intraveineuse ou intra-artérielle.
